# EUROPEAN PATENT APPLICATION

(11) **EP 4 378 480 A1**
(43) Date of publication of application: **05.06.2024**
(21) Application number: 22849399.5
(22) Date of filing: 22.07.2022
(51) Int. Cl.: A61K 45/00, A61K 38/08, A61K 38/10, A61K 38/16, A61P 31/20, C07K 7/04, C07K 14/00, C12N 15/11

(54) **ANTI-HEPATITIS B VIRUS AGENT TARGETING HOST FACTOR LIPG**

(30) Priority: 26.07.2021 JP 2021121446
(71) Applicant: Purotech Bio, Inc., Yokohama-shi, Kanagawa 230-0045 (JP)
(72) Inventor: YANAGAWA, Hiroshi, Yokohama-shi, Kanagawa 230-0045 (JP); TABATA, Noriko, Yokohama-shi, Kanagawa 230-0045 (JP); YONEMURA, Yuko, Yokohama-shi, Kanagawa 230-0045 (JP); IDE, Mayuko, Yokohama-shi, Kanagawa 230-0045 (JP); ITO, Satoru, Yokohama-shi, Kanagawa 230-0045 (JP); KANEKO, Shuichi, Kanazawa-shi, Ishikawa 920-1164 (JP); HONDA, Masao, Kanazawa-shi, Ishikawa 920-1164 (JP); SHIRASAKI, Takayoshi, Kanazawa-shi, Ishikawa 920-1164 (JP); MURAI, Kazuhisa, Kanazawa-shi, Ishikawa 920-1164 (JP); OKADA, Hikari, Kanazawa-shi, Ishikawa 920-1164 (JP)
(74) Representative: Mewburn Ellis LLP
(86) International application number: PCT/JP2022/028503
(87) International publication number: WO 2023/008337

(57) **Abstract**

An anti-HBV agent according to the present invention contains as an active ingredient a substance that binds to LIPG and inhibits the function(s) of LIPG. The inventors of the present application have found that LIPG is a factor playing an important role in HBV attachment and uptake, and functions as a cofactor that supports HBV entry into cells independently of NTCP, and also contributes to HBV proliferation in cells. T The LIPG-binding substance binds to LIPG on the surface of liver cells to inhibit HBV attachment and uptake into cells and, when delivered into liver cells, binds to intracellular LIPG to block any step(s) in the intracellular HBV proliferation process, thereby exerting an anti-HBV activity.

## Description

### TECHNICAL FIELD

The present invention relates to an anti-hepatitis B virus agent targeting a host factor. LIPG.

### BACKGROUND ART

Hepatitis B virus (HBV) infection is a major cause of hepatocellular carcinoma (HCC), and the number of people who are chronic carriers of the virus is said to be 0.25 billion (Non-Patent Document 1). HBV is a DNA virus carrying an incomplete circular double-stranded DNA (relaxed circular genome) of 3.2 kb in length, which is organized into overlapping open reading frames (Non-Patent Document 2).

Hepatitis B virus inhibitors that have been developed, including entecavir, adefovir, and tenofovir, are mainly nucleotide analogues and are designed to inhibit the reverse transcription step. The amount of FIBs antigen production and the control of HBV DNA replication were used as indexes for the research and development of hepatitis B virus inhibitors. That is, most of these inhibitors are classified as agents that inhibit the reverse transcription step of the HBV pregenome, and are responsible for reducing viral replication after infection. Recently, progress has also been made in the development of agents that inhibit nucleocapsid formation. Vaccines have been developed to control the spread of HBV via the route of maternal transmission as part of the WHO proposed program to eliminate HBV and have achieved some success. A target molecule of HBV important for viral infection had to be identified in order to more actively inhibit infection in the context of epidemic control. However, current attempts at identifying a specific receptor for HBV infection have been unsuccessful.

NTCP (Na'/taurocholate cotransporting polypeptide; bile acid transporter) has been identified in 2012 as a functional receptor specific for HBV (Non-Patent Document 3). Based on this information, many candidate agents that inhibit the system in which NTCP is used as a target infection receptor were immediately investigated, and agents that inhibit viral infection and entry were actively screened and developed (Non-Patent Documents 4 to 6). Furthermore, a more recent report has indicated that EGFR acts as a co-factor of NTCP to increase the susceptibility of HBV infection and facilitates the transition from viral infection to viral internalization (Non-Patent Document 7). This report has also indicated that administration of an anticancer drug targeting EGFR, gefitinib, reduces HBV infection in an *in vitro* cell system.

However, NTCP has been suggested as a necessary but insufficient for establishment of infection. HBV was indicated to enter HepaRG cells via the caveolin-1-mediated pathway (Non-Patent Document 8), whereas other researches using HepG2-NTCP cells indicated that caveolin-1 had no effect on HBV entry (Non-Patent Document 9) and suggested that involvement of the clathrin-dependent endocytosis in HBV entry (Non-Patent Document 10). Several receptors and cofactors necessary for hepatitis C virus (HCV) infection have been identified (Non-Patent Document 11), but the factors required for HBV attachment and entry are not yet fully understood.

Thus, Hashimoto and his colleagues established an HCl cell line that is rarely infected with HBV (approximately one infected cell per 3000 cells), performed single-cell transcriptome analysis (Nxl-Seq) on the resulting cell line, and identified four host factors that are highly expressed in HBV-infected cells (Non-Patent Document 12). One of the host factors, endothelial lipase (LIPG; Lipase G, Endothelial Type; NCBI Gene ID: 9388), is a member of the triglyceride lipase family and is expressed mainly in vascular endothelial cells (Non-Patent Document 13) and also in tissues of liver, kidney, lung, testis, placenta, and the like (Non-Patent Document 14). LIPG reportedly contributes to various function in the body, such as involvement in high density lipoprotein (HDL) metabolism, cytokine expression, lipid formation, and cancer development and progression (Non-Patent Documents 15 to 18), and also contributes to cellular uptake of lipoproteins by acting as a bridging molecule between lipoproteins and HSPGs (Non-Patent Document 19).

However, HBV infection is dependent not only on use of NTCP. Although cell entry inhibitors targeting NTPC are being developed for HBV, other pathways of infection remain unknown, and inhibitors for each of these pathways have not yet been studied or developed, leaving HBV free to infect cells. It is desirable that the identification of the route of HBV infection and the development of inhibitors against HBV be completed as soon as possible. That is, even cells that do not express NTCP are infected by HBV, suggesting the presence of an unknown infection receptor. Several infection receptors such as HSPGs, hCD81, DC-SIGN, LDI,R, SR-BI, CLDN-1, OCLN, and NPC1L1 have been identified for HCV, and the information on these receptors is contributing to progress of virology and also to drug development.

HSPG (heparan sulfate proteoglycan), which is discussed as a receptor in HCV infection, may also be used in HBV infection and is not recognized as a specific receptor but as a receptor with slightly less preferential affinity (Non-Patent Document 20). A molecule associated with HSPGs could function as the essence of a receptor. Although several proteins have been proposed as candidates for HSPG core proteins, only Glypican-5 has been identified as an I-ISPG core protein (Non-Patent Document 21), and the actual state of HSPGs remains to be elucidated. However, the mechanism of HBV entry into cells is gradually understood. The expression of NTCP may be reduced by the addition of bile acids (CDCA), which fluctuate throughout the day. Therefore, the elucidation of infection routes other than NTCP is important.

LIPG is one of the molecules identified as functioning to maintain HBV genes. One of the inventors of the present application, Kaneko, and his colleagues discovered four host genes involved in the maintenance of HBV in infected cells and filed a patent application for the genes (Patent Document 1). Among the four genes is the LIPG gene. Patent Document 1 has confirmed that a shRNA-dependent LIPG gene silencing system and GSK264220A, also known as a LIPG inhibitor, are effective in reducing HBV-cccDNA. However, no LIPG inhibitor has yet been developed for use in patients with HBV infection.

### PRIOR ART DOCUMENT(S)

### PATENT DOCUMENT(S)

Patent Document 1: WO 2017/082202 A1

### NON-PATENT DOCUMENT(S)

Non-Patent Document 1: Thomas, DL., N. Engl. J. Med., 380: 2041-2050, 2019
Non-Patent Document 2: Sukuda, S. Watashi K., Antiviral Res, 104925, 2020
Non-Patent Document 3: Yan, H. et al., eLife, 1: e00049, 2012
Non-Patent Document 4: Ogura, N. et al., Biochem. Biophys. Res. Commun., 498: 64-71, 2018
Non-Patent Document 5: Passioura, T. et al., Cell Chem. Biol., 25: 1-10, 2018
Non-Patent Document 6: Zhao, K. et al., Emerg. Microbes Infect., 8: 186, 2018
Non-Patent Document 7: Iwamoto, M. et al., Proc. Natl. Acad. Sci. USA., 116: 8482-8492, 2019
Non-Patent Document 8: Macovei. A. et al., J. Virol., 84: 243-253, 2010
Non-Patent Document 9: Umetsu, T. et al., Biochem. Biophys. Rep., 14: 20-25, 2018
Non-Patent Document 10: Huang, HC., J. Virol., 86: 9443-9453, 2012
Non-Patent Document 11: Gerold, G., et al., Cold Spring Harb. Perspect Med., 10, a036830, 2020
Non-Patent Document 12: Hashimoto, S. et al., PLoS One, 16:e0246313, 2021
Non-Patent Document 13: Jaye, M. et al.. Nat. Genet. 21: 424-428, 1999
Non-Patent Document 14: Hirata. K. et al., J. Biol. Chem., 274: 14170-14175, 1999
Non-Patent Document 15: Yu, J. E. et al., Histol. Histopathol. 33, 1-10, 2018
Non-Patent Document 16: Nielsen, JE. et al., Int. J. Androl., 33:e207-215, 2010
Non-Patent Document 17: Slebe. F. et al., Nat. Commun. 7, 11199, 2016
Non-Patent Document 18: Lo, PK. et al., Elife, 7, 2018
Non-Patent Document 19: Fuki, IV. et al., J. Biol. Chem. 278: 34331-34338, 2003
Non-Patent Document 20: Urban, S. et al., Gastroenterol., 147: 48-64, 2014
Non-Patent Document 21: Verrier, ER. et al., Hepatol., 36: 35-48, 2016
Non-Patent Document 22: Nemoto, N. et al., FEBS Lett., 414: 405-408, 1997
Non-Patent Document 23: Miyamoto-Sato, E. et al., Nucleic Acids Res., 28: 1176-1182, 2000
Non-Patent Document 24: Miyamoto-Sato, E. et al., Nucleic Acids Res., 31: e78, 2003
Non-Patent Document 25: Doi, N. et al., J. Biotechnol., 131: 231-239, 2007

### SUMMARY OF THE INVENTION

### PROBLEMS TO BE SOLVED BY THE INVENTION

LIPG is expected to be a target for HBV treatment because knock-down of the LIPG gene or use of a LIPG inhibitor significantly reduces the levels of HBV DNA and HBV cccDNA in host cells. Therefore, there is a strong demand for elucidating the mechanism of maintenance of HBV, including the behavior of cccDNA, and discovering a new anti-HBV drug targeting LIPG for HBV treatment in order to completely remove HBV from liver cells and prevent or treat hepatitis B. An object of the present invention is to provide a novel anti-HBV drug targeting LIPG.

### MEANS FOR SOLVING THE PROBLEM

EL/LIPG is also reported to interact with HSPGs (Non-Patent Document 19), suggesting the possibility that EL/LIPG functions as a cofactor involved in infection. When the LIPG gene was identified as a gene required for the maintenance of HBV, reduction of HBV-DNA/HBV-cccDNA was confirmed in a LIPG gene silencing system by means of shRNA (Patent Document 1). On the contrary, a later study has confirmed that forced expression of LIPG results in elevation of the HBV-DNA level. From this result, the inventors expected the involvement of LIPG as a receptor and found that LIPG can be considered as a new receptor candidate. The inventor also established a screening system for infection inhibitors targeting LIPG and found that HBV infection occurs even in cells expressing very low levels of NTCP, provided that the cells express LIPG. Although the possibility that LIPG contributes to HBV infection by exerting an enzymatic function has already been confirmed by the inhibitory effect of GSK264220A, it is still possible that functions of LIPG other than direct enzymatic activity may contribute to HBV infection. Inhibitors that target host factors are generally required to have fewer side effects. Accordingly, the information about the side effects associated with inhibiting a target will suggest in advance the appropriateness of developing such inhibitors. A LIPG (endothelial lipase)-KO mouse was reported in 2011, and the mouse showed an increase in HDL, which is known as good cholesterol, and no other phenotypes were described (Hara, T. et al., J. Lipid Res., 52: 57-67 (2011)). In addition, a mAb against LIPG (endothelial lipase) has recently been developed and is already in clinical trials, and no particular side effects have been reported (LeLay, JE. et al., Sci. Transl. Med., 13:eabb0602 (2021)). In consideration of these results, the appropriateness of targeting LIPG is suggested by the less significant side effects.

The inventors of the present application conducted extensive research and consequently found that LIPG increases HBV attachment and entry into cells in an NTCP-independent manner, that LIPG, HSPGs present on the cell membrane surface, and HBV particles bind to each other, leading to the uptake of HBV particles into liver cells by caveolin-dependent endocytosis, and that LIPG increases caveolin-1 expression and thus HBV entry in a caveolin-1-dependent manner. Aiming at development of a peptide drug useful for complete cure of hepatitis B virus, the inventors conducted further extensive research and finally developed polypeptides that bind to LIPG (LIPG-binding peptides) and thus can inhibit LIPG-mediated HBV attachment and entry into host cells.

The inventors conducted further extensive research and surprisingly found that the LIPG-binding peptides, which inhibit HBV attachment and entry into liver cells on the liver cell surface, have a much greater anti-HBV effect when delivered into liver cells than when not delivered into liver cells.

The invention of the present application, as completed by the extensive research described above, includes the following modes.

[1] An anti-hepatitis B virus agent containing as an active ingredient a substance that binds to LIPG and inhibits the function(s) of LIPG.
[2] The anti-hepatitis B virus agent according to [1], wherein the inhibition of LIPG function(s) is at least one selected from inhibition of binding of LIPG to N4BP1 and inhibition of suppression by LIPG of the RNase activity of N4BP1.
[3] The anti-hepatitis B virus agent according to [1] or [2], wherein the substance has an activity to inhibit hepatitis B virus entry into cells mediated by LIPG.
[4] The anti-hepatitis B virus agent according to [3], wherein the substance binds to the heparin-binding region of LIPG to inhibit the binding of LIPG and heparan sulfate proteoglycan to hepatitis B virus or the binding of the LIPG-heparan sulfate proteoglycan complex to hepatitis B virus.
[5] The anti-hepatitis B virus agent according to any one of [1] to [4], wherein the substance is at least one polypeptide selected from the following polypeptides (1) to (30):
   (1) a polypeptide of the sequence NCRHNTAG (SEQ ID NO: 25);
   (2) a polypeptide of the sequence NARHNTAG (SEQ ID NO: 26);
   (3) a polypeptide of the sequence LNCRDNTR (SEQ ID NO: 23);
   (4) a polypeptide of the sequence LNARDNTR (SEQ ID NO: 24);
   (5) a polypeptide of the sequence LNCRDNTRPVMSAMTC (SEQ ID NO: 1);
   (6) a polypeptide of the sequence IRNVNHSDH (SEQ ID NO: 9);
   (7) a polypeptide of the sequence LNVGYVFYP (SEQ ID NO: 18);
   (8) a polypeptide of the sequence SLYTGFRAH (SEQ ID NO: 15);
   (9) a polypeptide of the sequence RCRQSWNTM (SEQ ID NO: 14);
   (10) a polypeptide of the sequence GADLRRGCC (SEQ ID NO: 11);
   (11) a polypeptide of the sequence RKSGGVCLNCRHNTAG (SEQ ID NO: 21);
   (12) a polypeptide of the sequence LNCRHNTAGRHCHYCK (SEQ ID NO: 22);
   (13) a polypeptide of an amino acid sequence shown in SEQ ID NO: 28;
   (14) a polypeptide composed of a partial sequence of the polypeptide (13) and containing a region of amino acid positions 370 to 377 in the sequence of SEQ ID NO: 28:
   (15) a polypeptide having 95% or more identity to the amino acid sequence shown in SEQ ID NO: 28, which is represented by an amino acid sequence identical to the amino acid sequence shown in SEQ ID NO: 28 except that one or more amino acids are substituted, deleted, inserted, and/or added in any region outside a region of amino acid positions 370 to 377;
   (16) a polypeptide composed of a partial sequence of the polypeptide (15) and containing the region of amino acid positions 370 to 377;
   (17) a polypeptide of the sequence LCECRDVLSCYYITDT (SEQ ID NO: 4);
   (18) a polypeptide of the sequence CPCVNGATRHRPTSLC (SEQ ID NO: 8);
   (19) a polypeptide of the sequence LVPWLRYAY (SEQ ID NO: 17);
   (20) a polypeptide of the sequence NXRHNTAG (SEQ ID NO: 75) (X represents an amino acid except for cysteine and alanine);
   (21) a polypeptide of the sequence LNXRDNTR (SEQ ID NO: 76) (X represents an amino acid except for cysteine and alanine);
   (22) a polypeptide of the sequence LNCRHNTAG (SEQ ID NO: 77);
   (23) a polypeptide of the sequence SNCRINTFRTVPIEQK (SEQ ID NO: 78);
   (24) a polypeptide of the sequence SNCRINTFR (SEQ ID NO: 79);
   (25) a polypeptide of the sequence TSNCRINTFR (SEQ ID NO: 80);
   (26) a polypeptide of an amino acid sequence shown in SEQ ID NO: 82;
   (27) a polypeptide composed of a partial sequence of the polypeptide (26) and containing a region of amino acid positions 456 to 464 in the sequence of SEQ ID NO: 82;
   (28) a polypeptide having 95% or more identity to the amino acid sequence shown in SEQ ID NO: 82, which is represented by an amino acid sequence identical to the amino acid sequence shown in SEQ ID NO: 82 except that one or more amino acids are substituted, deleted, inserted, and/or added in any region outside a region of amino acid positions 456 to 464;
   (29) a polypeptide composed of a partial sequence of the polypeptide (28) and containing the region of amino acid positions 456 to 464;
   (30) a polypeptide having an identity of 80% or more and less than 100% to any of (1) to (14) and (17) to (27).
[6] The anti-hepatitis B virus agent according to [5], wherein the polypeptide (15) has 98% or more identity to SEQ ID NO: 28 and the polypeptide (28) has 98% or more identity to SEQ ID NO: 82.
[7] The anti-hepatitis B virus agent according to [5], containing at least one polypeptide selected from said (1) to (13) and (22) to (26) as an active ingredient.
[8] The anti-hepatitis B virus agent according to [5], containing at least one polypeptide selected from said (1) to (12) and (17) to (25) as an active ingredient.
[9] The anti-hepatitis B virus agent according to any one of [5] to [8], wherein the polypeptide is in a form in which the polypeptide is attached to a carrier molecule for delivery into hepatocytes.
[10] The anti-hepatitis B virus agent according to [9], wherein the carrier molecule is an antibody, antibody fragment, or single-chain antibody that binds to the asialoglycoprotein receptor.
[11] The anti-hepatitis B virus agent according to [10], wherein the antibody, antibody fragment, or single-chain antibody has:
   a heavy chain CDR1 containing an amino acid sequence shown in SEQ ID NO: 83, 89, 95, or 101, or an amino acid sequence which is identical thereto except that the amino acid residues are partially substituted and has 80% or more identity thereto,
   a heavy chain CDR2 containing an amino acid sequence shown in SEQ ID NO: 84, 90, 96, or 102, or an amino acid sequence which is identical thereto except that the amino acid residues are partially substituted and has 80% or more identity thereto,
   a heavy chain CDR3 containing an amino acid sequence shown in SEQ ID NO: 85, 91, 97, or 103, or an amino acid sequence which is identical thereto except that the amino acid residues are partially substituted and has 80% or more identity thereto,
   a light chain CDR1 containing an amino acid sequence shown in SEQ ID NO: 86, 92, 98, or 104, or an amino acid sequence which is identical thereto except that the amino acid residues are partially substituted and has 80% or more identity thereto,
   a light chain CDR2 containing an amino acid sequence shown in SEQ ID NO: 87, 93, 99, or 105, or an amino acid sequence which is identical thereto except that the amino acid residues are partially substituted and has 80% or more identity thereto, and
   a light chain CDR3 containing an amino acid sequence shown in SEQ ID NO: 88, 94, 100, or 106, or an amino acid sequence which is identical thereto except that the amino acid residues are partially substituted and has 80% or more identity thereto.
[12] The anti-hepatitis B virus agent according to [10] or [11], wherein the antibody, antibody fragment, or single-chain antibody is attached to the polypeptide via a cleavage sequence that is cleaved by an endogenous enzyme.
[13] The anti-hepatitis B virus agent according to any one of [5] to [12], wherein the polypeptide is in a form in which the polypeptide is attached to a cell membrane penetration-enhancing molecule.
[14] The anti-hepatitis B virus agent according to [13], wherein the cell membrane penetration-enhancing molecule is a polypeptide of an amino acid sequence shown in SEQ ID NO: 107 or 108.
[15] The anti-hepatitis B virus agent according to any one of [5] to [14], wherein the polypeptide is in a form in which a nuclear localization signal is attached to the polypeptide.
[16] Use of at least one selected from the following polypeptides (1) to (30) as a LIPG-binding peptide:
   (1) a polypeptide of the sequence NCRHNTAG (SEQ ID NO: 25);
   (2) a polypeptide of the sequence NARHNTAG (SEQ ID NO: 26);
   (3) a polypeptide of the sequence LNCRDNTR (SEQ ID NO: 23);
   (4) a polypeptide of the sequence LNARDNTR (SEQ ID NO: 24);
   (5) a polypeptide of the sequence LNCRDNTRPVMSAMTC (SEQ ID NO: 1);
   (6) a polypeptide of the sequence IRNVNHSDH (SEQ ID NO: 9);
   (7) a polypeptide of the sequence LNVGYVFYP (SEQ ID NO: 18);
   (8) a polypeptide of the sequence SLYTGFRAH (SEQ ID NO: 15);
   (9) a polypeptide of the sequence RCRQSWNTM (SEQ ID NO: 14);
   (10) a polypeptide of the sequence GADLRRGCC (SEQ ID NO: 11);
   (11) a polypeptide of the sequence RKSGGVCLNCRHNTAG (SEQ ID NO: 21);
   (12) a polypeptide of the sequence LNCRHNTAGRHCHYCK (SEQ ID NO: 22);
   (13) a polypeptide of an amino acid sequence shown in SEQ ID NO: 28;
   (14) a polypeptide composed of a partial sequence of the polypeptide (13) and containing a region of amino acid positions 370 to 377 in the sequence of SEQ ID NO: 28;
   (15) a polypeptide having 95% or more identity to the amino acid sequence shown in SEQ ID NO: 28, which is represented by an amino acid sequence identical to the amino acid sequence shown in SEQ ID NO: 28 except that one or more amino acids are substituted, deleted, inserted, and/or added in any region outside a region of amino acid positions 370 to 377;
   (16) a polypeptide composed of a partial sequence of the polypeptide (15) and containing the region of amino acid positions 370 to 377;
   (17) a polypeptide of the sequence LCECRDVLSCYYITDT (SEQ ID NO: 4);
   (18) a polypeptide of the sequence CPCVNGATRI IRPTSLC (SEQ ID NO: 8);
   (19) a polypeptide of the sequence LVPWLRYAY (SEQ ID NO: 17);
   (20) a polypeptide of the sequence NXRHNTAG (SEQ ID NO: 75) (X represents an amino acid except for cysteine and alanine);
   (21) a polypeptide of the sequence LNXRDNTR (SEQ ID NO: 76) (X represents an amino acid except for cysteine and alanine);
   (22) a polypeptide of the sequence LNCRHNTAG (SEQ ID NO: 77);
   (23) a polypeptide of the sequence SNCRINTFRTVPIEQK (SEQ ID NO: 78);
   (24) a polypeptide of the sequence SNCRINTFR (SEQ ID NO: 79);
   (25) a polypeptide of the sequence TSNCRINTFR (SEQ ID NO: 80);
   (26) a polypeptide of an amino acid sequence shown in SEQ ID NO: 82;
   (27) a polypeptide composed of a partial sequence of the polypeptide (26) and containing a region of amino acid positions 456 to 464 in the sequence of SEQ ID NO: 82;
   (28) a polypeptide having 95% or more identity to the amino acid sequence shown in SEQ ID NO: 82, which is represented by an amino acid sequence identical to the amino acid sequence shown in SEQ ID NO: 82 except that one or more amino acids are substituted, deleted, inserted, and/or added in any region outside a region of amino acid positions 456 to 464;
   (29) a polypeptide composed of a partial sequence of the polypeptide (28) and containing the region of amino acid positions 456 to 464;
   (30) a polypeptide having an identity of 80% or more and less than 100% to any of (1) to (14) and (17) to (27).
[17] A LIPG-binding peptide consisting of at least one polypeptide selected from the following polypeptides (1) to (30):
   (1) a polypeptide of the sequence NCRHNTAG (SEQ ID NO: 25);
   (2) a polypeptide of the sequence NARHNTAG (SEQ ID NO: 26);
   (3) a polypeptide of the sequence LNCRDNTR (SEQ ID NO: 23);
   (4) a polypeptide of the sequence LNARDNTR (SEQ ID NO: 24);
   (5) a polypeptide of the sequence LNCRDNTRPVMSAMTC (SEQ ID NO: 1);
   (6) a polypeptide of the sequence IRNVNHSDH (SEQ ID NO: 9);
   (7) a polypeptide of the sequence LNVGYVFYP (SEQ ID NO: 18);
   (8) a polypeptide of the sequence SLYTGFRAH (SEQ ID NO: 15);
   (9) a polypeptide of the sequence RCRQSWNTM (SEQ ID NO: 14);
   (10) a polypeptide of the sequence GADLRRGCC (SEQ ID NO: 11);
   (11) a polypeptide of the sequence RKSGGVCLNCRHNTAG (SEQ ID NO: 21);
   (12) a polypeptide of the sequence LNCRHNTAGRHCHYCK (SEQ ID NO: 22);
   (13) a polypeptide of an amino acid sequence shown in SEQ ID NO: 28;
   (14) a polypeptide composed of a partial sequence of the polypeptide (13) and containing a region of amino acid positions 370 to 377 in the sequence of SEQ ID NO: 28;
   (15) a polypeptide having 95% or more identity to the amino acid sequence shown in SEQ ID NO: 28, which is represented by an amino acid sequence identical to the amino acid sequence shown in SEQ ID NO: 28 except that one or more amino acids are substituted, deleted, inserted, and/or added in any region outside a region of amino acid positions 370 to 377;
   (16) a polypeptide composed of a partial sequence of the polypeptide (15) and containing the region of amino acid positions 370 to 377;
   (17) a polypeptide of the sequence LCECRDVLSCYYITDT (SEQ ID NO: 4);
   (18) a polypeptide of the sequence CPCVNGATRHRPTSLC (SEQ ID NO: 8);
   (19) a polypeptide of the sequence LVPWLRYAY (SEQ ID NO: 17);
   (20) a polypeptide of the sequence NXRHNTAG (SEQ ID NO: 75) (X represents an amino acid except for cysteine and alanine);
   (21) a polypeptide of the sequence LNXRDNTR (SEQ ID NO: 76) (X represents an amino acid except for cysteine and alanine);
   (22) a polypeptide of the sequence LNCRHNTAG (SEQ ID NO: 77);
   (23) a polypeptide of the sequence SNCRINTFRTVPIEQK (SEQ ID NO: 78);
   (24) a polypeptide of the sequence SNCRINTFR (SEQ ID NO: 79);
   (25) a polypeptide of the sequence TSNCRINTFR (SEQ ID NO: 80);
   (26) a polypeptide of an amino acid sequence shown in SEQ ID NO: 82;
   (27) a polypeptide composed of a partial sequence of the polypeptide (26) and containing a region of amino acid positions 456 to 464 in the sequence of SEQ ID NO: 82;
   (28) a polypeptide having 95% or more identity to the amino acid sequence shown in SEQ ID NO: 82, which is represented by an amino acid sequence identical to the amino acid sequence shown in SEQ ID NO: 82 except that one or more amino acids are substituted, deleted, inserted, and/or added in any region outside a region of amino acid positions 456 to 464;
   (29) a polypeptide composed of a partial sequence of the polypeptide (28) and containing the region of amino acid positions 456 to 464;
   (30) a polypeptide having an identity of 80% or more and less than 100% to any of (1) to (14) and (17) to (27).

### EFFECT OF THE INVENTION

The present invention provides for the first time anti-HBV agents targeting LIPG. An anti-HBV agent according to the present invention can reduce HBV DNA and cccDNA in cells and, therefore, is expected to completely cure hepatitis B. A substance that binds to LIPG to inhibit the function thereof exerts an anti-HBV effect by inhibiting LIPG-mediated HBV entry into cells even when not bound to a carrier molecule for delivery into hepatocytes, and can exert a stronger anti-HBV effect when bound to a carrier molecule for delivery into hepatocytes. Of the polypeptides (1) to (12), (17) to (25), (14), (16), (29), and (30) described above, smaller polypeptides can be easily prepared by chemical synthesis, and the chemically synthesized polypeptides are unlikely to be contaminated with host-cell materials, unlike those produced by genetic engineering, and have advantages as pharmaceutical products.

### BRIEF DESCRIPTION OF THE DRAWINGS

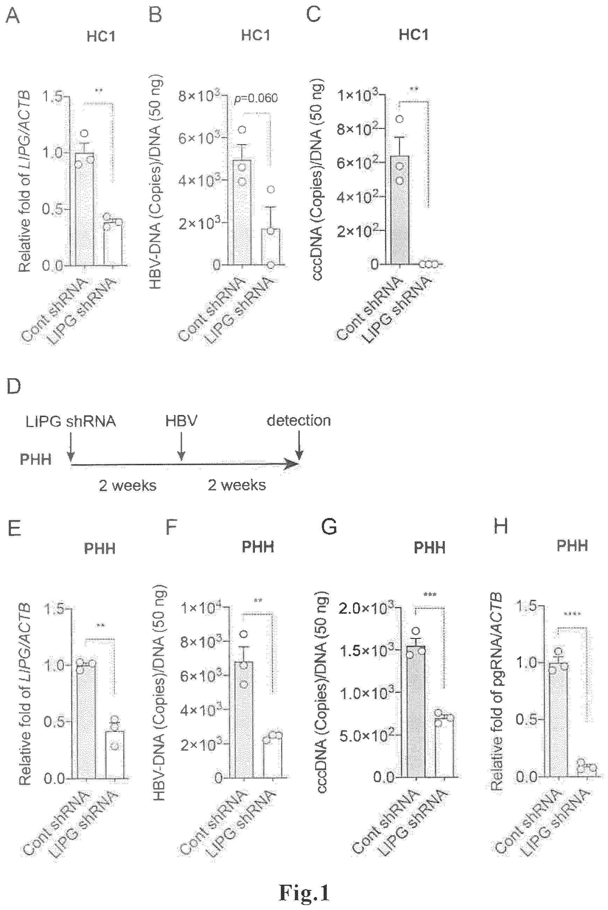

[Fig. 1] Knockdown of LIPG expression negatively regulates the life cycle of HBV. (A) The results of qRT-PCR analysis of LIPG mRNA levels in HCl cells transfected with Cont shRNA and HCl cells transfected with LIPG shRNA. The results are values normalized to the ACTB level. (B) The results of qRT-PCR analysis of HBV-DNA levels in HCl cells transfected with Cont shRNA and I-ICl cells transfected with LIPG shRNA at 7 days after HBV infection. (C) The results of qRT-PCR analysis of cccDNA levels in HCl cells transfected with Cont shRNA and HCl cells transfected with LIPG shRNA at 7 days after HBV infection. (D) A schematic diagram indicating the experiment plan. (E) The results of qRT-PCR analysis of LIPG mRNA levels in PHH cells transfected with Cont shRNA and PHH cells transfected with LIPG shRNA. The results are values normalized to the result of ACTB. (F) The results of qRT-PCR analysis of HBV-DNA levels in PHH cells transfected with Cont shRNA and PHH cells transfected with LIPG shRNA (at 2 weeks after HBV infection). (G) The results of qRT-PCR analysis of cccDNA levels in PHH cells transfected with Cont shRNA and PHH cells transfected with LIPG shRNA at 2 weeks after HBV infection. (H) The results of qRT-PCR of pgRNA transcript levels in PHH cells transfected with Cont shRNA and PHH cells transfected with LIPG shRNA at 2 weeks after HBV infection. The results are values normalized to the result of ACTB. The data shown is the mean ± SEM of three independent experiments. A statistical test employed is unpaired two-tailed *t-*test. ****: p < 0.0001, ***: p < 0.001, **: p < 0.01.

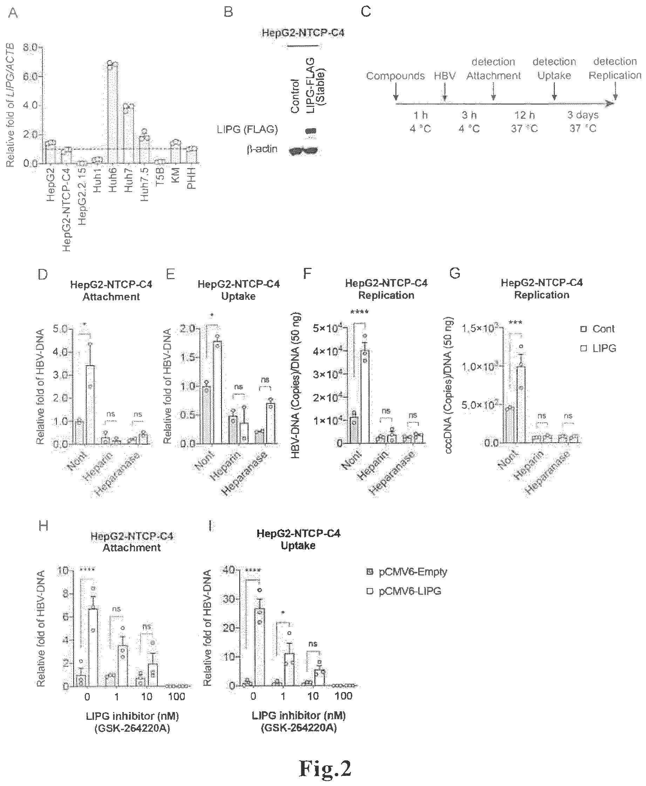

[Fig. 2] LIPG positively regulates the life cycle of HBV in HepG2-NTCP cells. (A) The results of qRT-PCR analysis of LIPG mRNA levels in HepG2, HepG2-NTCP-C4, HepG2.2.15, Huh1, Huh6, Huh7, T5B, HCl, and PHH cells. The results are values normalized to the result of ACTB. (B) Immunoblotting analyses of FLAG-tagged LIPG and β-actin in HepG2-NTCP-C4 cells (Control: HepG2-NTCP-C4 cells transfected with empty vector) and HepG2-NTCP-C4-LIPG cells (LIPG-FLAG). (C) A schematic diagram indicating the experiment plan. (D) qPCR analysis of HBV-DNA levels in HepG2-NTCP-C4 cells (Cont) and HepG2-NTCP-C4-LIPG cells (LIPG) at 3 hours after HBV inoculation at 4°C in the presence or absence of heparin or heparanase. (E) qPCR analysis of HBV-DNA levels in HepG2-NTCP-C4 cells and HepG2-NTCP-C4-LIPG cells at 12 hours after being recultured at 37°C. (F) qPCR analysis of HBV-DNA levels in HepG2-NTCP-C4 cells and HepG2-NTCP-C4-LIPG cells at 3 days after being recultured at 37°C. (G) qPCR analysis of cccDNA levels in HepG2-NTCP-C4 cells and HepG2-NTCP-C4-LIPG cells at 3 days after being recultured at 37°C. (H) qPCR analysis of HBV-DNA levels in HepG2-NTCP-C4 cells and LIPG-overexpressing HepG2-NTCP-C4 cells at 3 hours after HBV inoculation at 4°C in the presence of a LIPG inhibitor. (I) qPCR analysis of HBV-DNA levels in HepG2-NTCP-C4 cells and LIPG-overexpressing HepG2-NTCP-C4 cells at 12 hours after being recultured at 37°C. The data shown was the mean ± SEM of three independent experiments (A, F, G, H, and I) or two independent experiments (D and E). Statistical tests employed are two-way analysis of variance and Tukey's multiple comparison test. ****: p < 0.0001, ***: p < 0.001, *: p < 0.05, no significance (ns).

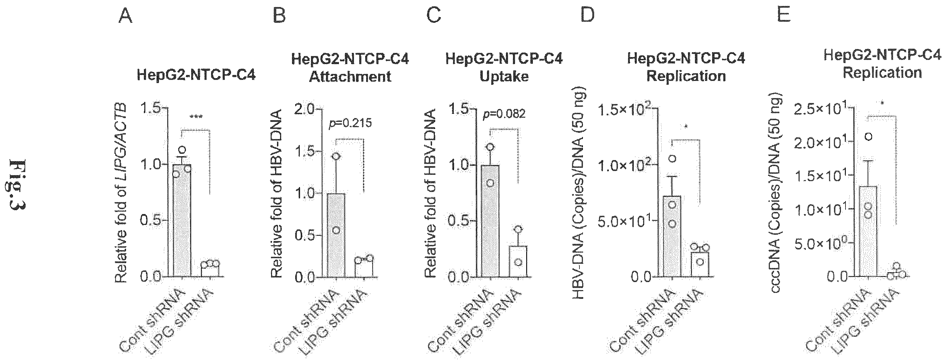

[Fig. 3] Knock-down of the LIPG gene reduces the attachment, uptake, and replication of HBV in HepG2-NTCP-C4 cells. (A) The results of qRT-PCR analysis of LIPG mRNA levels in HepG2-NTCP-C4 cells transfected with Cont shRNA and HepG2-NTCP-C4 cells transfected with LIPG shRNA. The results are values normalized to the result of ACTB. (B) qPCR analysis of HBV-DNA levels in HepG2-NTCP-C4 cells transfected with Cont shRNA and HepG2-NTCP-C4 cells transfected with LIPG shRNA at 3 hours after HBV inoculation at 4°C. (C) qPCR analysis of HBV-DNA levels in HepG2-NTCP-C4 cells transfected with Cont shRNA and HepG2-NTCP-C4 cells transfected with LIPG shRNA after being recultured at 37°C. (D) qPCR analysis of HBV-DNA levels in HepG2-NTCP-C4 cells transfected with Cont shRNA and HepG2-NTCP-C4 cells transfected with LIPG shRNA at 3 days after being recultured at 37°C. (E) qPCR analysis of cccDNA levels in HepG2-NTCP-C4 cells transfected with Cont shRNA and HepG2-NTCP-C4 cells transfected with LIPG shRNA at 3 days after being recultured at 37°C. The data shown is the mean ± SEM of two or three independent experiments. A statistical test employed is unpaired two-tailed t-test. ***: p < 0.001, *: p < 0.05.

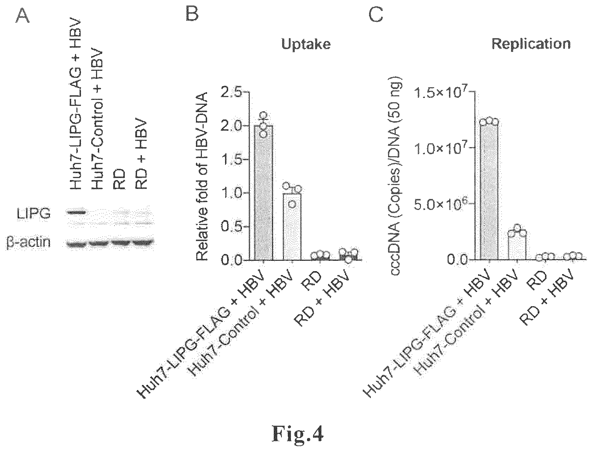

[Fig. 4] HBV uptake and replication are not supported in RD cells. (A) Immunoblotting analyses of LIPG and β-actin in Huh7 cells infected with HBV (Hulz7-Control + HBV), Huh7-LIPG cells infected with HBV (Huh7-LIPG-FLAG + HBV), RD cells not inoculated with HBV (RD) and RD cells inoculated with HBV (RD + HBV). (B) qPCR analysis of HBV-DNA levels in Huh7 cells infected with HBV, Huh7-LIPG cells infected with HBV, RD cells not inoculated with HBV, and RD cells inoculated with HBV at 12 hours after HBV inoculation. (C) qPCR analysis of cccDNA levels in Huh7 cells infected with HBV, Huh7-LIPG cells infected with HBV, RD cells not inoculated with HBV, and RD cells inoculated with HBV at 3 days after HBV inoculation. The data shown is the mean ± SEM of three independent experiments.

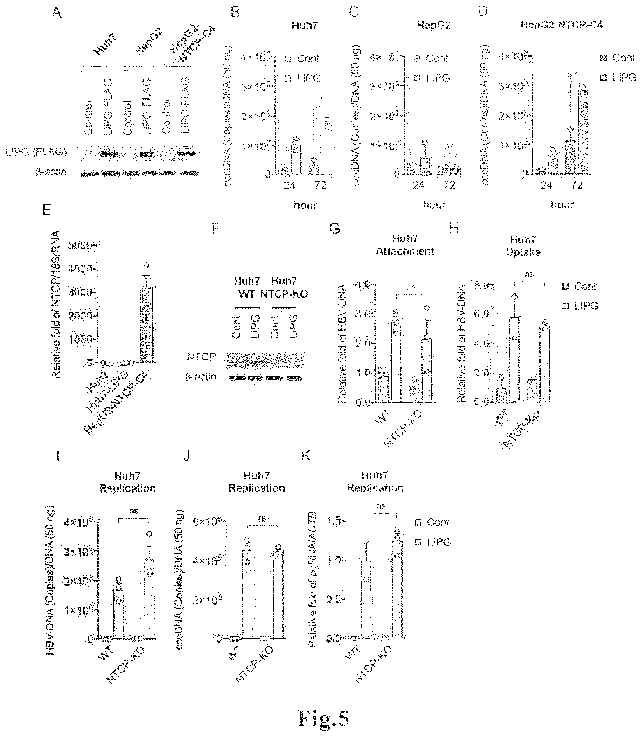

[Fig. 5] LIPG supports the life cycle of HBV in an NTCP-independent manner in Huh7 cells. (A) Immunoblotting analyses of Flag-tagged LIPG and β-actin in Huh7 cells (Control), Huh7-LIPG cells (LIPG-FLAG). HepG2 cells (Control), HepG2-LIPG cells (LIPG-FLAG), HepG2-NTCP-C4 cells (Control), HepG2-NTCP-C4-LIPG cells (LIPG-FLAG). (B) qPCR analysis of cccDNA levels in Huh7 cells (Cont) and Huh7-LIPG cells (LIPG) at 24 and 72 hours after HBV infection. (C) qPCR analysis of cccDNA levels in HepG2 cells (Cont) and HepG2-LIPG cells (LIPG) at 24 and 72 hours after HBV infection. (D) qPCR analysis of cccDNA levels in HepG2-NTCP-C4 cells (Cont) and HepG2-NTCP-C4-LIPG cells (LIPG) at 24 and 72 hours after HBV infection. (E) qRT-PCR analysis of LIPG mRNA levels in Huh7, Hul7-LIPG, and HepG2-NTCP-C4 cells. The results are values normalized to the 18S rRNA level. (F) Immunoblotting analyses of NTCP and β-actin in Huh7-WT and Huh7-NTCP-KO cells (under control or under overexpression of LIPG). (G) qPCR analysis of HBV-DNA levels in Huh7-WT and Huh7-NTCP-KO cells (under control or under overexpression of LIPG, at 3 hours after HBV inoculation at 4°C). (H) qPCR analysis of HBV-DNA levels in Huh7-WT and Huh7-NTCP-KO cells (under control or under overexpression of LIPG, at 12 hours after being reincubated at 37°C). (1) qPCR analysis of HBV-DNA levels in Huh7-WT and Huh7-NTCP-KO cells (under control or under overexpression of LIPG, at 3 days after being reincubated at 37°C). (J) qPCR analysis of cccDNA levels in Huh7-WT and Huh7-NTCP-KO cells (under control or under overexpression of LIPG, at 3 days after being reincubated at 37°C). (K) qRT-PCR analysis of pgRNA transcription levels in Huh7-WT and Huh7-NTCP-KO cells under control or under overexpression of LIPG at 3 days after being recultured at 37°C. The results are values normalized to the result of ACTB. The data shown is the mean ± SEM of three independent experiments (G, I, J, and K) or two independent experiments (B, C, D, and H). Statistical tests employed are two-way analysis of variance and Tukey's multiple comparison test. *: p < 0.05. no significance (ns).

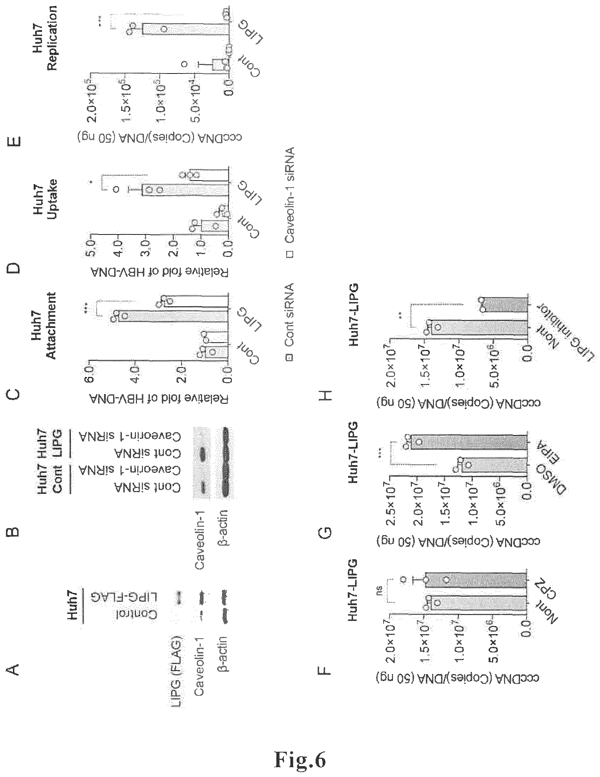

[Fig. 6] LIPG contributes to caveolae-dependent HBV uptake. (A) Immunoblotting analyses of flag-LIPG, caveolin-1, and β-actin in Huh7 cells (Control) and Huh7-LIPG cells (LIPG-FLAG). (B) Immunoblotting analyses of Caveolin-1 and β-actin in Huh7-Cont cells and Huh7-LIPG cells. (C) qPCR analysis of HBV-DNA levels in I-Iuh7 cells (Cont) and Huh7-LIPG cells (LIPG). (D) qPCR analysis of HBV-DNA levels in Huh7 cells (Cont) and Huh7-LIPG cells (LIPG) transfected with Cont siRNA or LIPG siRNA at 12 hours after being recultured at 37°C. (E) qPCR analysis of cccDNA levels in Huh7 cells (Cont) and Huh7-LIPG cells (LIPG) transfected with Cont siRNA or LIPG siRNA at 3 days after being recultured at 37°C. (F) qPCR analysis of cccDNA level in Huh7-LIPG cells infected with HBV at 3 days after chlorpromazine (CPZ) treatment. (G) qPCR analysis of cccDNA level in Huh7-LIPG cells infected with HBV at 3 days after 5-(N-ethyl-N-isopropyl)amiloride (EIPA) treatment. (H) qPCR analysis of cccDNA level in Huh7-LIPG cells infected with HBV at 3 days after treatment with a LIPG inhibitor. The data shown is the mean ± SEM of two or three independent experiments. Statistical tests employed are two-way analysis of variance and Tukey's multiple comparison test (C, D, and E) or unpaired two-tailed *t*-test (F, G, and H). ***: p < 0.001, **: p < 0.01, *: p < 0.05, no significance (ns).
[Fig. 7] LIPG increases vimentin expression. (A) Immunoblotting analyses of FLAG-LIPG, vimentin. β-actin in Huh7 cells (Control) and Huh7-LIPG cells (LIPG-FLAG). (B) Immunoblotting analyses of FLAG (LIPG), vimentin, Pan-cadherin, E-cadherin, and β-actin in Huh7 cells (Control), Huh7-LIPG cells (LIPG-FLAG), HepG2 cells (Control). HepG2-LIPG cells (LIPG-FLAG), HepG2-NTCP-C4 cells (Control), and HepG2-NTCP-C4-LIPG cells (LIPG-FLAG).

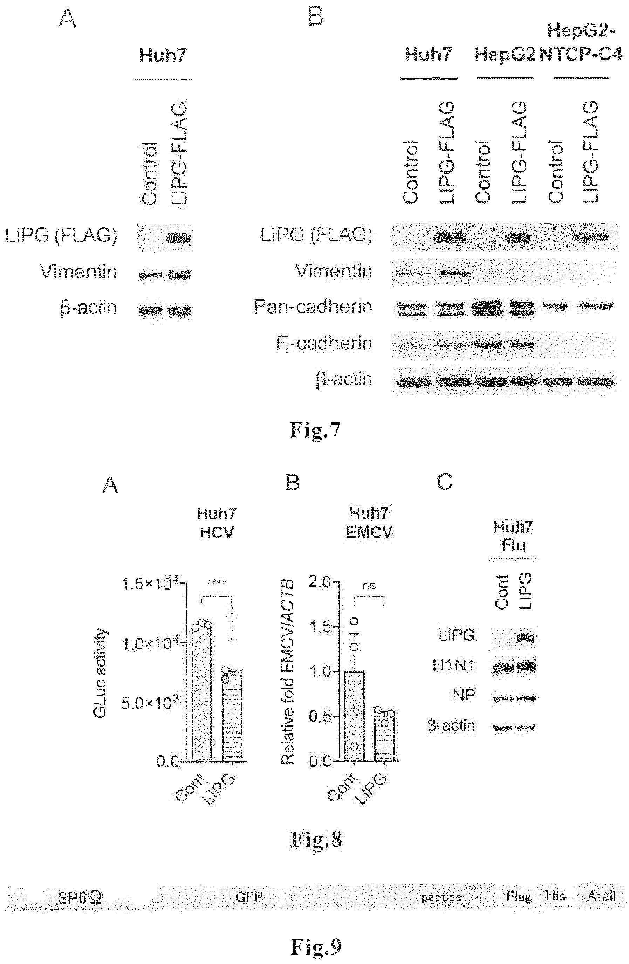

[Fig. 8] LIPG has no effect on clathrin-dependent viral endocytosis. (A) GLuc activity in Huh7 cells (Cont) and Huh7-LIPG cells (LIPG) at 3 days after HCV infection (at multiplicity of infection (MOI) of 1.0). (B) qRT-PCR analysis of EMCV levels in I-Iuh7 cells (Cont) and Huh7-LIPG cells (LIPG) at 12 hours after EMCV infection (MOI 1.0). The results are values normalized to the ACTB level. (C) Immunoblotting analyses of LIPG, influenza A H1N1, influenza A nucleoprotein (NP), and β-actin in Huh7 cells (Cont) and Huh7-LIPG cells (LIPG) at 12 hours after Flu infection (MOI 1.0). The data shown is the mean ± SEM of three independent experiments. A statistical test employed is unpaired two-tailed t-test. ****: p < 0.0001, no significance (ns).
[Fig. 9] The design of a DNA library for IVV screening. In the direction where the 5' is upstream, a 5' untranslated region (UTR) containing a SP6 RNA polymerase promoter and a part of the omega sequence of tobacco mosaic virus as a translation enhancer was followed by green fluorescent protein (GFP). A sequence encoding a random peptide of 16 amino acids or 9 amino acids was employed as the peptide portion. A Flag tag, a His tag and Atail were further added.
[Fig. 10] A schematic diagram of the binding of endothelial lipase LIPG and heparan sulfate proteoglycan to HBV virus. The present research revealed an important role of endothelial lipase LIPG and heparan sulfate proteoglycans (HSPGs) in HBV entry. In addition, LIPG and HSPGs are known to bind to each other, and LIPG is known to bind to heparin in the form of a homodimer (Gastroenterology 2014; 147: 48, and PLoS ONE, 2013, 8(3): e55716). Some peptides that bind to the binding regions of LIPG and HSPGs in this portion are thought to have inhibitory activity against HBV entry.
[Fig. 11] The design of a biotinylated LIPG serving as a bait. Two copies of the heparin-binding region of LIPG were directly linked in tandem, followed by a flexible glycine-serine linker and a biotinylation sequence at the end.
[Fig. 12] Vector construction for expression of biotinylated LIPG-heparin binding sites. A vector containing a sequence of the full-length LIPG gene fused with a biotinylation sequence, a Flag-tag sequence, and a His-tag sequence was constructed, and Fragment 1, Fragment 2, Fragment 3, and Fragment 4 were then produced from the vector. Finally, a final form of a bait gene was constructed using these four fragments by in-fusion cloning.

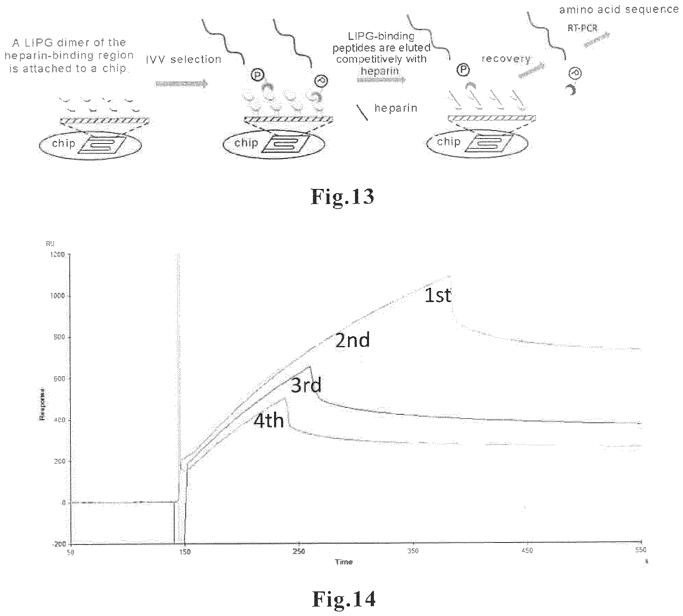

[Fig. 13] A schematic diagram of an experiment for selecting LIPG-binding peptides. A dimer of the heparin-binding region of LIPG was attached to a sensor chip SA, and random peptide IVV molecules were then bound to the resulting chip. Unbound peptides were washed away well with a buffer. Heparin was then added to recover LIPG-binding peptides by competitive elution. The recovered random peptide IVV molecules were recovered in the form of DNA by RT-PCR and subjected to the next round of selection. The DNA from the final round of selection was sequenced to determine the amino acid sequence of the random peptide.
[Fig. 14] Biacore sensorgrams from an experiment for selection of LIPG-binding peptides. The Biacore sensorgrams from the first to the fourth round of the experiment for selection of LIPG-binding peptides are merged into one.
[Fig. 15] The number of PCR cycles and the intensities of the DNA bands in the RT-PCR of the experiment for selection of LIPG-binding peptides. Aliquots of 5 µl of PCR product were electrophoresed on 1.3% agarose gel, and the absorption of ethidium bromide was measured by a MOLECULAR IMAGER FX (Bio-rad) to determine the number of PCR cycles.
[Fig. 16] A pull-down assay of LIPH4 peptides against LIPG. The full length of LIPG (500 amino acids) was used as a bait for the pull-down assay. Plasmids carrying a sequence encoding a LIPG-binding peptide fused with a secretion signal were prepared and then transfected into 293T cells. The culture supernatant was added directly to the bait to pull-down peptides, and the results were compared to those obtained using no bait.
[Fig. 17] The anti-HBV activities of LIPG-binding peptides in HepG2-NTCP-C4 cells. A: The timeline of the assay. B: Effect of LIPG-binding peptides on the HBV-DNA copy number. C: Effect of LIPG-binding peptides on the cccDNA copy number.
[Fig. 18] The anti-HBV activities of LIPG-binding peptides in PXB cells. A: Effect of LIPG-binding peptides on the HBV-DNA copy number. B: Effect of LIPG-binding peptides on the cccDNA copy number.
[Fig. 19] The anti-HBV activities of LIPG-binding peptides in PXB cells. A: The timeline of the assay. B: Effect of LIPH4-23s on the HBV-DNA copy number. C: Effect of LIPH4-23s on the cccDNA copy number. D: Effect of LIPH4-NTNBs on the HBV-DNA copy number. E: Effect of LIPH4-NTNBs on the cccDNA copy number.
[Fig. 20] The ratio of copy numbers of HBV-DNA (A) and the ratio of copy numbers of cccDNA (B) in PXB cells between each concentration of two LIPG-binding peptides, LIPH4-NTNBsA and LIPH4-NTNBs. The amino acid sequence of LIPH4-NTNBsA is identical to the amino acid sequence of LIPH4-NTNBs except for the substitution of alanine for cysteine at position 2.
[Fig. 21] The anti-HBV activity of full-length Netrin-1 and the effect of shRNA-mediated knock down of the Neterin-1 gene on HBV in PXB cells. A: Effect of Netrin-1 on the HBV-DNA copy number. B: Effect of Netrin-1 on the cccDNA copy number. C: Effect of shRNA-mediated knock down of the Netrin-1 gene on the HBV-DNA copy number. D: Effect of shRNA-mediated knock down of the Netrin-1 gene on the cccDNA copy number.

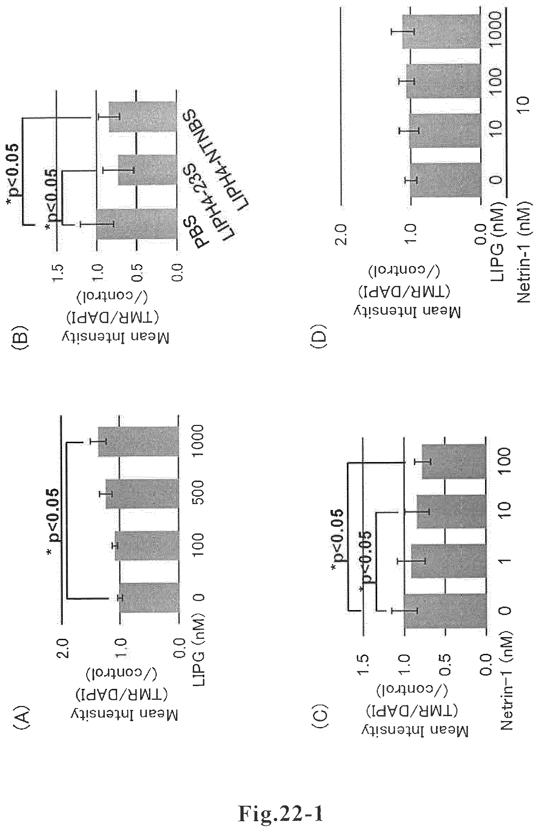

[Fig. 22-1] The effect of LIPG to promote HBV entry into cells and the effect of a LIPG-binding peptide and Netrin-1 to inhibit HBV entry into cells. A: PXB cells were mixed with a purified form of FLAG-LIPG and incubated for 3 hours. The resulting cells were mixed with a preS 1 peptide fluorescently labeled with TMR (tetramethylrhodamine) and incubated for 30 minutes, and then observed with a confocal microscope. The fluorescence of the preS1 peptide was detected, and the fluorescence intensity was normalized to the DAPI fluorescence intensity. *: p < 0.05. B: PXB cells were treated with 100 nM LIPG-binding peptide LIPH4-23S or LIPH4-NTNBS for 15 hours, and a purified form of FLAG-LIPG was added thereto, followed by incubation for 3 hours. The resulting cells were mixed with a preS1 peptide fluorescently labeled with TMR and incubated for 30 minutes, and then observed with a confocal microscope. The fluorescence of the preS1 peptide was detected, and the fluorescence intensity was normalized to the DAPI fluorescence intensity. *: p < 0.05. C: PXB cells were mixed with Netrin-1 and incubated for 3 hours. The resulting cells were mixed with a preS 1 peptide fluorescently labeled with TMR and incubated for 30 minutes, and then observed with a confocal microscope. The fluorescence of the preS 1 peptide was detected, and the fluorescence intensity was normalized to the DAPI fluorescence intensity. *: p < 0.05. D: PXB cells were mixed with Netrin-1 and incubated for 3 hours. The cells were then mixed with FLAG-LIPG and incubated for 3 hours. The resulting cells were mixed with a preS1 peptide fluorescently labeled with TMR and incubated for 30 minutes, and then observed with a confocal microscope. The fluorescence of the preS 1 peptide was detected, and the fluorescence intensity was normalized to the DAPI fluorescence intensity.

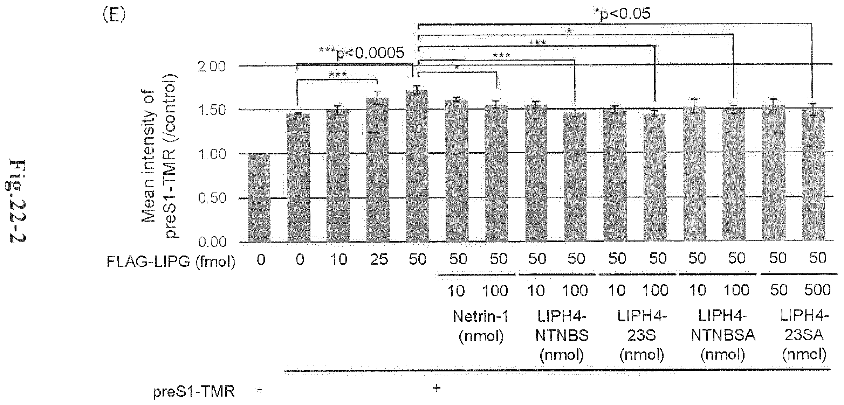

[Fig. 22-2] The effect of LIPG to promote HBV entry into cells and the effect of a LIPG-binding peptide and Netrin-1 to inhibit HBV entry into cells. E: A biotin-labeled form of heparan sulfate was added to a streptavidin-coated 96 well plate and left overnight at 4°C to allow the heparan sulfate to bind to the plate. This plate was washed with TBS, and 10, 25, or 50 fmol of FLAG-LIPG was then added to each well to allow the reaction to proceed at room temperature for 2 hours. After the plate was washed, 10 or 100 nmol of LIPG-binding peptide LIPH4-NTNBS, LIPH4-23S, LIPH4-NTNBSA, or LIPH4-23SA was added to each well to allow the treatment to proceed at room temperature for 1 hour. Subsequently, the plate was washed, and 0.1 nmol of preS1 -TMR was added to each well to allow the treatment to proceed at room temperature for another 1 hour. The plate was washed and then placed in a plate reader to measure the fluorescence of TMR.
[Fig. 23] The HBV-DNA copy number in the liver of HBV-infected PXB mice administered with a test substance (LIPH4-NTNBs).
[Fig. 24] The cccDNA copy number in the liver of HBV-infected PXB mice administered with a test substance (LIPH4-NTNBs).
[Fig. 25] A histopathological examination (HBsAg immunostaining) of the liver tissue from a HBV-infected PXB mouse administered with a test substance (LIPH4-NTNBs). A: LIPH4-NTNBs 0.1 mg, B: control (PBS).
[Fig. 26] A histopathological examination (I IBcAg immunostaining) of the liver tissue from a HBV-infected PXB mouse administered with a test substance (LIPI 14-NTNBs). A: LIPH4-NTNBs 0.1 mg, B: control (PBS).
[Fig. 27] The design of a biotinylated asialoglycoprotein receptor.
[Fig. 28] The cloned sequences of the extracellular domains of ASGR1 and ASGR2.
[Fig. 29] Constructs for biotinylated asialoglycoprotein receptor.
[Fig. 30] The structure of antibody. (left) The basic structure of IgG antibody. The IgG antibody is composed of two H chains and two L chains connected by S-S bonds and is divided into variable regions (V_{H} and V_{L}), which form an antigen binding site, and other regions called constant regions (C_{H} and C_{L}). The Fc region in the constant region functions as a site for binding to Fc receptors, (right) Complementarity determining regions (CDRs) present in variable regions. Regions other than CDRs are called framework regions.
[Fig. 31] The structure of single-chain antibody (scFv). The C-terminus of VH is connected to the N-terminus of VL via a peptide linker.
[Fig. 32] A scheme for preparing a cDNA library of mouse single-chain antibodies.
[Fig. 33] A scheme for preparing a cDNA library of human single-chain antibodies.
[Fig. 34] An IVV-based experiment to select ASGR-binding single-chain antibodies.
[Fig. 35] Pull-down assays with ASGR-binding single-chain antibodies (1).
[Fig. 36] Pull-down assays with ASGR-binding single-chain antibodies (2).
[Fig. 37] A putative mode of action of a fusion of a LIPG-binding peptide and an anti-asialoglycoprotein receptor antibody in cells. An intracellular binding partner of LIPG can be, for example, N4BP1.
[Fig. 38] A construct for a fusion of a LIPG-binding peptide and an anti-asialoglycoprotein receptor antibody.
[Fig. 39] A sequence comparison of LIPG-binding peptides used for fusion with an anti-asialoglycoprotein receptor antibody. All of the peptides contain the NCRXNT sequence (SEQ ID NO: 136, X = D, H, or I).
[Fig. 40] A pull-down assay of N4BP1 against LIPG.
[Fig. 41A] The anti-HBV activities of hepatocyte-targeted LIPG-binding peptides in PXB cells. The timeline of the assay.
[Fig. 41B] The anti-HBV activities of hepatocyte-targeted LIPG-binding peptides in PXB cells. Effect of 10M-L23N, 10M-L23SN, 10M-L23SN-S39, and 10M-LNTSN on the HBV-DNA copy number.
[Fig. 41C] The anti-HBV activities of hepatocyte-targeted LIPG-binding peptides in PXB cells. Effect of 10M-L23N, 10M-L23SN, 10M-L23SN-S39 and 10M-LNTSN on the cccDNA copy number.
[Fig. 42] The anti-HBV activities of hepatocyte-targeted LIPG-binding peptides in PXB cells (evaluated in the presence of 4% PEG). (A) The timeline of the assay. (B) Effect of the hepatocyte-targeted LIPG-binding peptides 10M-LN4SN, 10M-LN4N. 10M-LLNTSN, and 10M-LLNTSN-S39 on the HBV-DNA copy number. (C) Effect of the hepatocyte-targeted LIPG-binding peptides 10M-LN4SN, 10M-LN4N, 10M-LLNTSN, and 10M-LLNTSN-S39 on the cccDNA copy number.
[Fig. 43] Inhibition of the RNase activity of N4BP1 by LIPG and rescue of the inhibition by LIPH-NTNBs.
[Fig. 44] A sample dosing schedule in an experiment using chimeric mice with human liver cells.
[Fig. 45] The body weight change in mice. A comparison is made between 10M-LNTSN and control (PBS).
[Fig. 46] The measurement of HBsAg levels in blood. A comparison is made between 10M-LNTSN and control (PBS). **: p < 0.01.
[Fig. 47] The measurement of HBeAg levels in blood. A comparison is made between 10M-LNTSN and control (PBS). *: p < 0.05, **: p < 0.01.
[Fig. 48] The measurement of HBcrAg levels in blood. A comparison is made between 10M-LNTSN and control (PBS).
[Fig. 49] The measurement of HBV DNA levels in blood. A comparison is made between 10M-LNTSN and control (PBS).
[Fig. 50] The measurement of h-Alb levels in blood. A comparison is made between 10M-LNTSN and control (PBS).
[Fig. 51] The measurement of ALT levels in blood. A comparison is made between 10M-LNTSN and control (PBS).
[Fig. 52] The measurement of HBV-DNA levels in liver. A comparison is made between 10M-LNTSN and control (PBS). ^{†}p < 0.1.
[Fig. 53] The measurement of cccDNA levels in liver. A comparison is made between 10M-LNTSN and control (PBS). ^{†}p < 0.1.

### MODE FOR CARRYING OUT THE INVENTION

In the present invention, the term "anti-hepatitis B virus (anti-HBV)" includes treatment of HBV infection, prevention of HBV infection, inhibition of HBV proliferation, treatment of hepatitis B, and prevention of hepatitis B. An anti-HBV agent of the present invention can inhibit HBV proliferation in the body (liver) of the patients and treat hepatitis B when administered to hepatitis B patients. In addition, the anti-HBV agent can prevent HBV proliferation in the carriers and prevent the carriers from developing hepatitis B (prevention of hepatitis B) when administered to HBV carriers who are yet to develop hepatitis B. That is, HBV particles are proliferated in liver cells of patients or HBV carriers and then released to the outside of the cells, and the anti-HBV agent of the present invention prevents the HBV particles from re-entering the cells (reinfection), so that the proliferation of HBV DNA in the patients or carriers is suppressed to obtain effects to treat hepatitis B or to prevent the hepatitis B development.

The anti-HBV agent of the present invention contains as an active ingredient a substance that binds to LIPG and inhibits the function(s) of LIPG (hereinafter, the substance may be referred to as LIPG-binding substance; in cases where the substance is a polypeptide, the substance may be specifically referred to as LIPG-binding peptide). The inventors of the present application have found that LIPG is a factor playing an important role in HBV attachment and uptake, and functions as a cofactor that supports HBV entry into cells independently of NTCP, and also contributes to HBV proliferation in cells. The LIPG-binding substance binds to LIPG on the surface of liver cells to inhibit HBV attachment and uptake into cells and, when delivered into liver cells, binds to intracellular LIPG to block any step(s) in the intracellular HBV proliferation process, thereby exerting an anti-HBV activity.

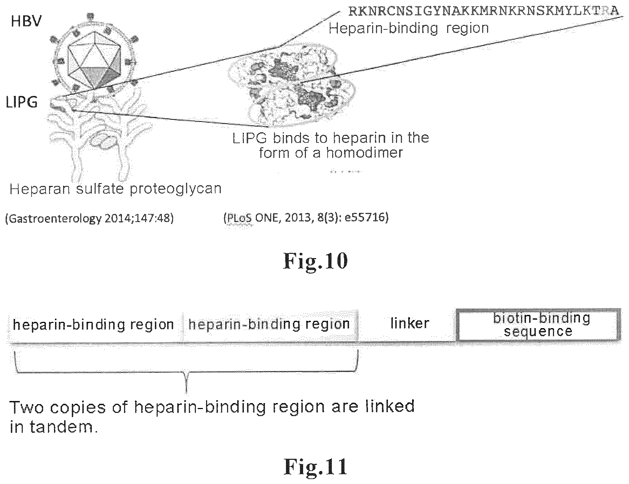

When the focus is placed on preventing attachment to cells and cellular uptake, the LIPG-binding substance can be described as a substance that binds to LIPG to inhibit LIPG-mediated HBV entry into cells. For example, the substance is a substance that binds to the heparin-binding region of LIPG to inhibit binding of LIPG, HSPG, or a LIPG-HSPG complex to HBV. LIPG has two isoforms, and the region of amino acid positions 312 to 340 in the amino acid sequence of LIPG isoform 1 shown in SEQ ID NO: 30 (NCBI Accession No. NP_006024.1) and the region of amino acid positions 238 to 266 in the amino acid sequence of LIPG isoform 2 shown in SEQ ID NO: 138 (NCBI Accession No. NP_001294935.1) are heparin-binding regions. Although it has been known that LIPG has an ability to maintain the genomic DNA or cccDNA of HBV in host cells (Patent Document 1), it was completely unknown at which step and how LIPG functions during the process of HBV infection. The inventors of the present application discovered for the first time that LIPG, HSPG, and HBV particles bind to each other (Fig. 10), and that a substance that binds to the heparin-binding region of LIPG can inhibit binding of LIPG, HSPG, or a LIPG-HSPG complex to HBV, thereby inhibiting HBV entry into host cells, so that HBV proliferation in host cells (in the body, particularly the liver, of HBV-infected patients) is inhibited. Modes of inhibition of binding of LIPG, IISPG_ and HBV to each other (ternary complex formation) on the cell surface include inhibition of binding between LIPG and HBV, inhibition of binding between an HSPG and HBV, and inhibition of binding between a LIPG-HSPG complex and HBV. A substance used as an active ingredient in the present application may be any one of these modes.

Examples of the substance that binds to the heparin-binding region of LIPG and inhibits binding of LIPG, HSPG, or a LIPG-HSPG complex to HBV include an antibody, an antigen-binding fragment thereof, or an aptamer that recognizes and binds to the heparin-binding region of LIPG, and a polypeptide that selectively binds to the heparin-binding region of LIPG.

For example, the inhibition of LIPG function(s) by a LIPG-binding substance in liver cells may be at least one selected from inhibition of binding of LIPG to N4BP1 and inhibition of suppression by LIPG of the RNase activity of N4BP1 (see Example 20 below). It was also first discovered by the present inventors that HBV proliferation is inhibited by LIPG inhibition in liver cells.

For example, the anti-HBV agent of the present invention may contain as an active ingredient at least one polypeptide (LIPG-binding peptide) selected from the polypeptides (1) to (30) below. The names of the polypeptides that are used in the examples below are shown in square brackets ([]).
(1) a polypeptide of the sequence NCRHNTAG (SEQ ID NO: 25) [LIPH4-NTNBs];
(2) a polypeptide of the sequence NARHNTAG (SEQ ID NO: 26) [LIPH4-NTNBsA];
(3) a polypeptide of the sequence LNCRDNTR (SEQ ID NO: 23) [LIPH4-23s];
(4) a polypeptide of the sequence LNARDNTR (SEQ ID NO: 24) [LIPH4-23SA];
(5) a polypeptide of the sequence LNCRDNTRPVMSAMTC (SEQ ID NO: 1) [LIPH4-23];
(6) a polypeptide of the sequence IRNVNHSDH (SEQ ID NO: 9) [LIPH4-74];
(7) a polypeptide of the sequence LNVGYVFYP (SEQ ID NO: 18) [LIPI-I4-15];
(8) a polypeptide of the sequence SLYTGFRAH (SEQ ID NO: 15) [LIPH4-2];
(9) a polypeptide of the sequence RCRQSWNTM (SEQ ID NO: 14) [LIPH4-14];
(10) a polypeptide of the sequence GADLRRGCC (SEQ ID NO: 11) [LIPH4-87];
(11) a polypeptide of the sequence RKSGGVCLNCRHNTAG (SEQ ID NO: 21) [LIPH4-NTNA];
(12) a polypeptide of the sequence LNCRHNTAGRHCHYCK (SEQ ID NO: 22) [I,IPH4-NTNB];
(13) a polypeptide of an amino acid sequence shown in SEQ ID NO: 28;
(14) a polypeptide composed of a partial sequence of the polypeptide (13) and containing a region of amino acid positions 370 to 377 in the sequence of SEQ ID NO: 28;
(15) a polypeptide having 95% or more identity to the amino acid sequence shown in SEQ ID NO: 28, which is represented by an amino acid sequence identical to the amino acid sequence shown in SEQ ID NO: 28 except that one or more amino acids are substituted, deleted, inserted, and/or added in any region outside the region of amino acid positions 370 to 377;
(16) a polypeptide composed of a partial sequence of the polypeptide (15) and containing the region of amino acid positions 370 to 377;
(17) a polypeptide of the sequence LCECRDVLSCYYITDT (SEQ ID NO: 4) [LIPH4-90];
(18) a polypeptide of the sequence CPCVNGA TRIIRPTSLC (SEQ ID NO: 8) [LIPH 4-16];
(19) a polypeptide of the sequence LVPWLRYAY (SEQ ID NO: 17) [LIPH4-12];
(20) a polypeptide of the sequence NXRHNTAG (SEQ ID NO: 75) (X represents an amino acid except for cysteine and alanine);
(21) a polypeptide of the sequence LNXRDNTR (SEQ ID NO: 76) (X represents an amino acid except for cysteine and alanine);
(22) a polypeptide of the sequence LNCRI-INTAG (SEQ ID NO: 77) [LIPH4-LNTNBs];
(23) a polypeptide of the sequence SNCRINTFRTVPIEQK (SEQ ID NO: 78) [LIPH4-N4BP1a];
(24) a polypeptide of the sequence SNCRINTFR (SEQ ID NO: 79) [LIPH4-N4BP1b];
(25) a polypeptide of the sequence TSNCRINTFR (SEQ ID NO: 80) [LIPH4-N4BP1c];
(26) a polypeptide of an amino acid sequence shown in SEQ ID NO: 82;
(27) a polypeptide composed of a partial sequence of the polypeptide (26) and containing a region of amino acid positions 456 to 464 in the sequence of SEQ ID NO: 82;
(28) a polypeptide having 95% or more identity to the amino acid sequence shown in SEQ ID NO: 82, which is represented by an amino acid sequence identical to the amino acid sequence shown in SEQ ID NO: 82 except that one or more amino acids are substituted, deleted, inserted, and/or added in any region outside the region of amino acid positions 456 to 464;
(29) a polypeptide composed of a partial sequence of the polypeptide (28) and containing the region of amino acid positions 456 to 464;
(30) a polypeptide having an identity of 80% or more and less than 100% to any of the polypeptides (1) to (14) and (17) to (27).

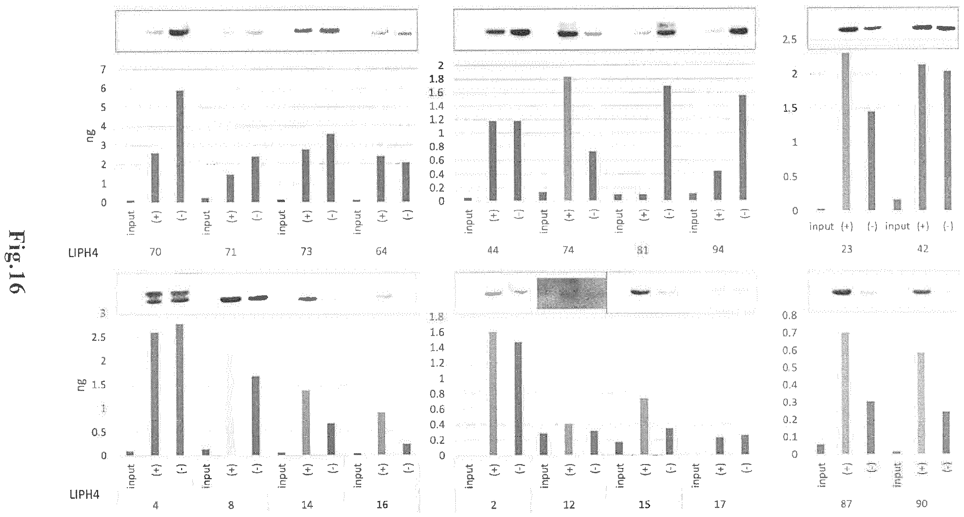

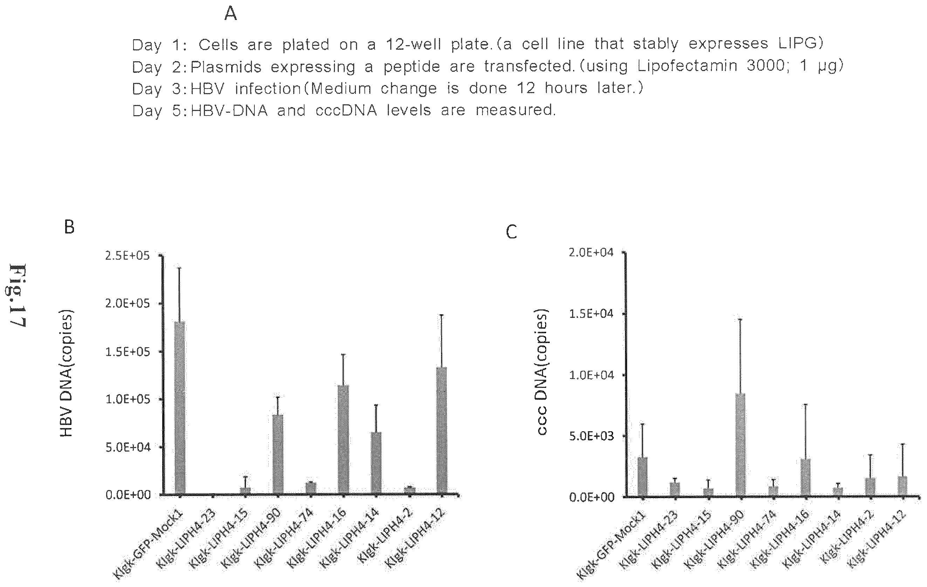

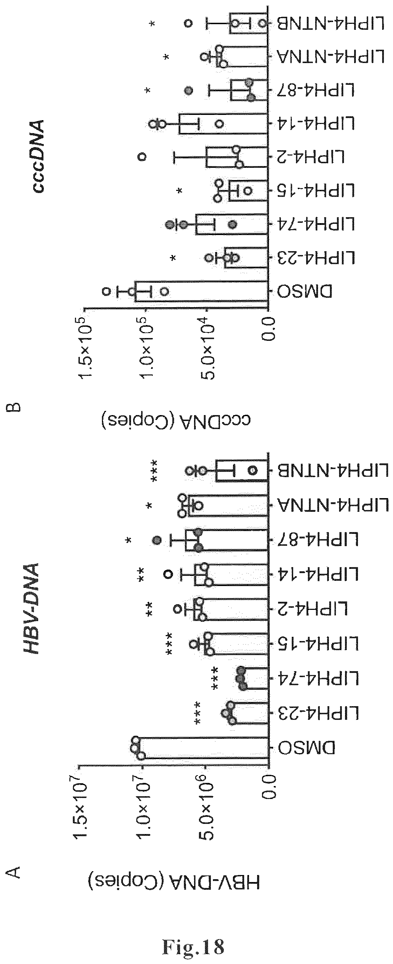

The polypeptides (5) to (10) and (17) to (19) are polypeptides which were identified as peptides binding to LIPG by the IVV method and were further confirmed to be positive for binding to LIPG by a pull-down assay using the full-length form of LIPG as a bait (Fig. 16) in the examples described below. Each of the polypeptides is effective in reducing HBV DNA and HBV cccDNA copy numbers in HepG2-NTCP-C4 or PXB cells (Fig. 17B-C. Fig. 18A-B). Among these polypeptides, the polypeptides (5) to (10) have a particularly strong effect to reduce cccDNA copy number in HBV-infected liver cell lines (Fig. 17C, Fig. 18B).

The polypeptides (11) and (12) are composed of a partial sequence of Netrin-1, which were confirmed to have anti-HBV activity by an experiment using HBV-infected PXB cells in Example 12 below (Fig. 18). Both of the polypeptides have an effect to reduce HBV DNA and cccDNA as strongly as the polypeptides (5) to (10), and can be preferably used as an active ingredient in an anti-HBV agent.

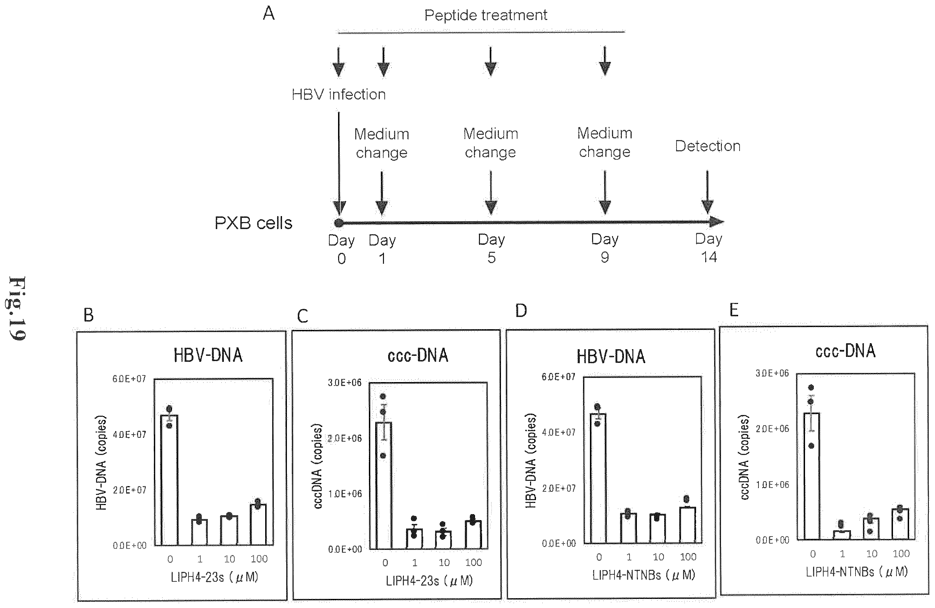

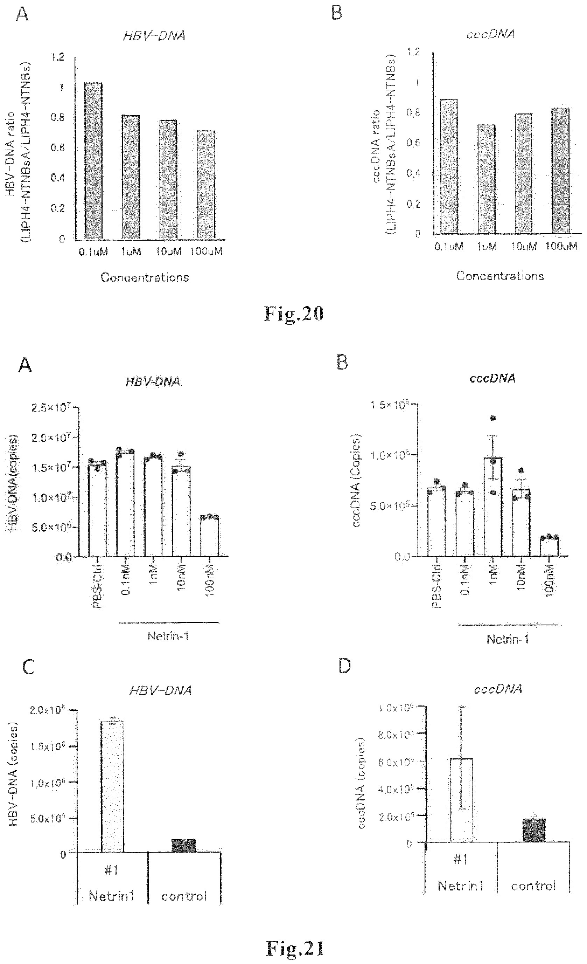

The polypeptides (1) to (4) are designed based on the polypeptides (5) and (12) which have a particularly strong effect to reduce HBV DNA and cccDNA copy numbers in HBV-infected liver cell lines. The polypeptide (1) is composed of amino acid residues 2 to 9 of the polypeptide (12), and the polypeptide (3) is composed of amino acid residues 1 to 8 of the polypeptide (5). Both of the polypeptides have a very strong effect to reduce HBV DNA and cccDNA copy numbers in HBV-infected liver cell lines (Fig. 19). The polypeptide (2) is a polypeptide whose sequence is the same as the polypeptide (1) except that alanine is substituted for cysteine at position 2, and the polypeptide (4) is a polypeptide whose sequence is the same as the polypeptide of (3) except that alanine is substituted for cysteine at position 3. Both of the polypeptides have an effect to reduce HBV DNA and cccDNA copy numbers as strongly as the original polypeptides (2) and (4) (Fig. 20).

The polypeptides (22) to (25) are additionally synthesized LIPG-binding peptides for an intracellular delivery experiment in Example 14. The polypeptide (22) is composed of a partial sequence of Netrin-1 and has the same sequence as the polypeptide (1), LIPH4-NTNBs, except that the N-terminus is extended by one amino acid residue. The polypeptides (23) to (25) is composed of a partial sequence of N4BP1 (NEDD4-binding protein 1) that is synthesized based on the homology between LIPH4-23 and N4BP1. All of the polypeptides have an effect to reduce HBV DNA and cccDNA copy numbers (see Examples 18 and 19) and can be used as anti-HBV agents.

The polypeptides (20) and (21) are a polypeptide whose sequence is the same as the polypeptide (1) except that an amino acid other than alanine is substituted for cysteine at position 2, and a polypeptide whose sequence is the same as the polypeptide (3) except that an amino acid other than alanine is substituted for cysteine at position 3. respectively. As shown by the data in Fig. 20, both (1) NCRIINTAG and (3) LNCRDNTR are not negatively affected by the substitutions for the cysteine residues in terms of anti-HBV activity. Therefore, a polypeptide of the sequence NXRHNTAG (SEQ ID NO: 75) and a polypeptide of the sequence LNXRDNTR (SEQ ID NO: 76) (X is an amino acid other than cysteine and alanine) are considered to have the same level of anti-HBV activity as the polypeptides (1) to (4). Preferred examples of X include, but are not limited to, glycine, isoleucine, leucine, and valine.

The polypeptide (13) is composed of the full-length sequence of Netrin-1. The sequences set forth in SEQ ID NOs: 27 and 28 are the base sequence of the coding region in the sequence of the Netrin-1 gene under NCBI Accession No. NM_004822.3 and the encoded amino acid sequence of Netrin-1 (NP_004813.2), respectively. Since the full-length form of Netrin-1 also reduces HBV DNA and cccDNA copy numbers in HBV-infected liver cells (Fig. 21A, B), the polypeptide (13) is also useful as an anti-HBV agent.

The polypeptide (14) is a polypeptide which is composed of a fragment (a partial sequence) of Netrin-1 (Netrin-1 partial polypeptide) and contains a region of amino acid positions 370 to 377 in the amino acid sequence of Netrin-1 shown in SEQ ID NO: 28. The sequence of the region of amino acid positions 370 to 377 is identical to the amino acid sequence of (1) (SEQ ID NO: 25). It is fully demonstrated in the examples below that a polypeptide consisting of the amino acid sequence of (1) has anti-HBV activity (see Fig. 19 and Example 13). Accordingly, a polypeptide composed of a fragment (a partial sequence) of Netrin-1 can exhibit anti-HBV activity as long as the polypeptide contains the region of amino acid positions 370 to 377 (the amino acid sequence of SEQ ID NO: 25), and the polypeptide is therefore useful as an anti-HBV agent. The polypeptide (14) includes a polypeptide composed of a fragment of (13) containing a region of amino acid positions 362 to 377 (the amino acid sequence of SEQ ID NO: 21) in the amino acid sequence of SEQ ID NO: 28 and a polypeptide composed of a fragment of (13) containing a region of amino acid positions 369 to 384 (the amino acid sequence of SEQ ID NO: 22) in the amino acid sequence of SEQ ID NO: 28.

The polypeptide of (15) is a polypeptide composed of a mutated sequence of Netrin-1. and is specifically a polypeptide having 95% or more identity to the amino acid sequence of Netrin-1 shown in SEQ ID NO: 28, which is represented by an amino acid sequence identical to the amino acid sequence shown in SEQ ID NO: 28 except that one or more amino acids are substituted, deleted, inserted, and/or added in any region outside the region of amino acid positions 370 to 377. Such a polypeptide can exhibit anti-HBV activity as long as the polypeptide contains the region of amino acid positions 370 to 377 (the amino acid sequence of SEQ ID NO: 25), even if a few mutations occur in amino acids located outside the region, and the polypeptide is therefore useful as an anti-HBV agent. The polypeptide (15) includes polypeptides comprising one or more amino acid substitutions, deletions, insertions, and/or additions in any region outside the region of amino acid positions 362 to 377 or outside the region of amino acid positions 369 to 384 in the amino acid sequence of SEQ ID NO: 28. The higher the sequence identity of the polypeptide (15) to the amino acid sequence of SEQ ID NO: 28, the more preferable the polypeptide (15). For example, it is more preferred that the sequence identity of the polypeptide (15) to the amino acid sequence of SEQ ID NO: 28 be 98% or more, or 99% or more.

As used herein, the "identity" of an amino acid sequence means the value calculated by aligning two amino acid sequences to be compared such that the number of matched amino acid residues becomes largest between the amino acid sequences, and dividing the number of matched amino acid residues by the total number of amino acid residues, which value is expressed as a percentage. In the alignment, a gap(s) is/are inserted, when necessary, into one or both of the two sequences to be compared. Such alignment of sequences may be carried out using a well-known program such as BLAST, FASTA, or CLUSTAL W. When a gap(s) is/are inserted, the above-described total number of amino acid residues is the number of residues calculated by counting one gap as one amino acid residue. When the thus counted total number of amino acid residues is different between the two sequences to be compared, the sequence identity (%) is calculated by dividing the number of matched amino acid residues by the total number of amino acid residues in the longer sequence.

Conservative substitutions, *i.e.* substitutions between amino acids with similar chemical properties, are very unlikely to affect the properties or activity of a protein. Amino acids with similar side chains have similar chemical properties. Based on the similarities among the side chains, amino acids can be grouped into, for example, a group of amino acids with an aliphatic side chain (glycine, alanine, valine, leucine, isoleucine), a group of amino acids with an aliphatic hydroxyl side chain (serine, threonine), a group of amino acids with an amide-containing side chain (asparagine, glutamine), a group of amino acids with an aromatic side chain (phenylalanine, tyrosine, tryptophan), a group of amino acids with a basic side chain (arginine, lysine, histidine), a group of amino acids with an acidic side chain (aspartic acid, glutamic acid), and a group of amino acids with a sulfur-containing side chain (cysteine, methionine). A conservative substitution refers to a substitution of an amino acid with another amino acid within the same amino acid group. Typical examples of the amino acid sequence of (15) include, but are not limited to, an amino acid sequence identical to SEQ ID NO: 28 except that a conservative substitution(s) is/are introduced into any region outside the region of amino acid positions 370 to 377 (NCRHNTAG).

The polypeptide (16) is a polypeptide composed of a fragment of the polypeptide (15) that is composed of a mutated sequence of Netriii- I (mutated Netrin-1 partial polypeptide), which fragment contains the above-described region of amino acid positions 370 to 377 (the amino acid sequence of SEQ ID NO: 25). A fragment of a polypeptide composed of a mutated sequence of Netrin-1 can also exhibit anti-HBV activity as long as the fragment contains the region of amino acid positions 370 to 377 (the amino acid sequence of SEQ ID NO: 25), and the fragment is therefore useful as an anti-HBV agent.

The polypeptide (26) is a polypeptide composed of the full-length sequence of N4BP1. The sequences set forth in SEQ ID NOs: 81 and 82 are the base sequence of the coding region in the sequence of the N4BP1 gene under NCBI Accession No. NM_153029.4 and the encoded amino acid sequence of N4BP1 (NP_694574.3), respectively. The binding of the full-length form of N4BP1 to LIPG is shown in Example 17 below, and the polypeptides (23) to (25), which are N4BP1 partial polypeptides containing a defined region, have an anti-HBV effect. Therefore, the full-length form of N4BP1 can also be used as an anti-HBV agent.

The polypeptide (27) is a polypeptide which is composed of a partial sequence of N4BP1 (N4BP1 partial polypeptide) containing a region of amino acid positions 456 to 464 in the amino acid sequence of N4BP1 shown in SEQ ID NO: 82. The region of amino acid positions 456 to 464 is a region common to the polypeptides (23) to (25) composed of a partial sequence of N4BP1, and also corresponds to the full length of the polypeptide (24), LIPH4-N4BP1b (SEQ ID NO: 79). The anti-HBV activity of the polypeptide (24) is shown in an example below (Fig. 17). Accordingly, a polypeptide composed of a partial sequence of N4BP1 can also exhibit anti-HBV activity as long as the polypeptide contains the region of amino acid positions 456 to 464, and the polypeptide is therefore useful as an anti-HBV agent. The polypeptide (27) includes a polypeptide composed of a partial sequence of N4BP1 containing a region of amino acid positions 455 to 464 (SEQ ID NO: 80) in the amino acid sequence of SEQ ID NO: 82, and a polypeptide composed of a partial sequence of N4BP containing a region of amino acid positions 456 to 471 (SEQ ID NO: 78) in the above-mentioned amino acid sequence. In addition, the polypeptides (23) to (25) are specific examples of the polypeptide (27).

The polypeptide (28) is a polypeptide composed of a mutated sequence of N4BP1, and is specifically a polypeptide having 95% or more identity to the amino acid sequence of N4BP1 shown in SEQ ID NO: 82, which is represented by an amino acid sequence identical to the amino acid sequence shown in SEQ ID NO: 82 except that one or more amino acids are substituted, deleted, inserted, and/or added in any region outside the region of amino acid positions 456 to 464. The polypeptide can exhibit anti-HBV activity as long as the polypeptide contains the region of amino acid positions 456 to 464 (the amino acid sequence of SEQ ID NO: 79), even if a few mutations occur in amino acids located outside the region, and the polypeptide is therefore useful as an anti-HBV agent. The polypeptide (28) includes polypeptides comprising one or more amino acid substitutions, deletions, insertions, and/or additions in any region outside the region of amino acid positions 455 to 464 or outside the region of amino acid positions 456 to 471 in the amino acid sequence shown in SEQ ID NO: 82. The higher the sequence identity of the polypeptide (28) to the amino acid sequence of SEQ ID NO: 82, the more preferable the polypeptide of (28). For example, it is more preferred that the sequence identity of the polypeptide (28) to the amino acid sequence of SEQ ID NO: 82 be 98% or more, or 99% or more.

The polypeptide (29) is a fragment of the polypeptide (28) that is composed of a mutated sequence of N4BP1, and is a polypeptide composed of a partial sequence of a mutated N4BP1 sequence, the partial sequence containing the above-described region of amino acid positions 456 to 464 (the amino acid sequence of SEQ ID NO: 79) (mutated N4BP1 partial polypeptide). A fragment of a polypeptide composed of a mutated N4BP1 sequence can also exhibit anti-HBV activity as long as the fragment contains the region of amino acid positions 456 to 464 (the amino acid sequence of SEQ ID NO: 79), and the fragment is therefore useful as an anti-HBV agent.

The polypeptide (30) is a polypeptide which is identical in sequence to any of the polypeptides (1) to (14) and (17) to (27) except that some residues are changed and has 80% or more identity to the original polypeptide. It is well known in the art that the activity of the polypeptide is maintained at a level equal to or higher than that of the original polypeptide even if very few residues are changed from those in the original polypeptide. Therefore, a polypeptide which is identical in sequence to any one of the polypeptides (1) to (14), (16) to (27), and (29) except for changes in a few residues (for example, 1 to 4 residues, 1 to 3 residues. 1 or 2 residues, or 1 residue, in the case of short polypeptides having a length of less than 20 residues, such as the polypeptides (1) to (12) and (17) to (25), or in the case of short polypeptides having a length of less than approximately 20 residues among the polypeptides (14) and (27); and for example. 1 to 20 residues, 1 to 15 residues, 1 to 10 residues, or 1 to several residues in the case of long-chain polypeptides among the polypeptides (14) and (27)) and has an identity of 80% or more, e.g. 85% or more, 90% or more, 95% or more, or 98% or more to the original sequence, can exhibit anti-HBV activity similarly to the original polypeptide, and the polypeptide is therefore useful as an anti-HBV agent. As used herein, changes in residues are substitutions, deletions, insertions, and/or additions, and are typically substitutions, especially conservative substitutions.

The Netrin-1 partial polypeptide (14), the mutated Netrin-1 partial polypeptide (16), the N4BP1 partial polypeptide (27), and the mutated N4BP1 partial polypeptide (29) may have a chain length of, for example, but not specifically limited to, 100 residues or less, 80 residues or less, 70 residues or less, 60 residues or less, 50 residues or less, 40 residues or less, 30 residues or less, 25 residues or less, or 20 residues or less, in consideration of the convenience in synthesis or delivery into liver cells.

The polypeptides (1) to (12) and (17) to (25) have a short chain length and therefore can be easily prepared by chemical synthesis. Specific examples of the method for chemical synthesis include the Fmoc method (the fluorenylmethyloxycarbonyl method) and the tBoc method (the *t*-butoxycarbonyl method). In addition, these polypeptides can be synthesized using a variety of commercially available peptide synthesizers by conventional methods.

The polypeptides (13) (Netrin-1) and (26) (N4BP1) can be prepared by a well-known genetic engineering procedure. Specifically, the polypeptide (13) or (26) can be prepared by extracting RNA from cells expressing Netrin-1 or N4BP1 (for example, a human-derived cell line or the like), using the RNA as a template for RT-PCR to prepare a cDNA fragment encoding Netrin-1 or N4BP1, inserting the resulting cDNA fragment into a suitable expression vector, introducing the resulting vector into a suitable type of host cells, and extracting and purifying the polypeptide of interest produced in the host cells. For primers used for the RT-PCR, the primers for Netrin-1 can be designed based on the base sequence shown in SEQ ID NO: 27 (a coding region sequence under NCBI Accession No. NM_004822.3, encoding an amino acid sequence shown in SEQ ID NO: 28), and the primers for N4BP1 can be designed based on the base sequence shown in SEQ ID NO: 81 (a coding region sequence under NCBI Accession No. NM_153029.4, encoding an amino acid sequence shown in SEQ ID NO: 82). Various expression vectors and host cell lines are known and commercially available, and are therefore easy to obtain. The polypeptides (15) and (27) can be prepared by introducing mutations into the Netrin-1-encoding cDNA and N4BP1-encoding cDNA fragments prepared as described above. Various kits for introducing mutations are also commercially available.

Among the polypeptides (14). (16), (27), (29), and (30), polypeptides having a short chain length can be easily prepared by chemical synthesis. Among the polypeptides (14), (16), (27), (29), and (30), polypeptides that are difficult to be prepared by chemical synthesis can be prepared as follows: a DNA fragment encoding a polypeptide of interest is synthesized by, for example, using primers (optionally mutagenized) that are set within the coding region to carry out RT-PCR using RNA extracted from cells expressing Netrin-1 or N4BP1 as a template, or in cases where Netrin-1 cDNA or N4BP1 cDNA has been synthesized or cloned, by using Netrin-1 cDNA or N4BP1 cDNA as a template in a PCR reaction to amplify a DNA fragment (optionally mutagenized); the resulting DNA fragment is inserted into a suitable expression vector to express the polypeptide in host cells; and the expressed polypeptide is extracted and purified.

Polypeptides prepared by chemical synthesis have an advantage as pharmaceutical products because those prepared by chemical synthesis are free of host cell-derived materials, unlike those prepared by genetic recombination. Therefore, among the polypeptides (1) to (30), the polypeptides (1) to (12) and (17) to (25) and short polypeptides (having a length of several tens of residues or less) among the polypeptides (14), (16), (27), (29), and (30) can be preferably used as an active ingredient in an anti-HBV agent. The polypeptides (14) and (16) contain the region of amino acid positions 370 to 377 (the amino acid sequence of SEQ ID NO: 25) and are therefore 9 residues or more in length, while the polypeptides (27) and (29) contain the region of amino acid positions 456 to 464 (the amino acid sequence of SEQ ID NO: 79 ) and are therefore 9 residues or more in length. Polypeptides which are included in the polypeptides (14) and (16) and have a length of 8 residues or more are identical to the polypeptide (1). The polypeptides (11), (12), and (22) are partial polypeptides of Netrin-1 containing the defined region and are therefore specific examples of the polypeptide (14). The polypeptides (23) to (25) are partial polypeptides of N4BP1 containing the defined region and are therefore specific examples of the polypeptide (27).

In the field of peptide preparations, the following techniques are used for the purpose of improving the *in vivo* stability of peptides and prolonging the half-life in blood of peptides: addition of polyethylene glycol (PEG) chains (Clin Nephrol. 2006 Mar; 65 (3): 180-90., Proc Natl Acad Sci USA. 2005 Sep 6; 102 (36): 12962-7., and the like), addition of sugar chains primarily at the N- or C-terminus (J Am Chem Soc. 2004 Nov 3; 126 (43): 14013-22, Angew Chem Int Ed Engl. 2004 Mar 12; 43 (12): 1516-20, and the like), replacing at least some of the amino acid residues with the D-form of the amino acids (J Pharmacol Exp Ther. 2004 Jun; 309 (3): 1190-7.J Pharmacol Exp Ther. 2004 Jun; 309 (3): 1183-9., and the like), addition of an appropriately modified Fc region of an antibody (for example, J. Immunol.. 154 (10), 5590-5600 (1995), Nature, 332, 563-564 (1998), Nature, 332, 738-740 (1998), BioDrugs. 2008;22: 11-26, and the like), amidation of the C-terminus, acetylation of the N-terminus, and the like. Such a technique can be applied to a polypeptide used as an active ingredient in an anti-HBV agent of the present invention. Furthermore, a polypeptide modified with other molecules, such as polypeptides that have the effect of enhancing hepatic delivery, can also be used as an active ingredient.

In the present invention, the phrases "a polypeptide of a sequence shown in SEQ ID NO: X", "a polypeptide represented by SEQ ID NO: X", and "a polypeptide composed of a sequence shown in SEQ ID NO: X" (SEQ ID NO: X shows an amino acid sequence having a total length of N amino acid residues) include polypeptides having a structure where a polypeptide having the amino acid sequence shown in SEQ ID NO: X and a total length of N residues (such a polypeptide is referred to as polypeptide X for convenience) is linked to (an)other functional polypeptide(s) such as the Fc region or a polypeptide having the effect of enhancing hepatic delivery. The phrase 'containing "a polypeptide of a sequence shown in SEQ ID NO: X", "a polypeptide represented by SEQ ID NO: X", or "a polypeptide composed of a sequence shown in SEQ ID NO: X" as an active ingredient' includes not only a mode of containing the polypeptide X having a total length of N residues as an active ingredient but also a mode of containing as an active ingredient a polypeptide having a structure where (an)other functional polypeptide(s) is/are linked to the polypeptide X. Any functional polypeptide may be attached to the polypeptide, provided that the anti-HBV activity of the polypeptide is not impaired by the functional polypeptide. Polypeptides having a structure where (an)other functional polypeptide(s) is/are linked thereto can be produced by chemical synthesis in cases where the functional polypeptide(s) has/have a short length, or by genetic engineering procedures in cases where the functional polypeptide(s) has/have a long length.

Furthermore, the LIPG-binding peptide may comprise a tag sequence(s) such as Flag tag or His tag attached thereto for convenience of purification and/or detection. Such a tag sequence can also be considered as an example of a functional molecule. For example, in the single-chain antibody fusions prepared in the examples below, Flag and His tags are attached to the C-terminus of each of the LIPG-binding peptides. However, the addition of such a tag sequence is optional, and LIPG-binding peptides without a tag sequence can be used to prepare anti-HBV agents of the present invention.

An example of a functional molecule may be a carrier molecule for delivery into hepatocytes. That is, a LIPG-binding peptide can be in a form where the LIPG-binding peptide is linked to a carrier molecule(s) for delivery into hepatocytes. As described in the examples below, the anti-HBV effect of the LIPG-binding peptides can be greatly enhanced when the LIPG-binding peptides are delivered into hepatocytes. DDSs that utilize the asialoglycoprotein receptor (ASGR), which is abundantly present in hepatic parenchymal cells, or the ApoE receptor are known as carrier molecules for drugs to be delivered into hepatocytes (Yuko Ito, STI Horizon, 2019, Vol. 5, No. 4, pp. 21-25). Carrier molecules that utilize the ASGR or the ApoE receptor to deliver drugs into liver cells can also be preferably used in the present invention.

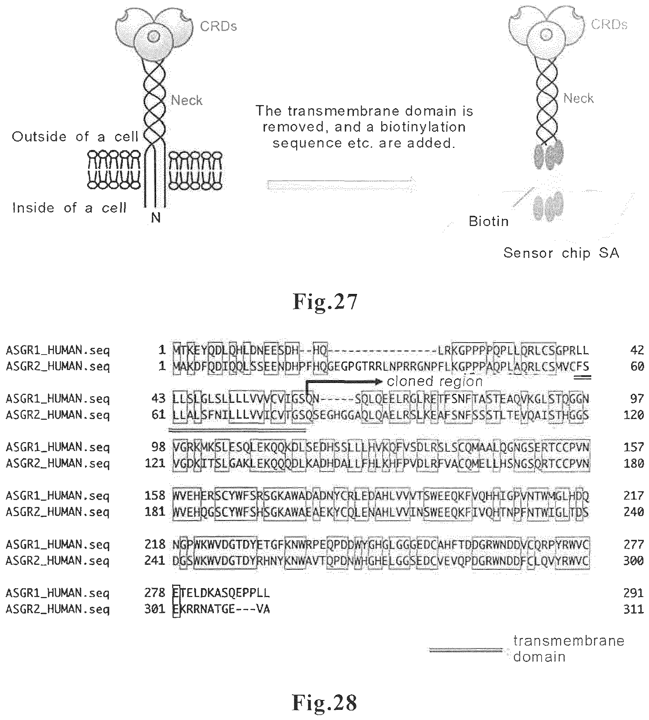

Examples of the drug delivery carrier utilizing the ASGR include ASGR-specific binding molecules (for example, antibodies, antibody fragments or single-chain antibodies, or aptamers), particularly antibodies, antibody fragments, or single-chain antibodies (scFvs) that bind to the ASGR. Asialoglycoprotein receptor (ASGR) are type II, single-pass transmembrane proteins with the N-terminus inside and the C-terminal carbohydrate recognition domain (CRD) outside the cell membrane, as shown in Fig. 27. In humanhepatocytes, the receptor is formed of a heterooligomer of ASGR1 and ASGR2. An ASGR-specific binding molecule used as a drug delivery carrier may be any molecule as long as it binds to the ASGR extracellular domain. Although a specific binding molecule that can bind to both ASGR1 and ASGR2 can be particularly preferably used, a specific binding molecule that binds to only one of them can also be used as a drug delivery carrier. The specific binding molecule that binds to only one of them may be used alone as a drug delivery carrier, or may be used in combination with a specific binding molecule that binds to the other. In the case of combination use, a LIPG-binding peptide linked to an ASGR1-specific binding molecule and a LIPG-binding peptide linked to an ASGR2-specific binding molecule may be prepared and later mixed to use as an anti-HBV agent.

The base sequence of the coding region in the sequence of human ASGR1 gene registered in GenBank under NM_001671.5 and the amino acid sequence of ASGR1 encoded thereby are shown in SEQ ID NOs: 113 and 114, respectively, and the base sequence of the coding region in the sequence of human ASGR2 gene registered under NM_001181.4 and the amino acid sequence of ASGR2 encoded thereby are shown in SEQ ID NOs: 115 and 1 16, respectively. The amino acids of positions 41 to 61 in SEQ ID NO: 114 (ASGR1) and the amino acids of positions 59 to 79 in SEQ ID NO: 116 (ASGR2) are transmembrane domains. The N-terminal sides of the transmembrane domains are intracellular domains, and the C-terminal sides of the transmembrane domains are extracellular domains. In the preparation of an anti-ASGR antibody, antibody fragment, or single-chain antibody, a region of amino acid positions 62 to 291 of ASGR 1 and a region of amino acid positions 80 to 331 of ASGR2 may be used as antigens or immunogens, and a heterooligomer composed of these extracellular domains is more preferably used. Hereinafter, the term "ASGR extracellular domain" includes hetero-oligomerized extracellular domains. The term "heterooligomer" refers to the hetero-oligomerized ASGR extracellular domains.

An anti-ASGR polyclonal antibody can be obtained by using the ASGR extracellular domain as an immunogen to immunize a non-human animal, collecting the blood from the animal, separating the serum from the blood, and recovering and purifying an antibody that binds to the ASGR extracellular domain from the serum. An anti-ASGR monoclonal antibody can be obtained by collecting antibody-producing cells such as spleen cells or lymphocytes from a non-human animal immunized with the ASGR extracellular domain, fusing the collected cells with myeloma cells to prepare hybridoma cells, selecting a hybridoma that produces an antibody that binds to the ASGR extracellular domain, and culturing the selected hybridoma to recover the anti-ASGR monoclonal antibody from the culture supernatant.

An anti-ASGR antibody fragment can be obtained by treating an anti-ASGR antibody with a proteinase such as papain or pepsin. Antibody fragment is defined as above and includes antibody fragments that retain the binding property to the antigen (the ASGR extracellular domain), such as Fab, Fab', and F(ab')₂.

An anti-ASGR scFv can be obtained by, for example, extracting mRNA from the hybridoma prepared as described above to prepare cDNA, carrying out PCR using primers specific for immunoglobulin H and L chains to amplify fragments of the immunoglobulin H and L chain genes, joining the fragments via a linker and adding appropriate restriction enzyme recognition sites allowing insertion into a plasmid vector, transforming *E. coli* with the resulting vector to express a scFv, and recovering the scFv from the *E. coli.*

In addition, an anti-ASGR scFv can be obtained from scFv libraries by techniques such as the phage display technique or the IVV method.

In the phage display technique, a library of phages that display scFVs on the phage surface is prepared as a scFv library. For example, a naive scFv phage library can be prepared according to the following procedure. Antibody-producing cells such as spleen cells or lymphocytes are harvested from healthy human or non-human animals, and mRNA is extracted from the cells to synthesize cDNA by a reverse transcription reaction, and cDNA fragments each encoding a region containing the heavy chain variable region (VH) (VH cDNA) and cDNA fragments each encoding a region containing the light chain variable region (VL) (VL cDNA) are comprehensively amplified by PCR. Next, the amplified VH cDNA and VL cDNA fragments are randomly joined via a suitable linker (for example, a linker composed of a triple repeat of the GGGGS unit, or the like) by a conventional method such as assembly PCR or fusion PCR to prepare scFv-encoding cDNA (scFv cDNA) fragments. The scFv cDNA fragments are inserted into a plasmid vector for phages to prepare a vector library, and a library of phage displaying scFvs is then produced by the phage display technique. There are two types of plasmid vectors for phages: phage vectors, which contain all phage genes necessary for phage particle production and can produce phage particles by themselves; and phagemid vectors, which contain the g3p gene but no other phage protein genes and require a helper phage to produce phage particles. In the present invention, a phagemid vector can be preferably used. A scFv cDNA library produced using a phagemid vector is introduced into *E. coli*, and a helper phage is introduced into the *E. coli* for double infection, so that a library of phases which contains individual vectors in the scFv cDNA library packaged thereinto and display scFvs expressed from the vectors on their surface can be produced. A phage library of scFv mutants can also be produced by randomly introducing mutations into the prepared VH cDNA and VL cDNA fragments or scFv cDNA fragments.

A phage displaying a scFv that binds to the ASGR extracellular domain is selected from the produced library (panning). The panning process can be performed using a solid-phase support (chip, plate, magnetic bead, or the like) on which the ASGR extracellular domain is immobilized, particularly preferably using a support on which the heterooligomer is immobilized. The extracellular domain of ASGR1 (ASGR1ex) and the extracellular domain of ASGR2 (ASGR2ex) are biotinylated, and the biotinylated ASGR1ex and ASGR2ex are brought into contact with a support coated with an avidin-type molecule so that the ASGR1ex and ASGR2ex can be combined into a heterooligomer on the support surface. The ASGR extracellular domain-immobilized support is contacted with the phage library, and after washing the support, phages bound to the support are harvested. The harvested phages are lysed, and the packaged vectors are recovered from the phages and then reintroduced into *E. coli.* A helper phage is introduced into the *E. coli* for double infection to produce phage particles again. These phage particles are again contacted with the ASGR extracellular domain-immobilized support. This process can be repeated several times to enrich for phage clones with a displayed scFv specific for the ASGR extracellular domain.

Although the phages enriched by multiple rounds of panning can be obtained as candidate anti-ASGR scFv clones, the clones can be screened for further selection of candidates. The scFv expression vectors are recovered from the enriched phages and introduced into a suitable host cell, such as *E. coli,* to prepare scFv-expressing host cell clones. These clones are tested for reactivity with the ASGR extracellular domain and the variable regions in the clones are sequenced to select clones with high specificity for binding to the ASGR extracellular domain. The reactivities of the clones can be determined by immunoassay, such as ELISA, using the ASGR extracellular domain, preferably the heterooligomer, as an antigen. Because multiple clones that contain variable regions with identical or highly similar sequences may be present, grouping of the clones may be carried out by checking for clone redundancy based on the nucleotide sequences of VH and VL. The candidate clones can be further screened by selecting clones with high specificity for binding to the ASGR extracellular domain through these clone selection steps.

From the candidate clones, the scFv expression vectors are recovered, and scFv cDNA fragments are amplified from the scFv expression vectors. The amplified scFv cDNA fragments are inserted into a suitable plasmid expression vector to express scFvs in suitable host cells, and the scFvs are collected and purified. Finally, the purified scFvs can be tested for reactivity with ASGR1ex and ASGR2ex or reactivity with the heterooligomer to obtain a scFv that specifically binds to the ASGR extracellular domain.

The IVV method (Nemoto, N. et al., FEBS Lett.. 414: 405-408, 1997; Miyamoto-Sato, E. et al., Nucleic Acids Res., 28: 1176-1182, 2000: WO 03/106675 A1) is a technique developed by one of the inventors of the present application, Yanagawa, and his colleagues. In the IVV method, an antibiotic, puromycin, is attached to the 3' end of mRNAs via a PEG (polyethylene glycol) spacer, and a cell-free translation reaction is performed using the resulting mRNAs as templates to generate mRNA-protein fusion molecules (*in vitro* viruses; IVVs) in which the resulting protein molecules and the mRNA molecules encoding them are covalently linked via puromycin. From the IVV library constructed as described above, an IVV comprising a protein that binds to a bait (a protein, peptide, antigen, or the like) is pulled up *in vitro,* and the gene (mRNA) linked to the protein is amplified by reverse-transcription PCR, followed by analyzing the base sequence of the gene with a DNA sequencer, so that a protein, peptide, or antibody that binds to the bait can be readily identified. A method based on competitive elution with a free form of the bait is commonly used to elute and recover the bait-bound IVV. In cases where competitive elution is not feasible, a PEG spacer containing a photocleavable 2-nitrobenzyl linker can be used, and the space can be cleaved by UV irradiation at 365nm to allow elution and recovery of the mRNA moieties (Doi. N. et al., J. Biotechnol., 131: 231-239, 2007).

The details of the method for anti-ASGR scFv production by the IVV method are as described in the examples below. The method is briefly described below.

A scFv cDNA library is prepared as described above, and then mRNAs are synthesized from the cDNA library by a reverse transcription reaction, and puromycin is attached to the 3' end of the mRNAs via a PEG spacer to prepare a scFv mRNA-puromycin library. A cell-free translation reaction is performed using the library as a template to construct a library of mRNA-scFv fusion molecules (IVVs) in which the scFv molecules and the mRNA molecules encoding the scFv molecules are covalently linked via puromycin.

From the scFv IVV library, scFvs that bind to the ASGR extracellular domain are screened. This screening process can also be performed, similarly to the phage display technique, by using a solid-phase support (chip, plate, magnetic bead, or the like) on which the ASGR extracellular domain, preferably the heterooligomer, is immobilized as a bait. The ASGR extracellular domain-immobilized support is contacted with the scFv IVV library, and after washing the support, IVVs bound to the support are eluted and recovered. The IVVs can be eluted and recovered by competitive elution using ASGR1ex or ASGR2ex. In cases where the PEG spacer used is a PEG spacer containing a 2-nitrobenzyl linker, the spacer can be cleaved by UV irradiation to allow elution and recovery of the mRNA moieties of the IVVs.

After the first round of selection, a reverse-transcription PCR reaction is performed using the recovered IVVs or mRNA parts as a template to prepare a scFv cDNA library. An IVV library can be prepared from the cDNA library to perform the second round of selection. Preferably, several rounds of selection are performed with increasing selection pressure (time of contact between an IVV library and a bait, the amount of immobilized bait on the support, or the like) in a sequential manner.

After several rounds of selection, scFv coding regions from each of the prepared scFv cDNA libraries are amplified and cloned into a suitable plasmid vector, and libraries of vectors with insertion of the selected scFv cDNAs are prepared. The vectors are introduced into a suitable host cell, such as *E. coli,* to obtain a library of the selected scFv clones. In a similar manner to clone selection in phage display technique, evaluation of reactivity with the ASGR extracellular domain, analysis of variable region sequences, and grouping are performed on the resulting clone library to screen clones, and vectors are recovered from the selected clones to prepare scFvs. Finally, the scFvs are tested for reactivity with ASGR1ex and ASGR2ex or reactivity with heterooligomer, so that a scFv with high specificity for binding to the ASGR extracellular domain can be obtained.

Particularly preferred examples of an anti-ASGR antibody, antibody fragment, or scFv include an antibody, antibody fragment, or scFv comprising the following regions:
a heavy chain CDR1 comprising an amino acid sequence shown in SEQ ID NO: 83, 89, 95, or 101;
a heavy chain CDR2 comprising an amino acid sequence shown in SEQ ID NO: 84, 90, 96, or 102;
a heavy chain CDR3 comprising an amino acid sequence shown in SEQ ID NO: 85, 91, 97, or 103;
a light chain CDR1 comprising an amino acid sequence shown in SEQ ID NO: 86, 92, 98, or 104;
a light chain CDR2 comprising an amino acid sequence shown in SEQ ID NO: 87, 93, 99, or 105; and
a light chain CDR3 comprising an amino acid sequence shown in SEQ ID NO: 88, 94, 100, or 106.
These CDR sequences are based on the CDR sequences of anti-ASGR scFvs obtained in the examples below. In the examples below, the following anti-ASGR scFvs have been obtained: an anti-ASGR scFv having heavy chain CDRs 1 to 3 of amino acid sequences shown in SEQ ID NOs: 83 to 85 and light chain CDRs 1 to 3 of amino acid sequences shown in SEQ ID NOs: 86 to 88; an anti-ASGR scFv having heavy chain CDRs 1 to 3 of amino acid sequences shown in SEQ ID NOs: 89 to 91 and light chain CDRs 1 to 3 of amino acid sequences shown in SEQ ID NOs: 92 to 94: an anti-ASGR scFv having heavy chain CDRs 1 to 3 of amino acid sequences shown in SEQ ID NOs: 95 to 97 and light chain CDRs 1 to 3 shown in SEQ ID NOs: 98 to 100; and an anti-ASGR scFv having heavy chain CDRs 1 to 3 shown in SEQ ID NO: 101 to 103 and light chain CDRs 1 to 3 shown in SEQ ID NOs: 104 to 106.

In addition, the CDR sequences are not limited to the specific amino acid sequences described above, and the CDRs of the antibody or the like may comprise amino acid sequences which are the same as described above except that some of the bases are substituted and have 80% or more identity to the original amino acid sequences. For example, substitution of 1 residue is acceptable in the heavy and light chain CDR1s; substitution of 1 to 3 residues, 1 to 2 residues, or 1 residue is acceptable in the heavy and light chain CDR2s; substitution of 1 to 2 residues or 1 residue is acceptable in the heavy and light chain CDR3s. An antibody, antibody fragment, or scFv having such modified CDRs may also be used as an antibody, antibody fragment, or scFv specifically binding to the ASGR.

For example, an antibody or the like comprising CDRs of the determined sequences can be readily prepared by introducing a mutation(s) into the CDR regions in cloned VH and VL genes of any antibody or in a scFv gene encoding any scFv so that the gene(s) is/are modified to encode the amino acid sequences described above. The given antibody or scFv used as a base into which the CDR sequences described above are introduced may be an anti-ASGR antibody or an anti-ASGR scFv having CDRs with sequences different from those described above, or an antibody or scFv against another antigen.

An anti-ASGR antibody, antibody fragment, or scFv having the CDRs comprising the specific amino acid sequences described above or the modified CDRs described above is very advantageous not only as a delivery carrier for an anti-HVB agent of the present invention, but also as a delivery carrier for various drugs to be delivered into hepatocytes. The anti-ASGR antibody, antibody fragment, or scFv can be used as a drug carrier for delivery into hepatocytes, and a complex formed between a drug to be delivered into hepatocytes and the carrier by a technique known in the field of pharmaceutical formulation can be used as a pharmaceutical composition. The anti-ASGR antibody may be a human antibody, a humanized antibody, a human-non-human chimeric antibody, or a non-human antibody. When used as a delivery carrier for human drugs, the anti-ASGR antibody is preferably a human antibody, a humanized antibody, or a chimeric antibody, more preferably a human antibody or a humanized antibody, and especially preferably a human antibody. Similarly, the anti-ASGR scFv antibody may also be derived from a human antibody, a humanized antibody, a human-non-human chimeric antibody, or a non-human antibody. When used as a delivery carrier for human drugs, the anti-ASGR scFv antibody is preferably derived from a human antibody, a humanized antibody, or a chimeric antibody, more preferably from a human antibody or a humanized antibody, and most preferably from a human antibody.

When a carrier molecule for delivery into hepatocytes, such as an anti-ASGR antibody, is attached to a LIPG-binding peptide, the carrier molecule can be attached to either terminus of the LIPG-binding peptide. In cases where one or more other functional molecules are also used, the carrier molecule can be attached to the LIPG-binding peptide so that the other functional molecules can function properly. Preferably, the carrier molecule for delivery into hepatocytes is attached to the LIPG-binding peptide via a cleavage sequence which is cleaved by an endogenous enzyme present in the cells of a patient receiving administration of an anti-HBV agent of the present invention. An example of the cleavage sequence can be, but is not limited to, a sequence recognized and cleaved by Furin, RVRR (SEQ ID NO: 111).

Another example of a functional molecule is a cell membrane penetration-enhancing molecule. That is, a LIPG-binding peptide can be in a form in which the LIPG-binding peptide is linked to a cell membrane penetration-enhancing molecule. The term "cell membrane penetration-enhancing molecule" refers to a molecule that has the activity to promote release of the LIPG-binding peptide which has been taken up into cells by endocytosis from endosomes across the endosomal membrane into cytoplasm. Specific examples of the cell membrane penetration-enhancing molecule include the S19 peptide developed by Doi and his colleagues, which is a partial peptide of the Syncytin-1 protein involved in cell fusion during the human placentation (Sudo, K. et al., J. Control. Release, 255: 1-11, 2017, Sudo, K. el al., J. Control. Release. 255: 1-11, 2017, WO 2016/199674 A1), the S28 peptide (PFVIGAGVLGALGTGIGGITTSTQFYYK, SEQ ID NO: 107), a 28-residue partial peptide of Syncytin-1 and the S39 peptide (PFVIGAGVLGALGTGIGGITTSTQFYYKLSQELNGDMER, SEQ ID NO: 108), a 39-residue partial peptide of Syncytin-1, which were developed by a more recent study as membrane penetration-enhancing peptides that can function more efficiently than S19. These peptides, particularly the S28 and S39 peptides, are cell membrane penetration-enhancing molecules which can be preferably used in the present invention, but a cell membrane penetration-enhancing molecule that can be used is not limited to these peptides.

A cell membrane penetration-enhancing molecule can be attached to either terminus of a LIPG-binding peptide. However, in cases where a carrier molecule for delivery into hepatocytes and a cleavage sequence are attached to a LIPG-binding peptide, a cell membrane penetration-enhancing molecule is located closer to the LIPG-binding peptide than the cleavage sequence or is attached to a terminus opposite to the other terminus to which the carrier molecule is attached.

A further example of a functional molecule is a nuclear localization signal. That is, a LIPG-binding peptide can be in a form in which the LIPG-binding peptide is linked to a nuclear localization signal. Various nuclear localization signals are known and any of the known nuclear localization signals can be used. Although PAAKRVKLD (SEQ ID NO: 110) is used as a nuclear localization signal in the examples below, a nuclear localization signal that may be used is not limited to this particular example. A nuclear localization signal can be attached to either terminus of a LIPG-binding peptide. However, in cases where a carrier molecule for delivery into hepatocytes and a cleavage sequence are attached to a LIPG-binding peptide, a nuclear localization signal is located closer to the LIPG-binding peptide than the cleavage sequence or is attached to a terminus opposite to the other terminus to which the carrier molecule is attached. In cases where a cell membrane penetration-enhancing molecule and a nuclear localization signal are attached to a LIPG-binding peptide, the cell membrane penetration-enhancing molecule and the nuclear localization signal may be attached separately to both termini or together to one of the termini. In the latter case, either of the cell membrane penetration-enhancing molecule and the nuclear localization signal can be located at the tail end. In cases where a delivery carrier + a cleavage sequence, a cell membrane penetration-enhancing molecule, and a nuclear localization signal are attached to a LIPG-binding peptide, the delivery carrier + a cleavage sequence is attached to one terminus, and the cell membrane penetration-enhancing molecule and the nuclear localization signal are attached to the other terminus.

A subject administered with an anti-HBV agent of the present invention is mainly an HBV-infected patient, including a hepatitis B patient and an HBV-infected patient who has not developed hepatitis B (IIBV carriers). The patient is typically, but not limited to, a mammal, particularly human.

The dose of the anti-HBV agent of the present invention administered to a patient may be any dose as long as an anti-HBV effect is obtained. The effective dose can be appropriately selected based on, for example, the patient's condition, viral load, age, and body weight. The dose of the anti-HBV agent of the present invention may be, but is not specifically limited to, approximately 1 µg to 10,000 mg per kg body weight per day, for example approximately 100 µg to 1,000 mg per kg body weight per day, in terms of an active ingredient. The dose of an active ingredient as used herein means, when one of the polypeptides of (1) to (30) is used as an active ingredient, the amount of the polypeptide portion consisting of one of the amino acid sequences of (1) to (30), and does not include the amount of (an)other functional polypeptide(s) in cases where the polypeptide to which the (an)other functional polypeptide(s) is/are attached is used. In the case of administering a plurality of the polypeptides, the amount of an active ingredient is equal to the total amount of these polypeptides. The daily dose may be given all at once or in several divided doses. The dose may be given daily or every several days.

The route of administration of the anti-HBV agent of the present invention may be oral administration or parenteral administration. In general, parenteral administration, such as intramuscular administration, subcutaneous administration, intravenous administration, or intraarterial administration, is preferred as the route of administration.

An active ingredient in an anti-HBV agent of the present invention may be appropriately combined with pharmaceutically acceptable additives suitable for each route of administration, such as carriers, diluents, excipients, binders, lubricants, disintegrants, sweeteners, suspending agents, emulsifying agents, coloring agents, flavoring agents, and stabilizing agents, for drug formulation. Examples of the dosage form include oral agents such as tablets, capsules, granules, powders, and syrups; and parenteral agents such as inhalants, injections, suppositories, and liquids. Formulation methods and usable additives are well known in the field of pharmaceutical formulation, and any method and any additive can be used.

An anti-HBV agent comprising two or more of the polypeptides of (1) to (30) as an active ingredient may be a combination agent containing the two or more polypeptides in a single formulation or an agent comprising a combination of single agents each containing a single polypeptide. In the mode of the agent comprising a combination of single agents, the single agents are usually administered simultaneously or sequentially, and may be administered in series with appropriate time intervals.

### EXAMPLES

The present invention will be described below by way of examples. However, the present invention is not limited to the following examples.

### [Example 1] : Knockdown ofLIPG expression negatively regulates HBV infection

The effect of knocking down LIPG expression on HBV infection was investigated. Cells, viruses, reagents, and the like used for the experiments in Examples 1 to 4 are shown below. HCl cells (derived from human hepatocellular carcinoma), HepG2 cells (derived from human hepatocellular carcinoma), HepG2-NTCP-C4 cells (HepG2 cells overexpressing the HBV receptor NTCP; Iwamoto, M. et al., Biochem. Biophys. Res. Commun. 443: 808-813, 2014), HepG2.2.15 cells (a HepG cell line with integration of the HBV genome). Huh1 cells (derived from human hepatocellular carcinoma). Huh6 cells (derived from human hepatoblastoma), Huh7 cells (derived from human hepatocellular carcinoma), Huh7.5 cells (derived from Huh7), T5B cells (derived from normal humanhepatocytes), and RD cells (derived from human rhabdomyosarcoma) were maintained in DMEM (Thermo Fisher Scientific) supplemented with 10% fetal bovine serum (Thermo Fisher Scientific, Waltham, MA), 1% L-glutamine (Thermo Fisher Scientific), and 1% penicillin/streptomycin (Thermo Fisher Scientific) at 5% CO₂ and 37°C. PHH cells (primary human hepatocytes) were purchased from PhoenixBio (Hiroshima Prefecture). Heparin and heparanase were purchased from R&D systems (Minneapolis, MN). A LIPG inhibitor (GSK-264220A) was purchased from Tocris Bioscience (Minneapolis). Chlorpromazine (CPZ) and 5-(N-ethyl-N-isopropyl)amiloride (EIPA) were purchased from Sigma-Aldrich (St. Louis, MO). Primary antibodies against DYKDDDDK (FLAG) (#14793) (SEQ ID NO: 74), Caveolin-1 (#3238), vimentin (#5741), Pan-cadherin (#4068), E-cadherin (#3195) and β-actin (#4970) were obtained from Cell Signaling Technology (Beverly, MA), and NTCP (ab131084) and LIPG (ab24447) were obtained from abeam (Cambridge, UK). HBV used for experimental infection of PHH cells (primary human hepatocytes) was purchased from PhoenixBio. HBV derived from HepG2.2.15 cells was used for experimental infection of cultured cells other than PHH cells. A culture of HepG2.2.15 cells was filtered through a 0.45-µm filter for removal, and precipitation was carried out using 10% PEG 8000. HCV was propagated from a culture of Huh7.5 cells transfected with pHJ3-5/GLuc2A21 (Shimakami, T. et al., Sci. Rep. 4. 4688, 2014). Myocarditis virus (EMCV) was a gift from Dr. Mitstitoshi Yoneyama (Medical Mycology Research Center, Chiba University, Japan). Influenza virus strain A/WSN/33 was a gift from Dr. Masaki Enami (Laboratory of Molecular Genetics, Kanazawa University). A LIPG-targeting shRNA (target sequence: 5'-TTACACGGATGCG-GTCAATAA-3', SEQ ID NO: 73) cloned into the lentivirus-based pLKO.1-puro expression vector used for knocking down the LIPG gene was purchased from Sigma. The empty vector was used as a control shRNA (Cont shRNA). The vectors were co-transfected with a packaging plasmid into packaging cells (293FT cells) to generate lentivirus particles. Constitutive LIPG-knockdown cells were selected using a puromycin selectable marker.

HBV-DNA and cccDNA were quantified by qPCR as described below. Total DNA was extracted from cells using DNeasy Blood & Tissue Kit (Qiagen, Hilden, Germany). HBV-DNA was quantified by qPCR analysis as previously reported (Honda, M. et al.. J. Infect. Dis., 213, 1096-1106, 2016). The extracted DNA (50 ng) was treated with 10 U of Plasmid safe DNase I (Epicentre, Madison, WI) at 37°C for 60 minutes and then treated at 70°C for 30 minutes for DNase inactivation. cccDNA was quantified by qPCR analysis as previously reported (Honda, M. et al.. J. Infect. Dis., 213, 1096-1106, 2016). Quantitative RT-PCR was performed as described below. The GenEluteTM Mammalian Total RNA Miniprep Kit (Sigma-Aldrich) was used for extraction of total RNA, and the High Capacity cDNA Reverse Transcription Kit (Applied Biosystems, Carlsbad, CA) was used for cDNA synthesis. The 7500 Real Time PCR System (Applied Biosystems) was used for RT-PCR. Primers 5'-GCTCTGTATCGGGAGGCCTTA-3' (SEQ ID NO: 31) and 5'-TGAGTGCTGTATGGTGAGGAGAA-3' (SEQ ID NO: 32) and a probe 5'-FAM-AGTCTCCGGAACATT-MGB-3' (SEQ ID NO: 33) were used for pregenomic RNA. Primer pairs and probes for LIPG, NTCP, 18SrRNA, and ACTB were obtained from the TaqMan assay reagents library.

SDS-PAGE and immunoblotting were performed as described below. Cells were washed with phosphate-buffered saline (PBS) and dissolved in RIPA Lysis Buffer (EMD Millipore, Burlington. MA) containing complete Protease Inhibitor Cocktail (Roche Applied Science). Membranes were blocked with Blocking One solution (Nacalai Tesque, Kyoto Prefecture). Western blotting was performed by a conventional method. The ChemiDoc Imaging Systems (Bio-Rad, Hercules, CA) was used for detection.

Statistical analyses were performed as described below. All between-group comparisons were tested by one-way or two-way analysis of variance or two-tailed *t-*test, unless otherwise noted. The Prism 7 software (GraphPad Software, La Jolla, CA) was used for calculation.

HCl cells, established from human hepatocellular carcinoma, were infected with a lentivirus expressing a shRNA against the LIPG gene (LIPG shRNA) to generate constitutive LIPG-knockdown cells. As a result, the expression of LIPG was significantly reduced in these cells compared to control cells (Fig. 1A). Furthermore, when these cells were infected with HBV, HBV DNA (Fig. 1B) and cccDNA (Fig. 1C) were significantly reduced in the constitutive LIPG-knockdown cells. In particular, cccDNA was remarkably reduced (Fig. 1C). Next, the LIPG shRNA was also introduced into primary human hepatocytes (PHH) to generate constitutive LIPG-knockdown PHH cells. These cells were also infected with HBV (Fig. 1D), and as a result, similar to the HCl cells, the abundances of LIPG expression (Fig. 1E). HBV DNA (Fig. 1F), cccDNA (Fig. 1G), pregenomic RNA (pgRNA) transcription (Fig. 1H) were significantly reduced in the constitutive LIPG-knockdown PHH cells compared to control cells.

These results demonstrated that shRNA-dependent knockdown of the LIPG gene reduced HBV infection. In addition, these results suggest the involvement of LIPG in HBV infection.

### [Example 2] : LIPGpositively regulates HBV infection in HepG2-NTCP cells

Next, the relationship between the abundance of LIPG expression and HBV infection was investigated. First, a cell line with stable expression of a Flag-tagged LIPG protein was established. A forward primer 5'-TTAGAATTCATGAGCAACTCCGTTC-3' (containing an EcoRl recognition site. SEQ ID NO: 34) and a reverse primer 5'-AATTCTAGATCATCTGCTCATCTGTTGTTGTGGGAAGCTCCACAGTG-3' (containing an *Xba*I recognition site, SEQ ID NO: 35) were used together with a LIPG cDNA plasmid as a template in PCR amplification to prepare a lentivirus transfer plasmid encoding the LIPG gene (SEQ ID NO: 29). The PCR product was digested with EcoRI and *Xba*I*,* and the resulting product was ligated to a similarly digested pLVSIN-CMV Pur Vector (Takara Bio, Otsu, Japan) to obtain pLVSIN-CMV-Flag-tagged LIPG Vector. The FuGENE-HD transfection reagent (Promega, Madison, WI) was used to transfect the pLVSIN-CMV Pur Vector or pLVSIN-CMV-Flag-tagged LIPG Vector together with the Lentiviral High Titer Packaging Mix (Takara Bio) into Lenti-X 293T cells (Takara Bio) to generate virus particles. The supernatant collected 72 hours later was filtered through a 0.22 µm syringe filter, and the resulting virus particles were then transfected into the cells. Seventy-two hours after transfection, these cells were treated with puromycin (5 mg/mL) for antibiotic selection. To avoid clonal bias, a bulk antibiotic-resistant cell population was used for the experiment.

When the abundance of LIPG expression was compared among different liver cell lines. HC1 (KM), PHH, and HepG2-NTCP-C4 cells showed a similar abundance of LIPG expression (Fig. 2A). HepG2-NTCP-C4 cells with overexpression of FLAG-LIPG (HepG2-NTCP-C4-LIPG cells) were established (Fig. 2B), and the attachment and uptake of HBV into cells were assayed in the resulting cells. Heparin, which has been reported to inhibit HBV attachment (Schulze. A. et al.. Hepatology 46: 1759-1768, 2007), and heparanase, an endo-β-D-glucuronidase that cleaves the heparan sulfate side chain in heparan sulfate proteoglycans (HSPGs) (Bame, KJ. Glycobiology,11: 91R-98R,2001), were used as control compounds. Cells (HepG2-NTCP-C4-LIPG cells and HepG2-NTCP-C4 control cells) were pretreated with these compounds for 1 hour and then with HBV at 4°C for 3 hours to allow HBV to attach the cells, washed to wash out HBV, and further incubated at 37°C for 12 hours to detect uptake of HBV(Fig. 2C). In addition, the cells were further cultured in a conventional culture medium for 3 days after HBV inoculation to detect HBV DNA and cccDNA in the cells (Fig. 2C). HBV attachment was detected by qPCR of HBV-DNA at 3 hours after HBV inoculation at 4°C in the presence or absence of the compound (heparin or heparanase). HBV attachment (Fig. 2D), uptake (Fig. 2E), and replication of HBV DNA and cccDNA (Fig. 2F and 2G) were all increased in the HepG2-NTCP-C4-LIPG cells compared to the HepG2-NTCP-C4 control cells. Furthermore, the addition of heparin or the treatment with heparanase reduced this increase to the same level as the control.

Next, the effect of a LIPG inhibitor (GSK-264220A) on HBV attachment and uptake was examined. The LIPG inhibitor inhibited HBV attachment and uptake into HepG2-NTCP-C4-LIPG cells in a concentration-dependent manner (Fig. 2H and 1). At the same time, this inhibitor had no effect on HepG2-NTCP-C4 control cells. Constitutive LIPG-knockdown HepG2-NTCP-C4 cells were generated by introducing LIPH shRNA, and it was found that HBV attachment, uptake, and replication were all significantly reduced in these cells (Fig. 3).

These results demonstrated that LIPG contributes to HBV infection. Interestingly, HBV uptake and replication did not occur in RD cells, which are not derived from a hepatocyte cell line, even when LIPG was overexpressed (Fig. 4).

The question of whether or not the enhancement of HBV infection by LIPG was dependent on NTCP was investigated. Huh7, HepG2, and HepG2-NTCP-C4 cells constitutively overexpressing FLAG-LIPG were established using a lentiviral expression system (Fig. 5A). These cells were infected with HBV, and the cccDNA content was measured 24 or 72 hours later. Although expression of FLAG-LIPG functioned to maintain HBV replication in Huh7 cells (Fig. 5B), no effect of overexpressed LIPG on HBV replication was observed in HepG2 cells (Fig. 5C). In the case of HepG2-NTCP-C4 cells, HBV replication was maintained in the control cells, and was further enhanced by LIPG overexpression (Fig. 5D). At the same time, the abundance of NTCP expression was examined in each of the cell lines, and it was found that the abundance of NTCP expression was much lower in Huh7 and Huh7-LIPG cells than in HepG2-NTCP-C4 cells (Fig. 5E).

Then, NTCP was knocked out in Huh7 cells using the CRISPR-Cas9 gene editing technology to completely eliminate the effect of NTCP in Huh7 and Huh7-LIPG cells. An NTCP KO cell line was established as described below. The FuGENE-HD transfection reagent was used to co-transfect an sgRNA expression vector targeting NTCP, and a non-target control (abm, Richmond, Canada) and the 3rd Generation Packaging System Mix (abm) into Lenti-X 293T cells to generate an sgRNA CRISPR/Cas9 lentivirus. The supernatant collected 72 hours later was filtered through a 0.22 µm syringe filter, and the resulting virus particles were then transfected into Huh7 cells. Seventy-two hours after transfection, these cells were treated with puromycin (5 mg/mL) for antibiotic selection. To avoid clonal bias, a bulk antibiotic-resistant cell population was used for the experiment.

NTCP was detected in the Huh7 cells transfected with a non-target single guide RNA (sgRNA) containing a scramble sequence, but not in the NTCP-KO cells transfected with an NTCP-specific sgRNA (Fig. 5F). These cells were forced to express LIPG, and the attachment and uptake assay was performed on these cells by the method described above. HBV attachment to cells (Fig. 5G), uptake (Fig. 5H), and replication (HBV-DNA, cccDNA, pgRNA transcript) (Fig. 5I, J, and K) were increased to the same levels in both IIuh7-WT and Huh7-NTCP-KO cells by LIPG overexpression.

These results demonstrated that LIPG promotes HBV attachment and uptake in an NTCP-independent manner.

### [Example 3] : LIPG contributes to caveolae-dependent HBV uptake

Next, we investigated whether the LIPG-mediated increase in HBV uptake was clathrin-dependent or caveolae-dependent. When LIPG was overexpressed in Huh7 cells, the expression of caveolin-1 protein was promoted (Fig. 6A). Therefore, caveolin-1 was knocked down using a siRNA specific for caveolin-1, and the effect of the knockdown on LIPG-mediated increases in HBV attachment, uptake and replication was examined. Caveolin-1-specific and negative control siRNAs were obtained from Sigma-Aldrich. The Lipofectamine RNAiMAX Transfection Reagent (Invitrogen) was used to transfect the siRNAs. Caveolin-1 protein expression was detected in Huh7-Cont and Nuh7-LIPG cells transfected with the control siRNA and was significantly reduced in these cells with caveolin-1 knockdown (Fig. 6B). By the knockdown of caveolin-1. the LIPG overexpression-mediated increase in HBV attachment was suppressed (Fig. 6C), and the increases in HBV uptake and replication (cccDNA) were blocked to the same levels as the control (Fig. 6D and E). Furthermore, both chlorpromazine (CPZ), a clathrin-dependent endocytosis inhibitor, and 5-(N-ethyl-N-isopropyl)amiloride (EIPA), a macropinocytosis inhibitor. had no effect on the LIPG-mediated increase in HBV replication in Huh7-LIPG cells (Fig. 6F and 6G), whereas, the LIPG inhibitor reduced HBV replication in 1 Iuh7-LIPG cells (Fig. 6H).

These results indicate that LIPG is involved in caveolae-dependent HBV uptake and promotes HBV replication. In addition, in Huh7 cells with overexpressed LIPG, not only caveolin-1 expression but also vimentin expression was increased (Fig. 7). Since vimentin is known to play an important role in the attachment, entry, and replication of human papilloma virus (HPV), influenza A virus (IAV), dengue virus (DENV), and the like (Ramos, I. et al., Int. J. Mol. Sci. 21: 4675, 2020), the LIPG-mediated increase in vimentin expression was suggested to possibly contribute to the increased HBV attachment, entry, and replication. E-cadherin was recently reported to regulate HBV entry by affecting NTCP distribution (Hu, Q. et al., Front. Cell Infect. Microbiol. 10: 74, 2020), but our results indicated that LIPG overexpression had no effect on E-cadherin expression (Fig. 7). In addition, the expression of E-cadherin protein was much lower in HepG2-NTCP-C4 cells than in Huh7 cells (Fig. 7), suggesting that E-cadherin is not involved in the LIPG-mediated increase in HBV attachment, uptake, and replication.

### [Example 4] : LIPG has no effect on clathrin-dependent viral endocytosis

Furthermore, in order to investigate the effect of LIPG on viral uptake, the replication of hepatitis C virus (HCV), myocarditis virus (EMCV), and influenza virus (Flu), which enter into cells in a clathrin-dependent manner, was examined in Huh7-LIPG cells. Overexpression of LIPG tended to reduce the replication of HCV (Fig. 8A) and EMCV (Fig. 8B) and had no effect on Flu replication (Fig. 8C). This result indicates that LIPG has no effect on clathrin-dependent endocytosis.

The result described above demonstrated that LIPG is a factor playing an important role in HBV attachment and uptake, and functions as a cofactor that supports HBV entry into cells independently of NTCP. This suggests that the binding of HBV to the LIPG-HSPG complexes on the cell membrane surface leads to the uptake of HBV into liver cells by caveolin-dependent endocytosis. This pathway is consistent with the action of LIPG in the uptake of HDL into cells (Gerold, G. Cold Spring Harb. Perspect Med. 10, a036830, 2020), suggesting that HBV uses this pathway for non-specific entry into cells.

Next, aiming to develop peptide drugs useful for complete cure of hepatitis B virus, we used the *in vitro* virus (IVV) method (Nemoto, N. et al., FEBS Lett., 414: 405-408, 1997; Miyamoto-Sato, E. et al., Nucleic Acids Res., 28: 1176-1182, 2000; WO 03/106675 A1) to find a peptide that inhibits the interaction between LIPG endothelial lipase and HSPGs and consequently HBV entry into cells from a random peptide library (with a size in the order of 10¹³). Furthermore, we aimed to elucidate the mechanism of action of LIPG in complete cure of hepatitis B to understand the role of LIPG in hepatitis B virus entry.

### [Example 5] : Preparation of DNA library

### (5-1) Design of a DNA library

The design of a DNA *library* for IVV screening is shown in Fig. 9.

In the IVV method, an antibiotic, puromycin, is attached to the 3' end of mRNAs via a PEG (polyethylene glycol) spacer, and a cell-free translation reaction is performed using the resulting products as a template to generate simple mRNA-protein fusion molecules. IVVs, in which proteins and mRNAs are covalently linked to each other via puromycin (Miyamoto-Sato, E. et al., Nucleic Acids Res., 31: e78, 2003). From the IVV library constructed as described above, an IVV comprising a protein that binds to a bait (a protein, peptide, antigen, or the like) is pulled up *in vitro,* and the gene (mRNA) linked to the protein is amplified by reverse-transcription and PCR. followed by analyzing the base sequence of the amplified gene with a DNA sequencer, so that a protein, peptide, or antibody that binds to the bait can be readily identified.

### (5-2) Preparation of a DNA library

RNase-free water was added to 1 µl of a solution of DNA containing a green fluorescent protein (GFP) sequence, 10 µl of 10 × KOD plus buffer (TOYOBO), 10 µl of 2 mM dNTPs (TOYOBO), 4 µl of 25 mM MgSO₄, 3 µl of a forward primer: GSP6-GFP-F (10 pmol/µl), 3 µl of a reverse primer: GFP-R (10 pmol/µl), and 2 µl of KOD plus polymerase (TOYOBO) to give a total volume of 100 µl, and the resulting mixture was added to a tube. A total of 300 µl of the reaction mixture (in 3 tubes) was subjected to a PCR reaction. The PCR reaction was performed at 94°C for 5 minutes, followed by 16 cycles of 94°C for 30 seconds, 58°C for 30 seconds and 68°C for 2 minutes, and then a reaction at 68°C for 5 minutes. The resulting cDNA library was purified with the Wizard SV Gel PCR Clean-Up System (Promega) and collected as 50 µl of the GSP6-GFP-DNA solution.

RNase-free water was added to 1 µl of a DNA solution containing the A-tail sequence. 10 µl of 10 × KOD plus buffer (TOYOBO), 10 µl of 2 mM dNTPs (TOYOBO), 4 µl of 25 mM MgSO₄, 3 µl of a forward primer: Flag-His-F (10 pmol/µl), 3 µl of a reverse primer: Atail (R) (10 pmol/µl), and 2 µl of KOD plus polymerase (TOYOBO) to give a total volume of 100 µl, and the resulting mixture was added to a tube. A total of 300 µl of the reaction mixture (in 3 tubes) was subjected to a PCR reaction. The PCR reaction was performed at 94°C for 5 minutes, followed by 16 cycles of 94°C for 30 seconds, 58°C for 30 seconds and 68°C for 2 minutes, and then a reaction at 68°C for 5 minutes. The resulting cDNA library was purified with the Wizard SV Gel PCR Clean-Up System (Promega) and collected as 50 µl of the Flag-His Atail-DNA solution.

RNase-free water was added to 1 µl of the Flag-His Atail-DNA solution. 10 µl of 10 × KOD plus buffer (TOYOBO), 10 µl of 2 mM dNTPs (TOYOBO), 4 µl of 25 mM MgSO4, 3 µl of a forward primer: 16NNS-F or 9NNS-F (10 pmol/µl), 3 µl of a reverse primer: Atail (R) (10 pmol/µl), and 2 µl of KOD plus polymerase (TOYOBO) to give a total volume of 100 µl, and the resulting mixture was added to a tube. A total of 300 µl of the reaction mixture (in 3 tubes) was subjected to a PCR reaction. The PCR reaction was performed at 94°C for 5 minutes, followed by 12 cycles of 94°C for 30 seconds, 58°C for 30 seconds and 68°C for 2 minutes, and then a reaction at 68°C for 5 minutes. The resulting cDNA library was purified with the Wizard SV Gel PCR Clean-Up System (Promega) and collected as 50 µl of the 16NNS Atail-DNA solution or 9NNS Atail-DNA solution.

RNase-free water was added to 1 µl of the GSP6-GFP-DNA solution, 1 µl of the 16NNS Atail-DNA solution or 9NNS Atail-DNA solution, 10 µl of 10 × KOD plus buffer (TOYOBO), 10 µl of 2 mM dNTPs (TOYOBO), 4 µl of 25 mM MgSO₄, 3 µl of a forward primer: GSP6-GFP-F (10 pmol/µl), 3 µl of a reverse primer: Atail (R) (10 pmol/µl), and 2 µl of KOD plus polymerase (TOYOBO) to give a total volume of 100 µl, and the resulting mixture was added to a tube. A total of 300 µl of the reaction mixture (in 3 tubes) was subjected to an overlap PCR reaction. The PCR reaction was performed at 94°C for 5 minutes, followed by 12 cycles of 94°C for 30 seconds, 58°C for 30 seconds and 68°C for 2 minutes, and then a reaction at 68°C for 5 minutes. The resulting cDNA libraries were purified with the Wizard SV Gel PCR Clean-Up System (Promega) and collected as 50 µl of the cDNA libraries 16NNSLib and 9NNSLib.

**[Table 1]**

| Table 1. Primer sequences (1) | | |
|---|---|---|
| Name | Sequence (5'→3') | SEQ ID NO |
| GSP6-GFP-F | GGAAGATCTATTTAGGTGACACTATAGAACAACAACAAC AACAAACAACAACAAAATGAGTAAAGGAGAAGAACTTTT CACTGGAG | 36 |
| GFP-R | TTTGTATAGTTCATCCATGCCATGTG | 37 |
| Flag-His-F | GACTATAAGGACGACGACGACAAGCACCATC | 38 |
| A tail (R) | TTTTTTTTTCTTGTAGTAGAACTGGGTGCTGGTCGTAATCC CGCCAATGCC | 39 |
| 16NNS-F | GGATGAACTATACAAANNSNNSNNSNNSNNSNNSNNSNNS NNSNNSNNSNNSNNSNNSNNSNNSGACTATAAGGACGACG ACG | 40 |
| 9NNS-F | CATGGCATGGATGAACTATACAAANNSNNSNNSNNSNNSN NSNNSNNSNNSGACTATAAGGACGACGACG | 41 |

### [Example 6] : Preparation of IVV library

### (6-1) Transcription from a library

RNase-free water was added to 2 pmol each of the cDNA libraries 16NNSLib and 9NNSLib, 8 µl of 5 × SP6 buffer, 2 µl of ATP (100 mM), 2 µl of CTP (100 mM), 2 µl of UTP (100 mM), 4 µl of GTP (10 mM), 5 µl of the cap analog (m7G(5')PPP(5')G) (Thermo Fisher Scientific) (40 mM), and 4 µl of Enzyme Mix SP6 RNA polymerase (Promega) to give a total volume of 40 µl, and the resulting mixture was subjected to a reaction at 37°C for 3 hours. Thereafter, 10 µl of RQl RNase-Free DNase (Promega) was added to the reaction mixture to allow the reaction to continue at 37°C for 1 hour.

The RNA was purified with an RNeasy Mini Kit (Qiagen). That is. RNase-free water was added to the transcription reaction solution to give a total volume of 100 µl, and the resulting mixture was mixed with 350 µl of RLT buffer (Qiagen), 3.5 µl of 2-mercaptoethanol, and 250 µl of (100%) ethanol, and then applied to an RNeasy Mini spin column. After centrifugation at 12000 rpm for 15 seconds at 25°C, the flow-through solution was removed and 500 µl of RPE buffer (Qiagen) was added to the same column. After centrifugation at 12000 rpm for 15 seconds at 25°C, the flow-through solution was removed and another 500 µl of RPE buffer (Qiagen) was added to the same column. After centrifugation at 12000 rpm for 2 minutes at 25°C, the flow-through solution was removed, and the same column was transferred to a new tube, followed by centrifugation at 12000 rpm for 1 minute at 25°C. The same column was again transferred to a new tube and 33 µl of RNase-free water was added to the column, and the column was allowed to stand for 10 minutes at room temperature. The column was then centrifuged at 13200 rpm for 1 minute at 25°C to recover an RNA solution.

### (6-2) Ligation with a PEG spacer

A 32-pl aliquot of the RNA solution was mixed with 5 µl of 10 × T4 ligation buffer, 1 µl of 0.1 M DTT, 0.5 µl of 40 mM ATP, 5 µl of 1 00% DMSO, 1 µl of 0.1% BSA, 1 µl of an RNase inhibitor (TOYOBO), 0.5 µl of a puromycin-added polyethylene glycol photocleavable linker molecule (10 nmol), 1 µl of polyethylene glycol (PEG) 2000 (Nippon Oil & Fats Co., Ltd.) (30 nmol), and 3 µl of T4 RNA ligase (Takara) (40 U/µl), and the reaction was allowed to proceed in the dark at 15°C for 15 hours. The resulting spacer-linked PEG-RNAs were purified with an RNeasy Mini Kit (Qiagen).

### (6-3) Preparation of an IVV library

RNase-free water was added to 10 pmol of PEG-RNAs. 20 µl of a wheat germ extract (ZoeGene), 2 µl of creatine kinase (40 µg/µl) (ZoeGene), 3.2 µl of an RNase inhibitor (TOYOBO), and 20 µl of 5 × translation buffer (ZoeGene) to give a total volume of 100 µl, and the translation reaction was allowed to proceed in the dark at 26°C for 1 hour to prepare an IVV library.

### [Example 7] : Preparation of biotinylated LIPG

Fig. 10 shows a schematic diagram of the binding of endothelial lipase LIPG and heparan sulfate proteoglycan to HBV virus. The design of a biotinylated LIPG serving as a bait is shown in Fig. 11. The results in Examples 1 to 4 suggested that HBV binds to the LIPG-HSPG complexes on the cell membrane surface, leading to the uptake of HBV into liver cells by caveolin-dependent endocytosis. Since LIPG has been reported to bind to heparin in the form of a homodimer (Razzaghi et al., PLoS ONE, 2013, 8(3), e55716), the biotinylated LIPG was configured so that two copies of the heparin-binding region (RKNRCNSIGYNAKKMRNKRNSKMYLKTRA, SEQ ID NO: 42) of LIPG (SEQ ID NO: 30) were linked in tandem.

### (7-1) Vector construction for expression of biotinylated LIPG heparin-binding sites

First, the LIPG-BioFLAGHis-pcDNA vector was prepared, in which the full-length LIPG gene was fused with a biotinylation sequence, a Flag-tag sequence, and a His-tag sequence.

RNase-free water was added to 0.5 µl of LIPG expression vector (ORIGENE) pCMV6-LIPG (1 ng/µl), 12.5 µl of KAPA HiFi HS RM_ 0.75 µl of 10 µM LIPG-ifpc-F, and 0.75 µl of 10 µM LIPG-ifbio-R to give a total volume of 25 µl, and the resulting mixture was subjected to a PCR reaction. The PCR reaction was performed at 95°C for 5 minutes, followed by 25 cycles of 98°C for 20 seconds, 60°C for 15 seconds and 72°C for 1 minute, and then a reaction at 72°C for 1 minute. After a DNA band (1533 bp) was confirmed by agarose gel electrophoresis, the PCR product was purified with the Wizard SV Gel PCR Clean-Up System (Promega) and collected as 30 µl of a DNA solution to obtain LIPG-ifpcB.

The Bio-tag (Nucleic Acids Research, 2009, Vol. 37, No. 8, page e64), consisting of a portion of the oxaloacetate decarboxylase α-subunit gene (corresponding to 72 amino acids) of *Klebsiella pneumoniae* prepared from the BioEase^{™} plasmid by PCR using the F-Bio and R-Bio primers, was fused with a Flag-tag and a His-tag. RNase-free water was added to 2.37 µl of Bio-tag, 40 µl of 10 × KOD plus buffer (TOYOBO). 40 µl of 2 mM dNTPs (TOYOBO), 16 µl of 25 mM MgSO₄, 12 µl of F-Bio (10 pmol/µl), 12 µl of Bio-Flag-Histag A stop (10 pmol/µl), and 8 µl of KOD plus polymerase (TOYOBO) to give a total volume of 200 µl, and the resulting mixture was subjected to a PCR reaction. The PCR reaction was performed at 94°C for 5 minutes, followed by 20 or 25 cycles of 94°C for 30 seconds, 58°C for 30 seconds and 68°C for 2 minutes, and then a reaction at 68°C for 5 minutes. After a DNA band (264 bp) was confirmed by agarose gel electrophoresis, the PCR product was purified with the Wizard SV Gel PCR Clean-Up System (Promega) and collected as 50 µl of a DNA solution to obtain Bio-Flag-His.

RNase-free water was added to 1 µl of the LIPG-ifpcB solution, 1 µl of the Bio-Flag-His solution, 10 µl of 10 × KOD plus buffer (TOYOBO), 10 µl of 2 mM dNTPs (TOYOBO). 4 µl of 25 mM MgSO₄, 3 µl of LIPG-ifpc-F (10 pmol/µl), 3 µl of Flag-Histag A stop (10 pmol/µl), and 2 µl of KOD plus polymerase (TOYOBO) to give a total volume of 100 µl, and the resulting mixture was added to a tube. A total of 300 µl of the reaction mixture (in 3 tubes) was subjected to an overlap PCR reaction. The PCR reaction was performed at 94°C for 5 minutes, followed by 12 cycles of 94°C for 30 seconds. 58°C for 30 seconds and 68°C for 2 minutes, and then a reaction at 68°C for 5 minutes. The resulting cDNA library was purified with the Wizard SV Gel PCR Clean-Up System (Promega) and collected as 50 µl of a DNA solution to obtain LIPG-BioFlagHis.

LIPG-BioFlagHis was introduced into the pcDNA 3.3 vector using the TOPO Cloning Kit (Invitrogen) according to the product instructions. The sequence in the resulting clone was confirmed to be correct, and a colony of the clone was inoculated and cultured at 37°C for 16 hours. The LIPG-BioFlagHis-pcDNA plasmid was purified using the PureYield^{™} Plasmid Maxiprep System (Promega) from the bacterial pellet.

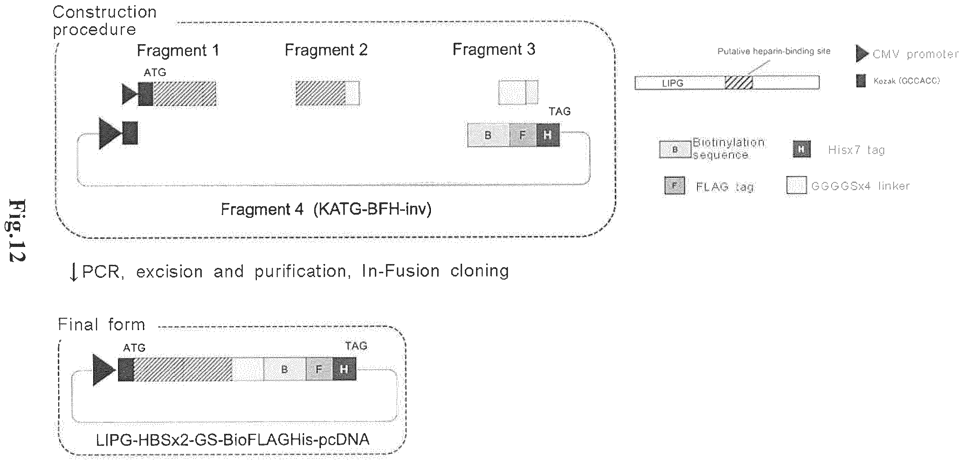

Fragment 2 was prepared from the LIPG-BioFlagHis-pcDNA vector containing the LIPG gene (Fig. 12). RNase-free water was added to 0.5 µl of LIPG-BioFlagHis-pcDNA (1 ng/yl), 12.5 µl of KAPA HiFi HS RM, 0.75 µl of 10 µM LIPG-hb-F2, and 0.75 µl of 10 µM LIPG-hb-if-R2 to give a total volume of 25 µl, and the resulting mixture was subjected to a PCR reaction. The PCR reaction was performed at 95°C for 5 minutes, followed by 25 cycles of 98°C for 20 seconds, 60°C for 15 seconds and 72°C for 1 minute, and then a reaction at 72°C for 1 minute. After a DNA band (102 bp) was confirmed by agarose gel electrophoresis, the PCR product was purified with the Wizard SV Gel PCR Clean-Up System (Promega) and collected as 30 µl of a DNA solution to obtain LIPGhb-2 (Fragment 2).

Fragment 1 was prepared from Fragment 2. RNase-free water was added to 0.5 µl of LIPGhb-2, 12.5 µl of KAPA HiFi HS RM, 0.75 µl of 10 µM LIPG-hb-ifpc-F1, and 0.75 µl of 10 µM L1PG-hb-if-R1 to give a total volume of 25 µl, and the resulting mixture was subjected to a PCR reaction. The PCR reaction was performed at 95°C for 5 minutes, followed by 25 cycles of 98°C for 20 seconds. 60°C for 15 seconds and 72°C for 1 minute, and then a reaction at 72°C for 1 minute. After a DNA band (117 bp) was confirmed by agarose gel electrophoresis, the PCR product was purified with the Wizard SV Gel PCR Clean-Up System (Promega) and collected as 30 µl of a DNA solution to obtain LIPGhb-1 (Fragment 1).

RNase-free water was added to 0.5 µl of a linker, 12.5 µl of KAPA HiFi HS RM, 0.75 µl of 10 µM GSlinker-F1, and 0.75 µl of 10 µM GSlinker-ifbio-R1 to give a total volume of 25 µl, and the resulting mixture was subjected to a PCR reaction. The PCR reaction was performed at 95°C for 5 minutes, followed by 25 cycles of 98°C for 20 seconds, 60°C for 15 seconds and 72°C for 1 minute, and then a reaction at 72°C for 1 minute. After a DNA band (75 bp) was confirmed by agarose gel electrophoresis, the PCR product was purified with the Wizard SV Gel PCR Clean-Up System (Promega) and collected as 30 µl of a DNA solution to obtain GSlink-ifBio (Fragment 3).

RNase-free water was added to 0.5 µl of LIPG-BioFLAGHis-pcDNA, 10 µl of KAPA HiFi HS RM, 0.6 µl of 10 µM F-Bio, and 0.6 µl of 10 µM pcDNA-KATG-inv to give a total volume of 20 µl, and the resulting mixture was subjected to a PCR reaction. The PCR reaction was performed at 95°C for 3 minutes, followed by 25 cycles of 98°C for 20 seconds, 60°C for 15 seconds and 72°C for 3 minutes, and then a reaction at 72°C for 1 minute. After a DNA band (5662 bp) was confirmed by agarose gel electrophoresis, the PCR product was purified with the Wizard SV Gel PCR Clean-Up System (Promega) and collected as 30 µl of a DNA solution to obtain KATG-BFH-inv (Fragment 4).

The 5x In-Fusion HD Enzyme Premix (Takara) in a volume of 1.0 µl was mixed with 1.0 µl of LIPGhb-1 (Fragment 1), 1.0 µl of LIPGhb-2 (Fragment 2), 1.0 µl of GSlink-ifBio (Fragment 3), and 1.0 µl of KATG-BFH-inv (Fragment 4), and the reaction was allowed to proceed at 50°C for 15 minutes. One Shot TOP10 competent cells were transformed with 2.5 µl of the reaction mixture and cultured overnight at 37°C to obtain a clone. The identity of the clone was confirmed by sequence analysis to be the plasmid shown in Fig. 12, LIPG-HBSx2-GS-BioFLAGHis-pcDNA.

**[Table 2]**

| Table 2. Primer sequences (2) | | |
|---|---|---|
| Name | Sequence (5'→3') | SEQ ID NO |
| LIPG-ifpc-F | CGGACTCTAGAGGATGCCACCATGAGCAACTCCGT | 43 |
| LIPG-ifbio-R | CGGGGTGCCGGCGCCGGGAAGCTCCACAGT | 44 |
| F-Bio | GGCGCCGGCACCCCGGTGAC | 45 |
| R-Bio | CGCCAGGGTCATCAGGGTGT | 46 |
| Bio-Flag-Histag A stop | CTAATGGTGATGGTGGTGATGGTGCTTGTCGTCGTC GTCCTTGTAGTCCGCCAGGGTCATCAGGGTGT | 47 |
| Flag-Histag A stop | CTAATGGTGATGGTGGTGATGGTGCTTGTCGTCGTC GTCCTTGTAGTC | 48 |
| LIPG-hb-ifpc-F1 | GAGGATGCCACCATGCGCAAGAACCGTTGTAATAG | 49 |
| LIPG-hb-if-R1 | ACAACGGTTCTTGCGTGCCCGGGTTTTTAGGTACA | 50 |
| LIPG-lib-F2 | CGCAAGAACCGTTGTAATAG | 51 |
| LIPG-hb-if-R2 | TGAACCGCCTCCACCTGCCCGGGTTTTTAGGTACA | 52 |
| Linker | GGTGGAGGCGGTTCAGGCGGAGGTGGCTCTGGCGG TGGCGGATCCGGAGGTGGTGGCAGC | 53 |
| GSlinker-F1 | GGTGGAGGCGGTTCAGGCGG | 54 |
| GSlinker-ifbio-R1 | CGGGGTGCCGGCGCCGCTGCCACCACCTCCGGATC | 55 |
| pcDNA-KATG-inv | CATGGTGGCATCCTCTAGAGTCC | 56 |

### (7-2) Expression and purification of biotinylated LIPG heparin-binding sites

Human embryonic kidney 293T cells were cultured in DMEM (1.0 g/l glucose) with L-Gln and Sodium Pyruvate, liquid (Nacalai) supplemented with 10% FCS (NICHlREI). Plating was performed in a 1:2 ratio on 6 cm dishes 24 hours before transfection. Lipofectamine 2000 in a volume of 20 µl was added to 500 µl of Opti-MEM I and allowed to stand for 5 minutes at room temperature, and the resulting mixture was gently added to each dish containing a mixture of 500 µl of Opti-MEM I and the LIPG-HBSx2-GS-BioFLAGI-Iis-pcDNA plasmid DNA. The resulting mixture was allowed to stand for 20 minutes at room temperature, and then added to a dish containing 293T cells, and the cells were cultured at 37°C in 5% CO₂ for 1 day.

The culture medium was removed from the dishes, and the cells were washed with 2 ml of cold PBS. To the cells, 800 µl of RIPA buffer (25 mM Tris-HCl, 150 mM NaCl, 1% NP-40, 1% sodium deoxycholate, 0.1% SDS, pH 7.6), 16 µl of 50x protease inhibitor for use with animal cell extracts (Cat#25955-11) (4-(2-aminoethyl)benzenesulfonyl fluoride hydrochloride (AEBSF), aprotinin, E-64, leupeptin hemisulfate monohydrate, bestatin, pepstatin A), and 8 µl of 0.1 M PMSF were added, and the resulting mixture was allowed to stand on ice for 15 minutes. The cells were scraped with a cell scraper, collected in a tube, homogenized with a 21-G injection needle, and then allowed to stand on ice for 60 minutes. After centrifugation at 12,300 rpm for 30 minutes at 4°C. the supernatant was transferred to a new tube as the cell fraction. The cell fraction was obtained from a total of two 6 cm dishes of transfected cells. This fraction was used for purification of biotinylated LIPG heparin-binding sites. TALON magnetic beads (Clontech) in a volume of 500 µl (50% slurry) were washed three times with 5.0 ml of TBS buffer (Nacalai) and then mixed with 8.0 ml of the cell fraction described above, and the resulting mixture was agitated overnight at 4°C by rotation on a mini-disk rotor (BIO CRAFT). The beads were washed three times with 5.0 ml of TBS buffer and mixed with 500 µl of TBS containing 500 mM imidazole, and the resulting mixture was agitated at 4°C for 1 hour by rotation on a mini-disk rotor, and then the supernatant was collected (elution fraction E1). To the remaining beads. 250 µl of TBS containing 500 mM imidazole was added, and the elution fraction E2 was collected by the same procedure as described above. Elution fraction E1 in a volume of 15 µl was mixed with 6.8 µl of LDS sample buffer (4x) containing 0.2 mM DTT, heated at 70°C for 10 minutes, and then subjected to SDS-PAGE. SDS-PAGE was performed using 4-12% Bis-Tris NuPAGE gel and MES electrophoresis buffer (Invitrogen) at 200 V and 400 mA for 35 minutes. After electrophoresis, the gel was stained with SimplyBlue^{™} SafeStain (Invitrogen), and biotinylated LIPG heparin-binding sites were successfully purified as a band with a molecular weight of 17,508 Da.

### [Example 8] : Selection of LIPG-binding peptides

An experiment to select LIPG-binding peptides was performed according to the procedure shown in Fig. 13.

### (8-1) Immobilization of LIPG

For Biacore, a Biacore 3000 system was used and the biotinylated LIPG was immobilized on a sensor chip SA. The flow of HBS-P buffer (10 mM HEPES-NaOH, pH 7.4, 150 mM NaCl, 0.005% Tween-20) was performed at a flow rate of 10 µl/min. A solution containing 50 mM NaOH and 1 M NaCl in a volume of 10 µl was injected three times into flow cells 1 to 4 as a pretreatment prior to immobilization. A biotinylated LIPG, HBSx2-GS-BioFLAGHis (0.7 nM), was used and immobilized on flow cells 1 to 4. The flow of HBS-P buffer was performed at a flow rate of 20 µl/min. By manual injection of 100 µl of biotinylated LIPG, a total of 807 RU of biotinylated LIPG was bound to flow cells 1 to 4. The sensor chip was washed with HBS-P buffer running at a flow rate of 10 µl/min, and a solution containing 50% isopropanol, 50 mM NaOH, and 1 M NaCl was used for extra washing.

### (8-2) Selection of LIPG-binding peptides

To the prepared IVV library in a volume of 100 µl, 4 µl of 0.5 M EDTA was added to give a final concentration of 20 mM, and the resulting mixture was agitated by rotation for 20 minutes at room temperature. A column (Bio-Rad) was packed with 1 ml of Sephadex G200 gel (Amersham Biosciences) that had been swollen and equilibrated with HBS-P, and the IVV library solution in a volume of 100 µl was applied to the column. Two drops from the column were added to each well of a 96-well plate, and the 1st to the 10th wells were recovered. Fluorescence from each well was detected using the Multi-detection Microplate Reader POWERSCAN HT at an excitation wavelength of 485 nm and a fluorescence wavelength of 528 nm, and the IVV fractions eluted in the 3rd to 6th wells were pooled. The resulting IVV fraction in a volume of approximately 200 µl was added to 100 µl of Strep Magne Sphae Paramagnetic Part (9013-20-1), which had been washed three times with 500 µl of HBS-P, and agitated by rotation for 20 minutes at room temperature, and the supernatant was injected into the Biacore (Fig. 14). The selection on the Biacore was performed by using HBS-P buffer at a flow rate of 40 µl/min with an association time of 240 seconds and a dissociation time of 5000 seconds, and then the bound analyte was eluted with 7 µl of 10 µM heparin sodium salt (17513-41, Nacalai) in accordance with the Biacore recovery procedure. The selection pressure in the selection experiment was progressively increased round by round, as shown in Table 3.

**[Table 3]**

| Table 3. Selection pressures in LIPG screening experiments | | | | |
|---|---|---|---|---|
| | 1st round | 2nd round | 3rd round | 4th round |
| Amount of immobilized bait | 807 RU | 545.4 RU | 438.9 RU | 423.1 RU |
| Time for association with IVVs | 240 s | 160 s | 120 s | 96 s |
| Wash flow rate in Biacore | 40 ul/min | 60 µl/min | 80 µl/min | 100 µl/min |
| Wash time in Biacore | 5000 s | 17000 s | 71500 s | 67000 s |
| Heparin concentration | 10 µM | 2 µM | 0.5 µM | 0.2 µM |

### (8-3) Recovery of cDNA library by RT-PCR

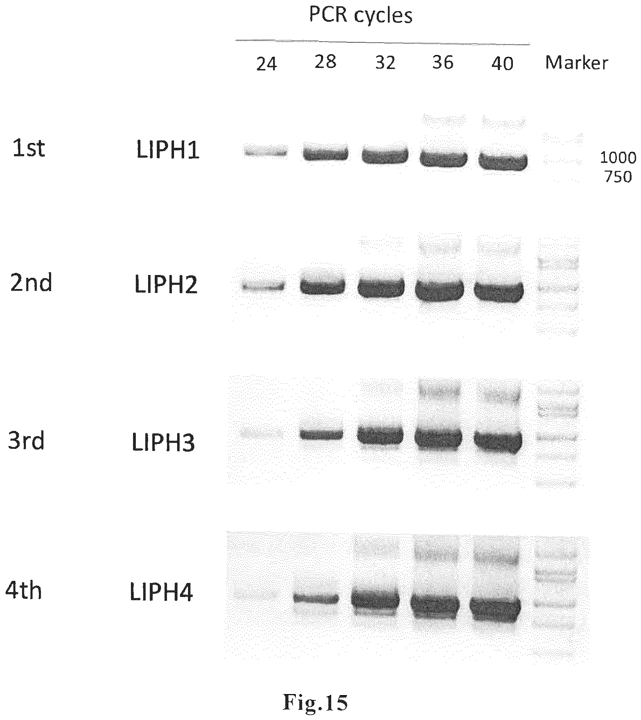

RNase-free water was added to 7 µl of the eluted solution collected in the selection experiment, 20 µl of 5 × RT buffer (TOYOBO), 10 µl of (10 mM) dNTPs (TOYOBO), and 5 µl of a reverse primer: Atail (10 pmol/µl) to give a total volume of 90 µl, and the resulting mixture was reacted at 65°C for 9 minutes and then immediately cooled on ice for 2 minutes. To the resulting mixture, 5 µl of ReverTra Ace (TOYOBO) and 5 µl of an RNase inhibitor (TOYOBO) were added, and the reverse transcription reaction was allowed to proceed at 50°C for 30 minutes and at 99°C for 5 minutes. RNase-free water was added to 100 µl of the reaction solution after the reverse transcription reaction, 100 µl of 10 × KOD plus buffer (TOYOBO), 100 µl of 2 mM dNTPs (TOYOBO), 40 µl of 25 mM MgSO₄, 30 µl of a forward primer: GSP6omega F (10 pmol/µl), 30 µl of a reverse primer: Atail (10 pmol/µl), and 20 µl of KOD plus polymerase (TOYOBO) to give a total volume of 1000 µl, and the resulting mixture was subjected to a PCR reaction. The PCR reaction was performed at 94°C for 5 minutes, followed by 20 to 40 cycles of 94°C for 30 seconds, 58°C for 30 seconds and 68°C for 2 minutes, and then a reaction at 68°C for 5 minutes. The result of electrophoresis after the PCR reaction is shown in Fig. 15.

### [Example 9] : Cloning and determination of base sequences

### (9-1) Cloning and base sequences

In-fusion cloning from a peptide library (LIPG)

### Preparation of inserts for a library

RNase-free water was added to 1 µl of the L1PH-4 library obtained after 4 rounds of selection experiments. 100 µl of 10 × KOD plus buffer (TOYOBO), 100 µl of 2 mM dNTPs (TOYOBO), 40 µl of 25 mM MgSO₄, 30 µl of GFP-F in (10 pmol/µl), 30 µl of His-Atail-R in (10 pmol/µl), and 20 µl of KOD plus polymerase (TOYOBO) to give a total volume of 1000 µl, and the resulting mixture was subjected to a PCR reaction. The PCR reaction was performed at 94°C for 5 minutes, followed by 8 cycles of 94°C for 30 seconds, 58°C for 30 seconds and 68°C for 2 minutes, and then a reaction at 68°C for 5 minutes. The resulting cDNA library was purified with the Wizard SV Gel PCR Clean-Up System (Promega) and collected as 50 µl of a DNA solution to obtain GFP-LIPH4 in.

RNase-free water was added to 8 µl of KIgk-GFP-FlagHis, 80 µl of 10 × KOD plus buffer (TOYOBO). 80 µl of 2 mM dNTPs (TOYOBO), 32 µl of 25 mM MgSO₄, 24 µl of Atail-F iv (10 pmol/µl), 30 µl of 3.3KIgk-GFP-Riv (10 pmol/µl), and 16 µl of KOD plus polymerase (TOYOBO) to give a total volume of 800 µl, and the resulting mixture was subjected to a PCR reaction. The PCR reaction was performed at 94°C for 2 minutes, followed by 25 cycles of 98°C for 10 seconds, 68°C for 5 minutes and 35 seconds. the resulting cDNA library was purified with the Wizard SV Gel PCR Clean-Up System (Promega) and collected as 40 µl of a DNA solution to obtain the KIgk-GFP-Atail 3.3 vector.

RNase-free water was added to a mixture of 0.08 µl of GFP-LIPH4 in, 0.14 µl of the KIgk-GFP-Atail 3.3 vector, and 1.0 µl of 5x In-Fusion HD Enzyme Premix (Takara) to give a total volume of 5 µl, and the reaction was allowed to proceed at 50°C for 15 minutes. One Shot TOP 10 competent cells were transformed with 2.5 µl of the solution after the reaction and cultured overnight at 37°C to obtain clones. The resulting clones were sequenced using the ValueRead Premix provided by the Eurofins DNA Sequencing Service.

**[Table 4]**

| Table 4. Primer sequences (3) | | |
|---|---|---|
| Name | Sequence (5'→3') | SEQ ID NO |
| GFP-F in | ATGAGTAAAGGAGAAGAACTTTTCACTGGAG | 57 |
| His-Atail-R in | ACCGATCACAAACGGATGGTGATGGTGGTG | 58 |
| Atail-F iv | CCGTTTGTGATCGGTGCCGGTGTTCTGGGC | 59 |
| 3.3KIgk-GFP-Riv | TTCTCCTTTACTCATGTCACCAGTGGAACCTGGAACCC AGAGCAGC | 60 |

### (9-2) Library sequencing

Sequencing of the library revealed that 8 clones having 16 amino acid residues and 12 clones having 9 amino acid residues, as shown in Table 5, were obtained.

**[Table 5]**

| Table 5. Amino acid sequences of LIPG-binding peptides obtained in IVV screening | | | |
|---|---|---|---|
| Clone name | Number of amino acid residues | Amino acid sequence of random peptide portion | SEQ ID NO |
| KIgk-LIPH4-23 | 16 | LNCRDNTRPVMSAMTC | SEQ ID NO:1 |
| KIgk-LIPI14-44 | 16 | SCSFAVLSDTQPQAHS | SEQ ID NO: 2 |
| KIgk-LIPH4-73 | 16 | DAFAATARDEYRAARN | SEQ ID NO: 3 |
| KIgk-LIPH4-90 | 16 | LCECRDVLSCYYITDT | SEQ ID NO: 4 |
| KIgk-LIPH4-42 | 16 | LGPGVETCSWQCCSDL | SEQ ID NO: 5 |
| KIgk-LIPH4-17 | 16 | CMLDRLACFGEILLVQ | SEQ ID NO: 6 |
| KIgk-LIPH4-4 | 16 | PRYHWNSTSMFNSESP | SEQ ID NO: 7 |
| KIgk-LIPH4-16 | 16 | CPCVNGATRHRPTSLC | SEQ ID NO: 8 |
| KIgk-LIPH4-74 | 9 | IRNVNHSDH | SEQ ID NO: 9 |
| KIgk-LIPH4-81 | 9 | NSSDARGCD | SEQ ID NO: 10 |
| KIgk-LIPH4-87 | 9 | GADLRRGCC | SEQ ID NO: 11 |
| KIgk-LIPH4-94 | 9 | AMHIDRRFR | SEQ ID NO: 12 |
| KIgk-LIPH4-64 | 9 | DLSFSAPNF | SEQ ID NO: 13 |
| KIgk-LIPH4-14 | 9 | RCRQSWNTM | SEQ ID NO: 14 |
| KIgk-LIPH4-2 | 9 | SLYTGFRAH | SEQ ID NO: 15 |
| KIgk-LIPH4-8 | 9 | MRKVSVKTS | SEQ ID NO: 16 |
| KIgk-LIPH4-12 | 9 | LVPWLRYAY | SEQ ID NO: 17 |
| KIgk-LIPH4-15 | 9 | LNVGYVFYP | SEQ ID NO: 18 |
| KIgk-LIPH4-70 | 9 | PPAQRSYIQ | SEQ ID NO: 19 |
| KIgk-LIPH4-71 | 9 | SRSPPGRRM | SEQ ID NO: 20 |

### [Example 10] : Evaluation of the activities of clones

### (10-1) Preparation of proteins

In-frame clones were inoculated from master plates into LB medium containing 20 pg/ml carbenicillin and incubated at 37°C for 16 hours. Plasmids were purified from the bacterial pellets using the PureYield^{™} Plasmid Miniprep System (Promega).

### (10-2) Preparation of proteins

Human embryonic kidney 293T cells were cultured in DMEM (1.0 g/l glucose) with L-Gln and Sodium Pyruvate, liquid (Nacalai) supplemented with 10% FCS (NICHIREI). Plating was performed in a 1:2 ratio on 6 cm dishes 24 hours before transfection. Lipofectamine 2000 in a volume of 20 µl was added to 500 µl of Opti-MEM I and allowed to stand for 5 minutes at room temperature, and the resulting mixture was gently added to a 6 cm dish containing a mixture of 500 µl of Opti-MEM I and 8 µg of a plasmid DNA. The resulting mixture was allowed to stand for 20 minutes at room temperature, and then added to a 6 cm dish containing 293T cells. and the cells were cultured at 37°C in 5% CO₂ for 4 to 6 day.

### (10-3) Pull-down experiment

Resin (Streptavidin MagneSphere Paramagnetic Particles) (Promega) was washed using the MagneSphere Technology Magnetic Separation Stand (tweleve-position). The resin in a volume of 40 µl was added to each 1.5 ml tube, and after removal of the solution, washed three times with 1 ml of PBS. The resin was transferred to new tubes and mixed with 0.5 nmol (109 µl) of LIPG-BioFLAGHis or with 109 µl of PBS as a no bait control, and the resulting mixtures were agitated at 4°C for 1 hour by rotation on a mini-disk rotor (BIO CRAFT) to allow binding. After removal of the solution, the resin was washed three times with 1 ml of TBST (containing 0.05% Tween 20). The resin was transferred to new tubes, blocked with 1 ml of ELISA BSA buffer (TBS, 0.05%, Tween 20, 1% BSA. Nacalai Tesque, Inc.) at 4°C for 1 hour, and then washed three times with 1 ml of ELISA BSA buffer. The resin was transferred to new tubes, and 1000 µl of the culture supernatant containing expressed KIgk-LIPH4 was mixed with 40 µl of the resin treated with LIPG-BioFLAGHis or without the bait, and the resulting mixtures were agitated at 4°C for 1 hour and 30 minutes by rotation on a mini-disk rotor (BIO CRAFT) to allow binding. After removal of the solution, the resin was washed three times with 500 µl of TBST (Tween 20, 0.1%), and the recovered resin was used for Western blotting.

Each resin was mixed with water and LDS sample buffer (4x), 0.2 mM DTT, heated at 70°C for 10 minutes, and then subjected to SDS-PAGE. SDS-PAGE was performed using 4-12% Bis-Tris gel and NuPAGE MES-SDS electrophoresis buffer (Invitrogen) at 200 V and 400 mA for 35 minutes. After electrophoresis, transfer was performed using Mini Format, 0.2-µm PVDF, Single-application (BIO-RAD) Trans-Blot Turbo. The membrane was blocked with a Blocking One Buffer : TBST (1:9) solution and reacted with an anti-Flag-HRP (Sigma: A8592) diluted 2:3000 in a Blocking One Buffer : TBST (1:9) solution. Detection was performed using ECL (Enhanced ChemiLuminescence) on a ChemiDoc (BIO-RAD).

A pull-down assay was performed on the full-length form of LIPG (500 amino acids) with LIPH4 peptides (Fig. 16). Twenty LIPH4 clones selected by 4 rounds of selection experiments were tested and 9 clones (KIgk-LIPH4-23 (SEQ ID NO: 1), KIgk-LIPH4-90 (SEQ ID NO: 4), KIgk-LIPH4-16 (SEQ ID NO: 8). KIgk-LIPH4-74 (SEQ ID NO: 9), KIgk-LIPH4-87 (SEQ ID NO: 11), KIgk-LIPH4-14 (SEQ ID NO: 14), KIgk-LIPH4-2 (SEQ ID NO: 15), KIgk-LIPH4-12 (SEQ ID NO: 17), KIgk-LIPH4-15 (SEQ ID NO: 18)) were found to be positive.

### (10-4) Cell culturing

A DMEM (ThermoFisher Scientific) medium supplemented with 10% FBS (Thermo Fisher Scientific), 1% L-glutamine (Thermo Fisher Scientific), and 1% penicillin/streptomycin (Thermo Fisher Scientific) was used to culture HepG2-NTCP-C4 cells, HepG2-NTCP-C4-Dox-inducible DOCK11-Halo cells (HepG2-NTCP-C4 cells expressing Halo-tagged DOCK11 under the control of the Tet inducible expression system), HepG2.2.15, HepG292 (HepG2 cells with stable expression of a helper plasmid that lacks the packaging signal (ε) located upstream of the HBV core gene), and Huh7-NTCP-YFP cells (Huh7 cells with stable expression of YFP-fused NTCP) in a 5% CO₂ incubator at 37°C.

### (10-5) Infection experiment using PXB cells

An infection source medium was prepared by adding the HBV-infected chimeric mouse serum (PhoenixBio), DMSO, and a PEG 8000 solution to the PXB cell culture medium (PhoenixBio) at final concentrations of 5 genome equivalents/cell, 2%, and 4%, respectively. Various synthesized peptides were diluted to different concentrations in this medium, and the resulting media were added individually to PXB cells (PhoenixBio). After the cells were left to stand in a 5% CO₂ incubator at 37°C for 20 to 28 hours, the medium was removed and the wells were washed once with PBS(-). DMSO and one of the various synthesized peptides were added to the PXB cell culture medium at a final concentration of 2% and different concentrations, respectively, and the resulting media were added individually to the PXB cells to perform medium exchange. This medium exchange was performed at 1, 5, and 9 days post infection and samples were collected at 14 days post infection.

### (10-6) Quantification of HBV-DNA and cccDNA

SMI TEST^{™} EX-R&D (Medical & Biological Laboratories) was used to extract DNA from the samples. The extracted DNA was used as a template for quantitative PCR of HBV-DNA. For cccDNA detection, the extracted DNA was treated with T5 exonuclease (New England Biolabs). and the resulting DNA was used as a template for quantitative PCR. The T5 exonuclease treatment was performed at 37°C for 30 minutes, and the DNase was later inactivated by heating at 80°C for 15 minutes. The following primer set was used for detection. Forward primer: 5'-ACTCACCAACCTCCTGTCCT-3' (SEQ ID NO: 61) Reverse primer: 5'-GACAAACGGGCAACATACCT-3' (SEQ ID NO: 62) TaqMan probe: 5'-(FAM)-TATCGCTGGATGTGTCTGCGGCGT-(TAMRA)-3'

### (SEQ ID NO: 63)

### (10-7) Infection experiment using HBV derived from HepG2.2.15

Cells were infected with HBV derived from HepG2.2.15 at 20,000 genome equivalents/cell, and plasmids carrying different peptides were transfected individually into the cells 12 to 15 days post infection using Lipofectamine 3000. Samples were collected 3 to 5 days after transfection for evaluation of HBV-DNA, cccDNA, pgRNA, and the like.

### [Example 11] : Anti-HBV activities of screened LIPG-binding peptides

### (11-1) Anti-HBV activities of LIPG-binding peptides in HcpG2-NTCP-C4 cells

For the clones positive in the pull-down experiment against full-length LIPG, the anti-HBV activities of the LIPG peptides were evaluated in HepG2-NTCP-C4 cells (Fig. 17). A secretion signal is added to a plasmid carrying each peptide. Therefore, the peptides are expected to be secreted from cells transfected with the plasmids and to bind to LIPG in the extracellular environment. Addition of KIgk-LIPH4-23 reduced the copy number of HBV-DNA to near zero. In addition, each of the other clones that were positive in the pull-down experiment also reduced the copy number of HBV DNA as compared to the control. Moreover, the copy number of cccDNA was reduced by almost all the clones.

### [Example 12] : Anti-HBV activities of LIPG-binding peptides in PXB cells

PXB cells, fresh human liver cells isolated from PXB mice (human hepatocyte chimeric mice), are human liver cells that maintain human liver functions at high levels and allow persistent infection with hepatitis B virus (HBV). Therefore, PXB cells were used to evaluate the anti-HBV activities of LIPG-binding peptides. LIPG-binding peptides were chemically synthesized because they were added extracellularly (Eurofins Genomics K.K.). Clones that had shown excellent results in the studies of Examples 10 and 11 (Klgk-LIPH4-23, KIgk-LIPH4-74, KIgk-LIPH4-15, KIgk-LIPH4-2. KIgk-LIPH4-14, KIgk-LIPH4-87) were selected for chemical synthesis of the peptides. In addition, LIPH4-23 (SEQ ID NO: 1), which had remarkable anti-HBV activity in HepG2-NTCP-C4 cells, was revealed through BLASTP homology search to have high homologies to netrins (Netrin-1 and Netrin-3). Therefore, LIPH4-NTNA and LIPH4-NTNB, which share a common sequence with netrins and consist of 16 amino acids, were also chemically synthesized. The chemically synthesized peptides were dissolved in DMSO for use in HBV assays in PXB cells.

**[Table 6]**

| Table 6. Amino acid sequences of chemically synthesized LIPG-binding peptides | | | |
|---|---|---|---|
| Peptide name | Number of amino acid residues | Amino acid sequence | SEQ ID NO |
| LIPH4-23 | 16 | LNCRDNTRPVMSAMTC | SEQ ID NO: 1 |
| LIPH4-74 | 9 | IRNVNHSDH | SEQ ID NO: 9 |
| LIPH4-15 | 9 | LNVGYVFYP | SEQ ID NO: 18 |
| LIPH4-2 | 9 | SLYTGFRAH | SEQ ID NO: 15 |
| LIPH4-14 | 9 | RCRQSWNTM | SEQ ID NO: 14 |
| LIPH4-87 | 9 | GADLRRGCC | SEQ ID NO: 11 |
| LIPH4-NTNA | 16 | RKSGGVCLNCRHNTAG | SEQ ID NO: 21 |
| LIPH4-NTNB | 16 | LNCRHNTAGRHCHYCK | SEQ ID NO: 22 |

The anti-HBV activities of the LIPG-binding peptides in PXB cells are shown in Fig. 18. In general. LIPH4-23, which had remarkable anti-HBV activity in HepG2-NTCP-C4 cells, and LIPH4-NTNB, which shares a sequence with netrins, exhibited high anti-HBV activity. Therefore, peptides designed by reducing the number of amino acid residues in these two peptides to 8 (LIPH4-23s and LIPH4-NTNBs) were next chemically synthesized. As a result, the peptides became soluble in PBS (water soluble). The anti-HBV activities of these two LIPG-binding peptides in PXB cells are shown in Fig. 19. The result demonstrated that LIPH4-23s, and LIPH4-NTNBs. which shares a sequence with Netrin-1, significantly reduced HBV-DNA and cccDNA copy numbers and exhibited very high anti-HBV activity.

In the amino acid sequence of LIPH4-NTNBs, C (cysteine residue) is located at the second position from the N-terminus. Since the cysteine residue may mediate dimerization, in order to understand the function of the cysteine residue in LIPH4-NTNBs, a peptide designed by replacing the cysteine residue with an alanine residue (LIPH4-NTNBsA) was chemically synthesized and the anti-HBV activity of the resulting peptide was examined (Fig. 20). Fig. 20 shows the ratio of copy numbers of HBV-DNA and the ratio of copy numbers of cccDNA in PXB cells between two LIPG-binding peptides, LIPH4-NTNBsA and LIPH4-NTNBs, in each concentration (Fig. 20A. Fig. 20B). LIPH4-NTNBsA exhibited activity equal to or greater than that of LIPH4-NTNBs in terms of copy number of both HBV-DNA and cccDNA. Consequently, the cysteine residue was found to have no effect on anti-HBV activity.

**[Table 7]**

| Table 7. Amino acid sequences of chemically synthesized LIPG-binding peptides | | | |
|---|---|---|---|
| Peptide name | Number of amino acid residues | Amino acid sequence | SEQ ID NO |
| LIPH4-23s | 8 | LNCRDNTR | SEQ ID NO: 23 |
| LIPH4-23SA | 8 | LNARDNTR | SEQ ID NO: 24 |
| LIPH4-NTNBs | 8 | NCRHNTAG | SEQ ID NO: 25 |
| LIPH4-NTNBsA | 8 | NARHNTAG | SEQ ID NO: 26 |

Next, the anti-HBV activity of full-length Netrin-1 was examined in PXB cells. A full-length human recombinant Netrin-1 protein consisting of 604 amino acid residues (molecular weight: 67.5 kDa, SEQ ID NO: 28) was purchased from R&D Systems. United States. A shRNA with the sequence CCGGGACTGCGATTCCTACTGCAAGctcgagcTTGCAGTAGGAATCGCAGTC TTTTTTG (single underline: terminal sequences for cloning, lowercase letters: hairpin loop sequence) (SEQ ID NO: 64) was used to knock down the Netrin-1 gene. The culture conditions for the HBV assay were the same as shown in Fig. 19A.

As shown in Figs. 21A and 21B, the full-length form of Netrin-1 at a concentration of 100 nM remarkably reduced HBV-DNA and cccDNA copy numbers compared to the control. In addition, when the Netrin-1 gene was knocked down using the shRNA, HBV-DNA and cccDNA copy numbers were remarkably increased compared to the control (Fig. 21C, Fig. 21D).

The result described above suggests that LIPH4-NTNBs. a LIPG-binding peptide that shares a sequence with Netrin-1, and full-length Netrin-1 inhibit HBV entry into cells. To further examine this suggestion, the Pre-S1 domain of HBV, which is a human hepatocyte recognition site and important for HBV infection. was used to investigate attachment and entry into cells. The preS1 region is known to be important for HBV infection of liver cells (Iwamoto, M. et al., Pro. Natl. Acad. Sci. USA, 116, 8487-8492, 2019). Therefore, a preS1 peptide (SEQ ID NO: 66) was prepared that carried the 2nd to 48th residues of the preS 1 region (SEQ ID NO: 65) and was modified with TAMRA at the C-terminus and a myristoyl group at the N-terminus. The preS1 peptide and PXB cells (primary human hepatocytes) were used to test the effects of LIPG, LIPG-binding peptides, and full-length Netrin-1 on HBV attachment or entry. PXB cells were treated with FLAG-tagged LIPG (FLAG-LIPG) to find that the preS1 peptide present in the cells was increased by FLAG-LIPG in a concentration-dependent manner (Fig. 22A). This result confirmed that LIPG enhances HBV attachment and entry.

Next, the effects of LIPG-binding peptides, LIPH4-23S and LIPH4-NTNBS. on LIPG-mediated HBV attachment and entry were investigated. PXB cells pretreated with a LIPG-binding peptide were treated with FLAG-LIPG. and the uptake of preS1 into the cells was examined, to find that the LIPG-binding peptides inhibited the uptake of the preS1 peptide (Fig. 22B).

From the above results, the LIPG-binding peptides were revealed to inhibit LIPG-mediated HBV attachment and entry by binding to LIPG. LIPG-binding peptides bind to both LIPG and HSPGs, suggesting the possibility that HBV attachment is inhibited by inhibiting the binding between LIPG and HSPGs. In addition. LIPG has been suggested to contribute to caveolae-dependent endocytosis of HBV (see Example 3), and it is known that HBV is first enriched by binding to polysaccharides, including HSPGs, on the cell surface and then binds to receptors such as NTCP to enter into cells (Watashi, K., Wakita. T. Cold Spring Harb. Perspect. Med., 5: a021378, 2015). Therefore, it is suggested that LIPG-binding peptides may inhibit binding between HSPGs and HBV, thereby reducing clathrin-dependent endocytosis of HBV.

LIPH4-23S has a homology to the sequence of Netrin-1, and LIPH4-NTNBS mimics the sequence ofNetrin-1. Therefore, the effect of Netrin-1 on HBV entry was next tested. PXB cells were treated with Netrin-1. and the uptake of the preS 1 peptide into the cells was examined, to find that the uptake of the preS 1 peptide was inhibited by Netrin-1 in a concentration-dependent manner (Fig. 22C).

Furthermore, PXB cells treated with Netrin-1 were further treated with FLAG-LIPG, and the uptake of the preS1 peptide was examined, to find that the enhancement of the uptake of the preS1 peptide by FLAG-LIPG was suppressed by Netrin-1 (Fig. 22D). In addition, a biotin-labeled heparan sulfate was immobilized on a streptavidin-coated plate and the preS1 peptide was added thereto to study the binding between the preS1 region of HBV and heparan sulfate present on the cell surface. The detected TMR fluorescence was increased by the addition of the preS 1 peptide, indicating that the preS1 region can bind to heparan sulfate by itself (Fig. 22E). Moreover, this binding was enhanced by FLAG-LIPG in a concentration-dependent manner, suggesting that the binding between preS1 and heparan sulfate is enhanced by LIPG (Fig. 22E). Additionally, LIPG-mediated enhancement of binding was also inhibited by Netrin-1, LIPG-binding peptides LIPH4-NTNBS and LIPH4-23S, and LIPH4-NTNBSA and L1PH4-23SA, which was designed by replacing the cysteine residues in LIPH4-NTNBS and LIPH4-23S with alanine residues, in a concentration-dependent manner inhibition (Fig. 22E).

These results indicate that LIPG promotes HSPG-mediated HBV entry into liver cells and that, conversely, LIPG-binding peptides and Netrin-1 inhibit HBV entry into liver cells. The LIPG-mediated promotion of HBV entry into liver cells is diminished in the presence of Netrin-1, suggesting the possibility that the binding of LIPG to HSPGs (heparan sulfate proteoglycans) is competitively blocked by Netrin-1 present on the liver tissue surface of organisms.

### [Example 13] Drug efficacy testing in HBV-infected PXB mice

The following experiment was performed by infecting PXB mice with HBV and administering a test substance (LIPH4-NTNBs, SEQ ID NO: 25) to the mice to confirm the drug efficacy of the test substance.

### (13-1) Test substance, solvent, and preparation method

### Agent A: LIPH4-NTNBs 4 mg

Characteristics: powder
Quantity: 288 mg (24 mg × 12 vials)
Storage: -80°C
Preparation method: 24 mg of the agent was dissolved in 1800 µL of PBS and administered at a dose of 300 µL/body (4 mg/body)

### Agent B: LIPH4-NTNBs 2 mg

Characteristics: powder
Quantity: 144 mg (12 mg × 12 vials)
Storage: -80°C
Preparation method: 12 mg of the agent was dissolved in 1800 µl of PBS and administered at a dose of 300 µL/body (2 mg/body)

### Agent C: LIPH4-NTNBs 0.1 mg

Characteristics: powder
Quantity: 7.2 mg (0.6 mg × 12 vials)
Storage: -80°C
Preparation method: 0.6 mg of the agent was dissolved in 1800 µl of PBS and administered at a dose of 300 µL/body (0.1 mg/body)

### Solvent

Name: PBS
Source: Thermo Fisher Scientific (Life) 10010049 PBS pH 7.4
Storage: refrigeration

### (13-2) Virus used

The virus used was HBV provided by PhoenixBio Co., Ltd.
Name: Hepatitis B Virus
Strain: PBB004 (Genotype C)
BSL: 2
Viral titer: 1.1E+09 copies/mL (10 µL/tube × 2 tubes)
Storage: stored in a deep freezer
Preparation method: one tube of virus solution frozen for storage was thawed and adjusted to a titer of 1.0E+06 copies/mL with saline (Otsuka Pharmaceutical Factory, Inc.).

### (13-3) Animal used

Animal species: mouse
Strain: PXB mice (mice generated by introducing human liver cells into cDNA-uPAwild/+/SCID mice. the mice harboring human liver cells with an expected replacement rate of 70% or more. calculated based on the h-Alb level in the mouse blood)
Source: PhoenixBio Co., Ltd.
Sex: male
Age: 12 weeks old or older (at the arrival time)
9 weeks or more after human liver cell transplantation
Number of animals used: 30
Reason of selecting the animal species: this mouse strain is commonly used as an HBV infection model

### (13-4) Animal care conditions

### 13-4.1 Rearing conditions (SOP/environment/504)

Room temperature: 24 ± 3°C. humidity: 50 ± 20%, ventilation: (10 to 25 times/1 hour), illumination: 12 hours (8:00 to 20:00)

### 13-4.2 Feed (SOP/rearing/206. SOP/rearing/512)

A 2:1 feed mixture of MF (Oriental Yeast Co., Ltd.) and PS-A (Oriental Yeast Co., Ltd.) was given *ad libitum.*

### 13-4.3 Drinking water (SOP/rearing/206, SOP/rearing/512)

Drinking water prepared by adding sodium hypochlorite (final concentration: 14.4 ppm) to autoclaved tap water was given *ad libitum.*

### 13-4.4 Cage (SOP/animal/301)

Autoclaved cages were used. Mice were housed 1 to 5 per cage.

Exchange of cages, feeders (replenishment), and exchange of water bottles were performed in the same period to reduce the risk of microbial contamination.

### 13-4.5 Quarantine and acclimation (SOP/animal/301)

The general condition of each animal was observed daily from the time of arrival until the end of quarantine and acclimation. The body weight of each animal was measured on the day of arrival and on the last day of quarantine and acclimation.

### 13-4.6 Grouping (SOP/test/002)

Blood samples were collected from mice at 6 weeks after virus inoculation, and the mice were divided into groups based on the HBV DNA levels in the blood samples at 8 weeks after virus inoculation (on the day of the first dose of a test substance).

### 13-4.7 Animal identification (SOP/test/001)

Individual animals were identified using oil-based ink on the dorsal surface of the tail. A label was attached to each cage, indicating the study number, animal number, test period, test group, name of the study director, and the like.

### (13-5) Test group organization and dosing schedule

The test group organization and the dosing schedule were as shown in Table 8 below.

**[Table 8]**

| Group | Administered substance | Dosage (/body) | Dosing route | Dosing period*¹ | Sampling date*² | Animal ID number (n) |
|---|---|---|---|---|---|---|
| 1 | agent A | 300 µL | ip | Day 0 to 27 | Day 28 | 11-15 (5) |
| 2 | agent B | 300 µL | ip | Day 0 to 27 | Day 28 | 21-25 (5) |
| 3 | agent C | 300 µL | ip | Day 0 to 27 | Day 28 | 31-35 (5) |
| 6 | solvent | 300 µL | ip | Day 0 to 27 | Day 28 | 61-65 (5) |

| | | | | | | |
|---|---|---|---|---|---|---|
| *1, Day 0: the day of the first dose of a test substance (at 8 weeks after virus inoculation) *2. samples were collected every two to three days (on Monday, Wednesday, and Friday) during the dosing period. | | | | | | |

### Virus inoculation

Mice were inoculated with HBV in the tail vein at a dose of 1.0E+05 copies/100 µL/body.

### Test substance administration

Each substance was administered intraperitoneally to mice every two to three days (on Monday, Wednesday, and Friday) at a dose of 300 µL/body starting 8 weeks after virus inoculation (Day 0) until the day before sample collection (Day 27).

### Autopsy

The mice were euthanized on Day28 to harvest the liver, and the weight of the liver was measured. Part of the liver was frozen and stored for analysis of HBV DNA and cccDNA, and the rest of the liver was fixed in formalin for pathohistological examination. The lungs, spleen, and kidney were fixed in formalin.

### Measurement of HBV DNA in the liver

DNA was extracted from the liver sample immersed in RNAlater using DNeasy (registered trademark) Blood & Tissue Kits (QIAGEN K.K., Tokyo) and dissolved in nuclease-free water. The DNA concentration was measured with a BioPhotometer (registered trademark) 6131 (Eppendorf Co.. Ltd.) and adjusted with nuclease-free water to a final concentration of 20 ng/pL. Five µL of the thawed DNA stock solution or diluted DNA and the TaqMan (registered trademark) Fast Advanced Master Mix were used to prepare a PCR reaction solution. The CFX96 Touch^{™} Real-Time PCR Detection System was used for PCR reaction and analysis. The PCR reaction was performed at 50°C for 2 minutes → 95°C for 20 seconds → (95°C for 3 seconds -> 60°C for 32 seconds) × 53 cycles. The concentration of HBV DNA in the liver was calculated as the average of those in 2 wells. The primer and probe sequences used are listed in Table 9 below. The lower detection limit of this quantification method is 50 copies/100 ng DNA. Sera obtained from HBV-infected PXB mice were used as the HBV DNA standard. The concentration of HBV DNA in the sera was quantified by digital PCR.

**[Table 9]**

| Identification information | Putative target site | Sequence information | | | SEQ ID NO |
|---|---|---|---|---|---|
| | | label | 5' nucleic acid sequence 3' | label | |
| Forward primer | 166-186 | n/a | CACATCAGGATTCCTAGGACC | n/a | 67 |
| Reverse primer | 344-325 | n/a | AGGTTGGTGAGTGATTGGAG | n/a | 68 |
| TaqMan probe | 242-267 | 6-FAM | CAGAGTCTAGACTCGTGGTGGACT TC | TAMRA | 69 |

| | | | | | |
|---|---|---|---|---|---|
| n/a: Not applicable | | | | | |

### Measurement of cccDNA

Five µL of the DNA stock solution which had been purified for the measurement of HBV DNA in the liver or the or diluted DNA and the TaqMan (registered trademark) Fast Advanced Master Mix were used for the preparation. The CFX96 Touch^{™} Real-Time PCR Detection System was used for PCR reaction and analysis. The PCR reaction was performed at 50°C for 2 minutes → 95°C for 20 seconds → (95°C for 3 seconds → 60°C for 32 seconds) × 55 cycles. The concentration of cccDNA in the liver was calculated as the average of those in 2 wells. The sequences of primers (Takara Bio Inc., Shiga Prefecture) and probe (Takara Bio Inc.) used are listed in Table 10 below. The lower detection limit of this quantification method was 1.0 × 10² copies/100 ng DNA. A plasmid carrying the HBV whole genome sequence was used as the HBV cccDNA standard.

**Table 10**

| Identification information | Putative target site | Sequence information | | | SEQ ID NO |
|---|---|---|---|---|---|
| | | label | 5' nucleic acid sequence 3' | label | |
| Forward primer | 1545-1563 | n/a | CTCCCCGTCTGTGCCTTCT | n/a | 70 |
| Reverse primer | 1900-1883 | n/a | GCCCCAAAGCCACCCAAG | n/a | 71 |
| TaqMan probe | 1602-1628 | 6-FAM | CGTCGCATGGARACCACCGTGAA CGCC | TAMRA | 72 |

| | | | | | |
|---|---|---|---|---|---|
| n/a: Not applicable | | | | | |

### Pathohistological examination (HBsAg immunostaining, HBcAg immunostaining)

The liver tissues were fixed in 10% neutral buffered formalin and then in 70% ethanol for replacement, and the resulting samples were sent to the Nara Pathology Laboratory (Nara Prefecture) to prepare paraffin-embedded blocks by a conventional method, and thin sectioned specimens were later prepared from the blocks.

Paraffin sections were deparaffinized and then treated with microwave for antigen retrieval. A primary antibody (HBsAg (anti-HBsAg antibody, code: OBT0990, Bio-Rad AbD Serotec Limited, Oxford, UK) or HBcAg (anti-HBcAg antibody, code: PAB14506, Abnova, Taipei City, Taiwa)) was reacted with the sections overnight at 4°C, a biotin-avidin-peroxidase complex, and then DAB for color development. Cell nuclei were stained with hematoxylin, and the sections were dehydrated, cleared, and then mounted.

The sections were then examined with a light microscope at Hamri Co., Ltd.

### (13-6) Observation and measurement items

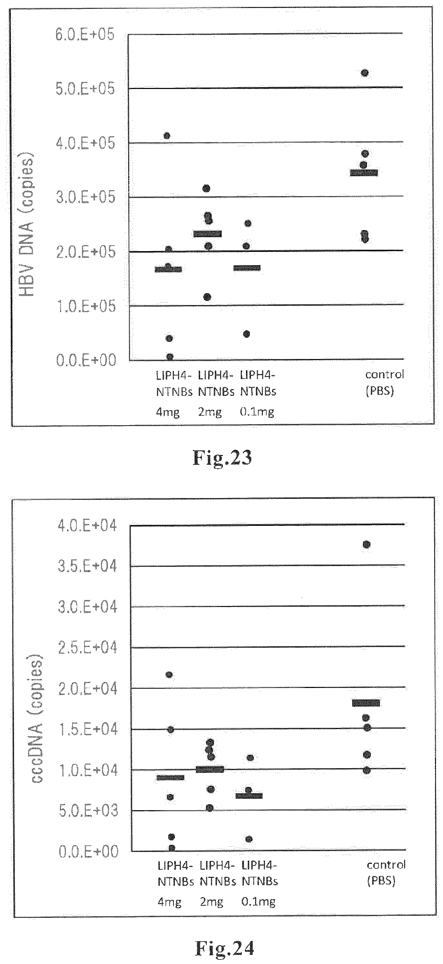

The HBV-DNA level was measured in the livers obtained during the autopsy (Fig. 23), to find that the LIPH4-NTNBs 4 mg, LIPH4-NTNBs 2 mg, and LIPH4-NTNBs 0.1 mg all reduced the copy number of HBV DNA compared to the control administration group. Similarly, the copy number of cccDNA in the livers was measured (Fig. 24), to find that the LIPH4-NTNBs 4 mg, LIPH4-NTNBs 2 mg, and LIPH4-NTNBs 0.1 mg all significantly reduced the copy number of HBV DNA compared to the control administration group.

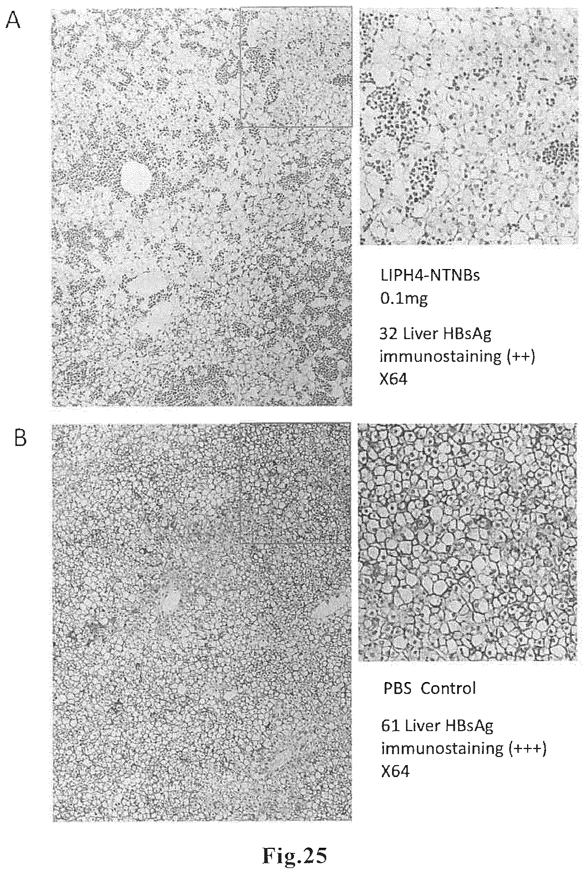

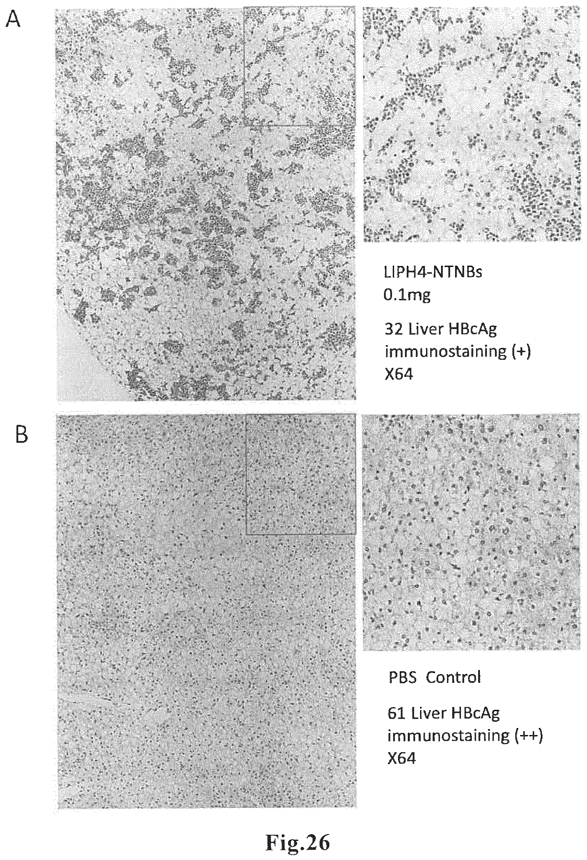

Micrographs of HBsAg immunostaining as a pathohistological examination are shown in Fig. 25. It was apparent that the area of staining of the antigen was smaller in the LIPH4-NTNBs 0.1 mg administration group than in the control (PBS) administration group. Micrographs of HBcAg immunostaining as a pathohistological examination are shown in Fig. 26. It was also apparent that the area of staining of the antigen was smaller in the LIPH4-NTNBs 0.1 mg administration group than in the control (PBS) administration group.

These results have demonstrated that the LIPG-binding peptide (LIPH4-NTNBs) reduces HBV-DNA and cccDNA in the liver in the animal experiment using human hepatocyte chimeric mice infected with HBV and has an apparent anti-HBV effect. In addition, the LIPG-binding peptide (LIPH4-NTNBs) was administered after the cells were fully infected with HBV in *in vitro* and *in vivo* assay systems, and therefore it is highly likely that the LIPG-binding peptide (LIPH4-NTNBs) inhibits the reinfection process, a process in which HBV particles once secreted extracellularly reenter into cells.

### [Example 14] : Preparation of more LIPG-binding peptides

LIPH4-23 (SEQ ID NO: 1), which had remarkable anti-HBV activity in HepG2-NTCP-C4 cells in Example 11, was also found by a further data analysis to share a homology also with N4BP1 (NEDD4 Binding Protein 1, SEQ ID NO: 82) in the common sequence shared with netrins. Then, three different N4BP1 partial peptides, LIPH4-N4BP1a, LIPH4-N4BP1b. and LIPH4-N4BP1c, containing the common sequence shared by N4BP1, were chemically synthesized. Additionally, based on the LIPH4-NTNBs peptide having a partial sequence of Netrin-1, which was revealed to have very high anti-HBV activity in Example 12 and was confirmed to also exhibit an *in vivo* anti-HBV effect in Example 13, a netrin partial peptide LIPH4-LNTNBs was chemically synthesized, which had the same sequence as the LIPH4-NTNBs except that the N-terminus was extended by one residue.

Seven LIPG-binding peptides, including those described above and LIPH4-23s, were used in the LIPG-binding peptide intracellular delivery experiment below.

**[Table 11]**

| Table 11. Amino acid sequences of chemically synthesized LIPG-binding peptides | | | |
|---|---|---|---|
| Peptide name | Number of amino acid residues | Amino acid sequence | SEQ ID NO |
| LIPH4-23 | 16 | LNCRDNTRPVMSAMTC | SEQ ID NO: 1 |
| LIPH4-23s | 8 | LNCRDNTR | SEQ ID NO: 23 |
| LIPH4-NTNBs | 8 | NCRHNTAG | SEQ ID NO: 25 |
| LIPH4-LNTNBs | 9 | LNCRHNTAG | SEQ ID NO: 77 |
| LIPH4-N4BP1a | 16 | SNCRINTFRTVPIEQK | SEQ ID NO: 78 |
| LIPH4-N4BP1b | 9 | SNCRINTFR | SEQ ID NO: 79 |
| LIPH4-N4BP1c | 10 | TSNCRINTFR | SEQ ID NO: 80 |

### [Example 15] : Preparation of ASGR-binding single-chain antibodies

Most proteins in the blood are sialic acid-attached glycoproteins. and the sialic acid-attached glycoproteins are deteriorated to asialoglycoproteins, and the resulting asialoglycoproteins are primarily degraded in the liver. Therefore, the liver contains the asialoglycoprotein receptor (ASGR) that specifically recognizes and takes up asialoglycoproteins in the blood. Similarly, anti-ASGR antibodies are believed to be taken up by liver cells after binding to the receptors. For the experimental selective delivery of a LIPG-binding peptide into liver cells, anti-ASGR single-chain antibodies were prepared as carriers for delivery into liver cells using the IVV method.

### (15-1) Preparation of biotinylated ASGR

First, biotinylated asialoglycoprotein receptors were prepared as antigens. As shown in Fig. 27, the asialoglycoprotein receptor (ASGR) is a type II. single-pass transmembrane protein with the N-terminus inside and the carbohydrate recognition domains (CRDs) outside the cell membrane. In order to made the receptor be presented on a Biacore sensor chip, constructs were prepared by removing the transmembrane domain and adding thereto a biotinylation sequence and the like. In human liver cells. ASGR1 (SEQ ID NO: 114) and ASGR2 (SEQ ID NO: 116) are present and form a heterooligomer. Human liver cells contain ASGR1 (SEQ ID NO: 114) and ASGR2 (SEQ ID NO: 116), which form a heterooligomer. Therefore, biotinylated ASGR1ex and biotinylated ASGR2ex, biotinylated extracellular domains of both ASGR proteins, were prepared.

### (15-1-i) Construction of expression vectors for biotinylated ASGR

The pcDNA3.3 TOPO HisBioFLAG-ASGRlex vector and the pcDNA3.3 TOPO HisBioFEAG-ASGR2ex vector were prepared by adding a His-tag sequence, a biotinylation sequence, and a Flag-tag sequence to the sequence of each ASGR extracellular domain.

As shown in Fig. 28, the extracellular domains of both ASGR1 and ASGR2 were cloned.

RNase-free water was added to 1 µl of cDNA Library. Human Liver (1 ng/µl) (Takara Bio), 10 µl of KAPA HiFi HS RM, 0.6 µl of ASGR1-ex-if-F1 or 0.6 µl of ASGR2-ex-if-F1 at a concentration of 10 µM, and 0.6 µl of ASGR1-if-R2 or 0.6 µl of ASGR2-if-R2 at a concentration of 10 µM to give a total volume of 20 µl, and the resulting mixture was subjected to a PCR reaction. The PCR reaction was performed at 95°C for 5 minutes, followed by 25 cycles of 98°C for 20 seconds. 60°C for 15 seconds and 72°C for 1 minute, and then a reaction at 72°C for 1 minute. After a DNA band was confirmed by agarose gel electrophoresis, the PCR product was purified with the Wizard SV Gel PCR Clean-Up System (Promega) and collected as 30 µl of a DNA solution to obtain ASGR1-if or ASGR2-if.

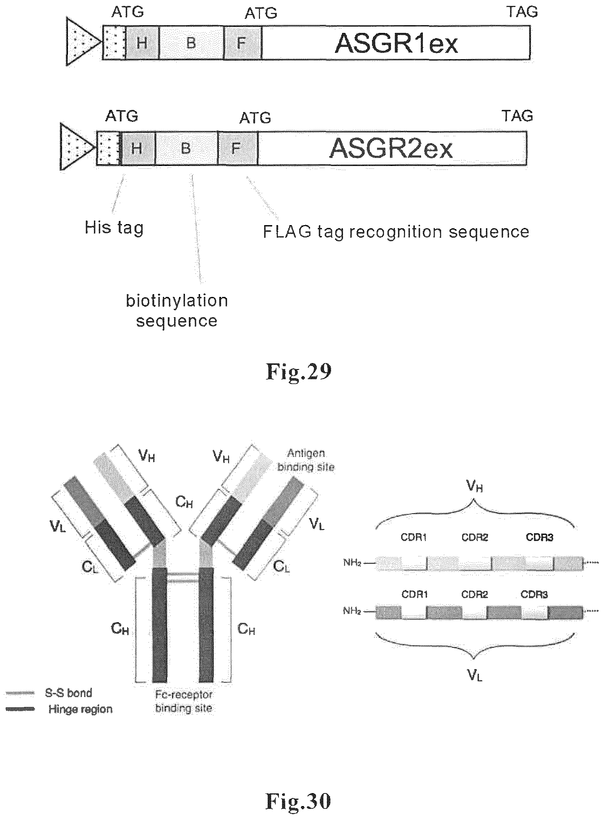

To prepare expression plasmids carrying the constructs as shown in Fig. 29_ the ASGR1-if or ASGR2-if was cloned by in-fusion cloning using the pcDNA3.3 TOPO KHisBioFlag vector containing a kozak sequence-His-tag sequence-biotinylation sequence-Flag-tag sequence. RNase-free water was added to 1.0 µl of pcDNA3.3 TOPO KHisBioFlagINV, 1.0 µl of ASGR1-if or 1.0 µl of ASGR2-if, and 1.0 µl of 5x In-Fusion HD Enzyme Premix (Takara) to give a total volume of 5 µl, and the reaction was allowed to proceed at 50°C for 15 minutes. One Shot TOP10 competent cells were transformed with 2.5 µl of the reaction mixture and cultured overnight at 37°C to obtain clones. The identity of the clones was confirmed by sequence analysis to be the pcDNA3.3 TOPO KHisBioFlag-ASGR1ex or pcDNA3.3 TOPO KHisBioFlag-ASGR2ex plasmid.

**Table 12. Primer sequences (4)**

| Name | Sequence (5'→3') | SEQ ID NO: |
|---|---|---|
| ASGR1-ex-if-F1 | GACGACGACGACAAGCAAAACTCCCAGCTGCAGGA | 117 |
| ASGR2-ex-if-F1 | GACGACGACGACAAGCAAAGTGAGGGTCACAGGGG | 118 |
| ASGR1-if-R2 | CTAACCGGTAGGGATTTAAAGGAGAGGTGGCTCCT | 119 |
| ASGR2-if-R2 | CTAACCGGTAGGGATTCAGGCCACCTCGCCGGTGG | 120 |

### (15-1-ii) Expression and purification of the biotinylated extracellular domain ASGR1ex or ASGR2ex

Human embryonic kidney 293T cells were transfected with the pcDNA3.3 TOPO KHisBioFlag-ASGRlex plasmid or the pcDNA3.3 TOPO KHisBioFlag-ASGR2ex plasmid. After incubation at 37°C in 5% CO₂ for 48 hours. the resulting cells were subjected to extraction with Cell Lysis Buffer (25 mM Tris-HCl pH 7.4, 137 mM NaCl, 2.68 mM KCI, 1% Triton X-100). M2 agarose beads (ANTI-FLAG (registered tradename) M2 Affinity Gel, Sigma, A2220) washed with TBS were added to the resulting cell extract and mixed at 4°C for 16 hours. The beads were washed three times with TBST (10x TBS-t, 1% Tween-20, Nacalai, 12749-21) and eluted by competitive elution with 150 µg/mL 3x FLAG peptide (Sigma, F4799) diluted in TBS.

Each FLAG-purified protein was added to 20 µL of SA beads (Streptavidin MagneSphere (registered tradename) Paramagnetic Particles, Promega) washed three times with 200 µL of TBST (10× TBS-T 1%Tween-20. Nacalai). The resulting mixture was mixed by rotation at room temperature for 1 hour and the beads were then washed three times with 200 µL of TBST. The beads were suspended in 10 µL of TBS and heated at 70°C for 10 minutes. Detection by SDS-PAGE and Western blotting with a Flag antibody confirmed KHisBioFlag-ASGR1ex as a band with a molecular weight of 35.597 Da and KHisBioFlag-ASGR2ex as a band with a molecular weight of 35,708 Da.

A cell extract containing HisBioFLAG-ASGR1ex. obtained by the same method, was allowed to be bound on SA beads. After washing the resulting beads, the buffer was changed to EKMax buffer (500 mM Tris-HCl. pH 8.0, 10 mM CaCl₂, 1% Tween-20). The EKMax^{™} enterokinase (Thermo Fisher Scientific) was added thereto, and mixed by rotation at 37°C O/N (16.5 h) for EKMax digestion, and the SA beads were then adsorbed on a magnetic stand to collect the supernatant. A pretreated EK Away resin was added to the collected supernatant and mixed for 15 minutes at r.t. to remove the EKMax. After centrifugation, the supernatant was collected (5000 rcf for 2min × 2). Detection by SDS-PAGE and Western blotting using a Flag antibody confirmed the ASGR1ex as a band with a molecular weight of 26394 Da.

### (15-2) Preparation of cDNA library of single-chain antibodies

### (15-2-i) Design of DNA library

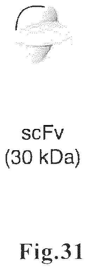

The N-terminal domains of H and L chains are responsible for antigen recognition by antibodies and are called variable regions. In contrast, the remaining domains are constant within each class and are called constant regions (Fig. 30, left). Both VH and VL chains are composed of three CDRs (complementarity determining regions) and four FRs (framework regions) (Fig. 30, right). The amino acid sequences of the CDRs are variable for each antibody, allowing the CDRs to bind to different antigens. One example of artificial antibody fragments is a single-chain antibody (scFv) composed of VH and VL chains linked by a peptide linker (Fig. 31). To ensure that a peptide linker consisting of 15 amino acids between VH and VL chains does not interfere with the association between the domains, a linker in which the GGGGS unit (SEQ ID NO: 112), which comprises glycine and a hydrophilic serine, is repeated three times is often used as a peptide linker that does not constrain the secondary structure.

### (15-3) Preparation of cDNA library of mouse-derived single-chain antibodies

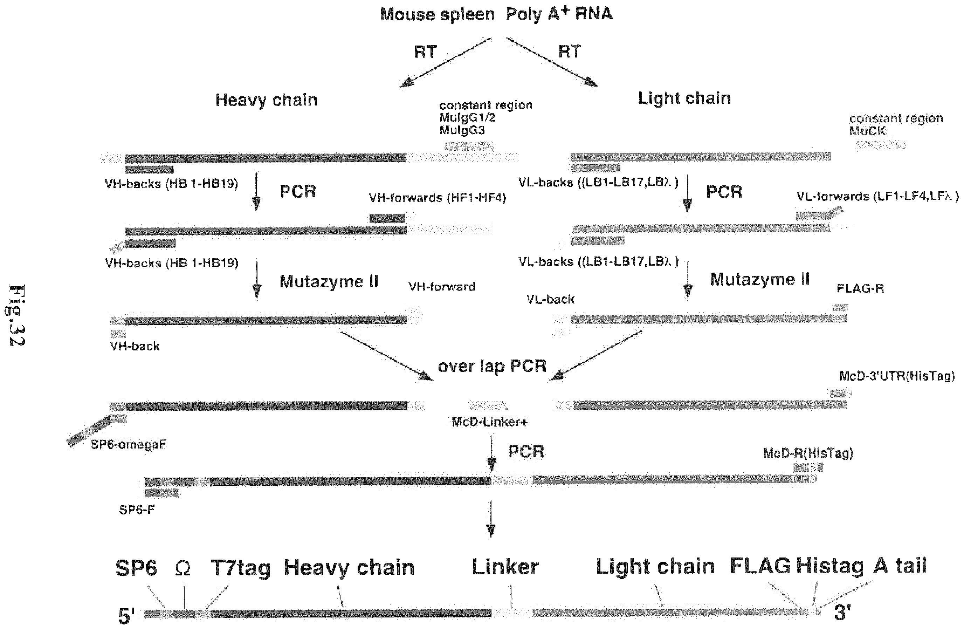

A cDNA library of mouse-derived single-chain antibodies was prepared, as shown in Fig. 32, using mouse spleen poly(A)⁺ RNA as the starting material. An H-chain DNA solution and an L-chain DNA solution were prepared, and the H and L chains were combined into single chains by PCR (Tabata, N., et al., Nucleic Acids Res., 37, e64, 2009).

### (15-3-i) Preparation of H-chain DNA solution

An H-chain DNA solution was first prepared for preparation of a cDNA library of single-chain antibodies. Mouse spleen poly(A)⁺ RNA (5 µg/µl) (in DEPC-treated water) (CLONTECH) was 100-fold diluted with RNase-free water, and 11 µl of the resulting dilution was mixed with 22 µl of 5 × RT buffer (TOYOBO), 11 µl of (10 mM) dNTPs (TOYOBO), 27.5 µl of the MulgG 1/2 forward primer (1 pmol/µl), and 27.5 µl of the MulgG3 forward primer (1 pmol/µl), and the resulting mixture was reacted at 65°C for 9 minutes and then immediately cooled to 4°C and held at 4°C for 2 minutes. To the resulting mixture, 5.5 µl of ReverTra Ace (TOYOBO) and 5.5 µl of an RNase inhibitor (TOYOBO) were added, and the reaction was allowed to proceed at 50°C for 30 minutes, followed by treatment at 99°C for 5 minutes, to synthesize cDNA-H. RNase-free water was added to 5 µl of the eDNA-H solution, 2.5 µl of one of the primers indicated as HB primers (1 pmol/µl), 2.5 µl of 10 × PCR buffer (TOYOBO), 1.25 µl of the MulgG1/2 forward primer (1 pmol/µl), 1.25 µl of the MulgG3 forward primer (1 pmol/µl), and 0.25 µl of KOD DASH polymerase (TOYOBO) to give a total volume of 25 µl, and the resulting mixtures were subjected to a PCR reaction. The PCR reaction was performed at 96°C for 5 minutes, followed by 25 cycles of 96°C for 30 seconds, 50°C for 30 seconds and 72°C for 1 minute, and then a reaction at 72°C for 5 minutes. The amplified genes were confirmed as bands in the range of 500-900 bp by 2% agarose gel electrophoresis and treated by phenol/chloroform. That is, an equal volume of a phenol:chloroform:isoamyl alcohol mixture (25:24:1) was added to each amplified gene and mixed thoroughly, and the resulting mixtures were centrifuged at 13,200 rpm for 5 minutes at 4°C, and each of the resulting aqueous layers was transferred to a new tube. Again, an equal volume of a phenol:chloroform:isoamyl alcohol mixture (25:24:1) was added to the aqueous layers and mixed thoroughly, and the resulting mixtures were centrifuged at 13,200 rpm for 5 minutes at 4°C, and each of the resulting aqueous layers was transferred to a new tube. Ethanol precipitation was performed on the resulting solutions. After drying under centrifugation for approximately 15 minutes, each DNA fragment (19 species in total) was dissolved in 20 µl of RNase-free water. RNase-free water was added to 1 µl of one of the solutions of the synthesized DNA fragments (19 species), 2 µl of corresponding one of the primers indicated as HB primers (10 pmol/µl), 10 µl of 10 × PCR buffer (TOYOBO), 10 µl of (2 mM) dNTPs (TOYOBO), and 2 µl of a VH forward primer mixture of HF1:HF2:HF3:HF4 (1:1:1:1) indicated as HF primers (10 pmol/µl), and 0.5 µl of KOD DASH polymerase (TOYOBO) to give a total volume of 100 µl, and the resulting mixtures were subjected to a PCR reaction. The PCR reaction was performed at 96°C for 5 minutes, followed by 20 cycles of 96°C for 30 seconds, 50°C for 30 seconds and 72°C for 1 minute, and then a reaction at 72°C for 5 minutes. The amplified genes were confirmed as bands in the range of 500-900 bp by 2% agarose gel electrophoresis, followed by phenol/chloroform treatment and ethanol precipitation. After drying under centrifugation for approximately 15 minutes, each of the DNA fragments (19 species) was dissolved in 10 µl of RNase-free water. The resulting 19 DNA fragments were electrophoresed on a 2% low melting point agarose gel (Sigma), and the corresponding bands were excised from the gel. Each of the DNA fragments (19 species) was dissolved in 10 µl of RNase-free water. The absorbance was measured at 260 nm for each DNA solution, and the DNA solutions were mixed at a ratio of HB1:HB2:HB3:HB4:HB5:HB6:HB7:HB8:HB9:HB10:HB11:HB12:HB13:HB14:HB 15:HB16:HB17:HB18:HB19 (8:9:4:4:12:4:1:4:12:4:4:2:2:4:4:8:6:1:1) to prepare a solution containing a total of 0.5 pmol of H-chain DNAs.

### (15-3-ii) Preparation of L-chain DNA solution

Mouse spleen poly(A)⁺ RNA (5 µg/µl) (in DEPC-treated water) (CLONTECH) was 100-fold diluted with RNase-free water, and 10 µl of the resulting dilution was mixed with 20 µl of 5 × RT buffer (TOYOBO), 10 µl of (10 mM) dNTPs (TOYOBO), and 50 µl of the MuCK forward primer (1 pmol/µl), and the resulting mixture was reacted at 65°C for 9 minutes and then immediately cooled to 4°C and held at 4°C for 2 minutes. To the resulting mixture, 5 µl of ReverTra Ace (TOYOBO) and 5 µl of an RNase inhibitor (TOYOBO) were added, and the reaction was allowed to proceed at 50°C for 30 minutes, followed by treatment at 99°C for 5 minutes, to synthesize cDNA-L. RNase-free water was added to 5 µl of the cDNA-L solution, 2.5 µl of one of the primers indicated as LB primers (1 pmol/µl). 2.5 µl of 10 × PCR buffer (TOYOBO), 2.5 µl of the MuCK forward primer (1 pmol/µl), and 0.25 µl of KOD DASH polymerase (TOYOBO) to give a total volume of 25 µl, and the resulting mixtures were subjected to a PCR reaction. The PCR reaction was performed at 96°C for 5 minutes, followed by 25 cycles of 96°C for 30 seconds, 48°C for 30 seconds and 72°C for 1 minute, and then a reaction at 72°C for 5 minutes. The amplified genes were confirmed as bands in the range of 500-900 bp by 2% agarose gel electrophoresis and treated by phenol/chloroform. Ethanol precipitation was performed on the resulting solutions. After drying under centrifugation for approximately 15 minutes, each DNA fragment (18 species in total) was dissolved in 20 µl of RNase-free water. RNase-free water was added to 1 µl of one of the solutions of the synthesized DNA fragments (18 species). 2 µl of corresponding one of the primers indicated as LB primers (10 pmol/µl). 10 µl of 10 × PCR buffer (TOYOBO), 10 µl of (2 mM) dNTPs (TOYOBO), 2 µl of a LH forward primer mixture of LF1:LF2:LF3:LF4:LFA, (1:1:1:1) indicated as LF primers (10 pmol/µl), and 0.5 µl of KOD DASH polymerase (TOYOBO) to give a total volume of 100 µl, and the resulting mixtures were subjected to a PCR reaction. The PCR reaction was performed at 96°C for 5 minutes, followed by 20 cycles of 96°C for 30 seconds, 48°C for 30 seconds and 72°C for 1 minute, and then a reaction at 72°C for 5 minutes. The amplified genes were confirmed as bands in the range of 500-900 bp by 2% agarose gel electrophoresis, followed by phenol/chloroform treatment and ethanol precipitation. After drying under centrifugation for approximately 15 minutes, each of the DNA fragments (18 species) was dissolved in 10 µl of RNase-free water. The resulting 18 DNA fragments were electrophoresed on a 2% low melting point agarose gel (Sigma), and the corresponding bands were excised from the gel. Each of the DNA fragments (18 species) was dissolved in 10 µl of RNase-free water. The absorbance was measured at 260 nm for each DNA solution, and the DNA solutions were mixed at a ratio of LB1:LB2:LB3:LB4:LB5:LB6:LB7:LB8:LB9:LB1O:LB11:LB12:LB13:LB14:LB15: LB16:LB17:LBλ (2:4:8:8:8:16:12:4:4:8:16:16:12:4:4:2:2:1) to prepare a solution containing a total of 0.5 pmol of L-chain DNAs.

### (15-3-iii) PCR to combine H and L chains into single chains

RNase-free water was added to 0.5 pmol of the synthesized H-chain DNA in solution, 0.5 pmol of the synthesized L-chain DNA in solution, 0.5 µl of 5'UTR (1 pmol/µl). 0.5 µl of McD-Linker+ (1 pmol/µl), 0.5 µl of McD 3'UTR (His Tag) (1 pmol/µl). 5 µl of 10 × PCR buffer (TOYOBO), 5 µl of (2 mM) dNTPs (TOYOBO). and 0.25 µl of KOD DASH polymerase (TOYOBO) to give a total volume of 45.75 µl, and the resulting mixture was subjected to a PCR reaction. The PCR reaction was performed at 96°C for 5 minutes, and then at 96°C for 30 seconds, followed by a temperature reduction to 58°C over 5 minutes, and thereafter 10 cycles of 58°C for 30 seconds and 72°C for 1 minute, and finally a reaction at 72°C for 5 minutes. Then. 45.75 µl of the PCR reaction solution, 2 µl of McD-F (1 pmol/µl), 2 µl of McD-R (His Tag) (1 pmol/µl), and 0.25 µl of KOD DASH polymerase (TOYOBO) were mixed together, and the reaction mixture was subjected to another PCR reaction. The PCR reaction was performed at 96°C for 5 minutes, followed by 15 cycles of 96°C for 30 seconds, 58°C for 30 seconds and 72°C for 1 minute, and then a reaction at 72°C for 5 minutes. The resulting DNA fragments were electrophoresed on a 1% low melting point agarose gel (Sigma), and the corresponding bands were excised from the gel. The DNA fragments were dissolved in 10 µl of RNase-free water to obtain a mouse-derived single-chain antibody library DNA.

### (15-4) Preparation of cDNA library of human-derived single-chain antibodies

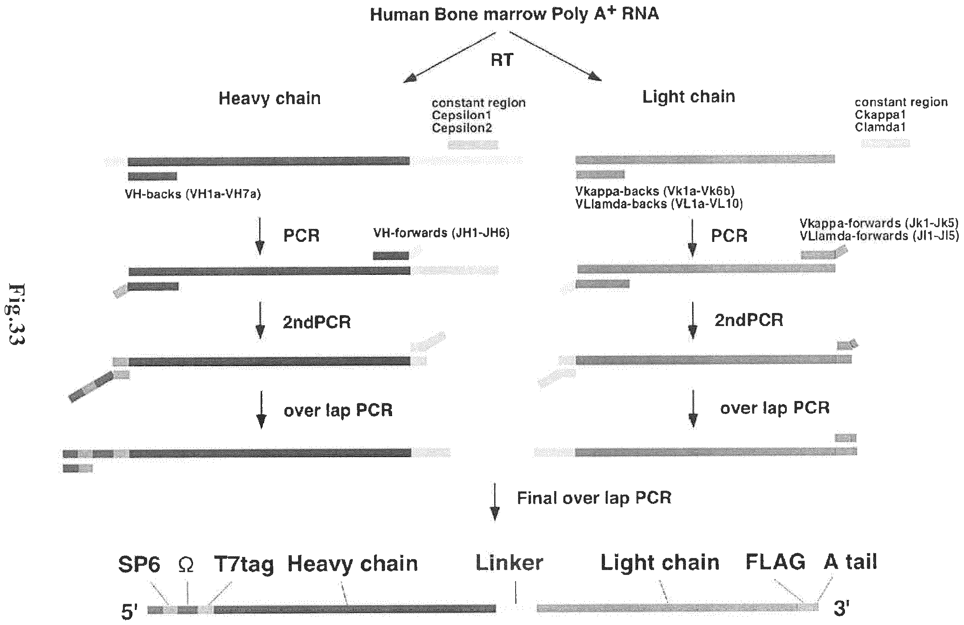

A cDNA library of single-chain antibodies from human bone marrow-derived lymphocytes was prepared, as shown in Fig. 33, using human bone marrow-derived poly(A)⁺ RNA as the starting material. Similar to the mouse cDNA library, an H-chain DNA solution and an L-chain DNA solution were prepared using multiple specific primers, and the H and L chains were combined into single chains to prepare a cDNA library of human single-chain antibodies for IVV selection (J. Mol. Biol., 222, 581-597, 1991; Nat. Biotechnol., 23, 344-348, 2005; Antibody Engineering, Springer Lab. Manual, 93-108, 2001).

In addition, random mutations were introduced into the prepared mouse H-chain DNA and mouse L-chain DNA or the prepared human H-chain DNA and human L-chain DNA by error-prone PCR using Mutazyme H. The mouse mutant H-chain DNA and mouse mutant L-chain DNA were used for PCR to combine the H and L chains into single chains, thereby completing the preparation of a cDNA library of mouse mutant single-chain antibodies. The human mutant H-chain DNA and human mutant L-chain DNA were used for PCR to combine the H and L chains into single chains, thereby completing the preparation of a cDNA library of human mutant single-chain antibodies.

### (15-5) Selection of ASGR-binding single-chain antibodies

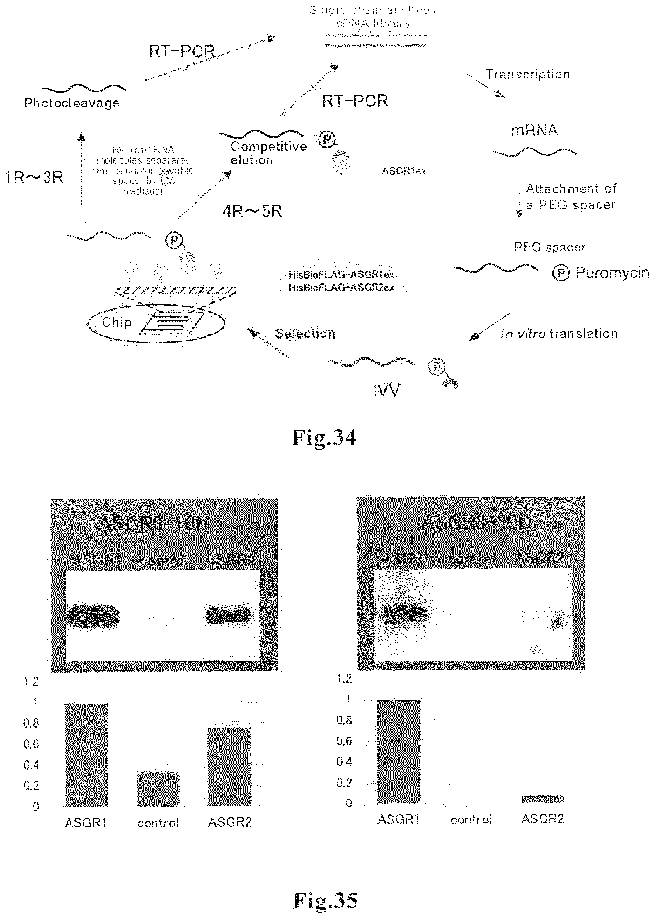

An experiment to select ASGR-binding single-chain antibodies was performed according to the procedure shown in Fig. 34.

### (15-5-i) Immobilization of ASGR

For Biacore, a Biacore 3000 system was used, and the ASGR was immobilized on a sensor chip SA. Flow was performed using HBS-P buffer (10 mM HEPES-NaOH, pH 7.4, 150 mM NaCl, 0.005% Tween-20) at a flow rate of 10 µl/min. A solution containing 50 mM NaOH and 1 M NaCl in a volume of 10 µl was injected three times into flow cells 1 to 4 as a pretreatment prior to immobilization. First, the biotinylated extracellular domain ASGR1ex was used and immobilized as a bait on flow cells 1 to 4. Flow was performed using HBS-P buffer at a flow rate of 20 µl/min. The amount of the immobilized bait is listed in Table 13. Then, the biotinylated extracellular domain ASGR2ex was used and immobilized on flow cells 3 and 4. Flow was performed using HBS-P buffer at a flow rate of 20 µl/min. The amount of the immobilized bait is listed in Table 7. The sensor chip was washed with HBS-P buffer running at a flow rate of 10 pllmin, and a solution containing 50% isopropanol. 50 mM NaOH. and 1 M NaCl was used for extra washing.

### (15-5-ii) Selection of ASGR-binding single-chain antibodies

To the prepared IVV library of single-chain antibodies in a volume of 100 µl, 4 µl of 0.5 M EDTA was added to give a final concentration of 20 mM, and the resulting mixture was agitated by rotation for 20 minutes at room temperature. A column (Bio-Rad) was packed with 1 ml of Sephadex G200 gel (Amersham Biosciences) that had been swollen and equilibrated with HBS-P, and the IVV library solution in a volume of 100 µl was applied to the column. Two drops from the column were added to each well of a 96-well plate, and the 1st to the 10th wells were recovered. Fluorescence from each well was detected using the Multi-detection Microplate Reader POWERSCAN HT at an excitation wavelength of 485 nm and a fluorescence wavelength of 528 nm, and the IVV fractions eluted in the 3rd to 7th wells were pooled. The resulting IVV fraction in a volume of approximately 200 µl was added to 100 µl of Strep Magne Sphae Paramagnetic Part (9013-20-1), which had been washed three times with 500 µl of HBS-P. and agitated by rotation for 20 minutes at room temperature, and the supernatant was injected into the Biacore. The selection conditions used for Biacore are listed in Table 13. As shown in Fig. 34, in the 1st to the 3rd rounds of selection experiments, the sensor chip was removed from the instrument after completion of the wash step, and 55 µl of RNase-free water was gently placed on the gold film of the sensor chip, and the spacer was cleaved by UV irradiation at 365 nm for 20 minutes to elute and collect mRNA moieties. In the 4th to 5th rounds of selection experiments, after completion of the wash step, competitive elution was performed using ASGR1ex to collect IVV fractions.

### (15-5-iii) Recovery of cDNA library by RT-PCR

RNase-free water was added to 55 µl of the recovered solution from the selection experiments. 20 µl of 5 × RT buffer (TOYOBO), 10 µl of (10 mM) dNTPs (TOYOBO), and 5 µl of a reverse primer: Flag His tag A02 (10 pmol/µl) to give a total volume of 90 µl, and the resulting mixture was reacted at 65°C for 9 minutes and then immediately cooled on ice for 2 minutes. To the resulting mixture, 5 µl of ReverTra Ace (TOYOBO) and 5 µl of an RNase inhibitor (TOYOBO) were added, and the reverse transcription reaction was allowed to proceed at 50°C for 30 minutes and at 99°C for 5 minutes. RNase-free water was added to 100 µl of the reaction solution after the reverse transcription reaction, 100 µl of 10 × KOD plus buffer (TOYOBO), 100 µl of 2 mM dNTPs (TOYOBO). 40 µl of 25 mM MgSO₄, 30 µl of a forward primer: SP6 Omega (10 pmol/µl). 30 µl of a reverse primer: Flag His tag A02 (10 pmol/µl), and 20 µl of KOD plus polymerase (TOYOBO) to give a total volume of 1000 µl, and the resulting mixture was dispensed in 100 µl aliquots into 10 tubes and subjected to a PCR reaction. The PCR reaction was performed at 94°C for 5 minutes, followed by 20 to 40 cycles of 94°C for 30 seconds, 58°C for 30 seconds and 68°C for 2 minutes, and then a reaction at 68°C for 5 minutes.

The selection pressure in the selection experiment using Biacore was progressively increased round by round, as shown in Table 13.

**Table 13: Selection pressure in selection experiments using Biacore**

| | 1st round | 2nd round | 3rd round |
|---|---|---|---|
| Amount of immobilized bait His-Bio-Flang-ASGR1ex | 654.7 RU | 403.5 RU | 353.4 RU |
| Amount of immobilized bait His-Bio-Flang-ASGR2ex | 191.4 RU | 147.8 RU | 96 RU |
| ASGR1ex : ASGR2ex | 3.4 : 1.0 | 2.7 : 1.0 | 3.7 : 1.0 |
| Time for association with IVVs | 240 sec | 240 sec | 120 sec |
| Wash flow rate in Biacore | 40 µl/min | 40 µl/min | 80 µl/min |
| Wash time in Biacore | 11367 sec | 59064 sec | 68342 sec |

| | 4th round | 5th round | |
|---|---|---|---|
| Amount of immobilized bait His-Bio-Flang-ASGR1ex | 538.9 RU | 504.5 RU | |
| Amount of immobilized bait His-Bio-Flang-ASGR2ex | 171.7 RU | 75.1 RU | |
| ASGR1ex : ASGR2ex | 3.1 : 1.0 | 6.7 : 1.0 | |
| Time for association with IVVs | 96 sec | 96 sec | |
| Wash flow rate in Biacore | 100 µl/min | 100 µl/min | |
| Wash time in Biacore | 64800 sec | 68400 sec | |

### (15-6) Cloning and determination of base sequences

### (15-6-i) Cloning and base sequences

From the library of ASGR-binding single-chain antibodies, inserts for a library were prepared for in-fusion cloning. RNase-free water was added to 1 µl of the library obtained after 3 rounds of selection experiments, 100 µl of 10 × KOD plus buffer (TOYOBO). 100 µl of 2 mM dNTPs (TOYOBO), 40 µl of 25 mM MgSO₄, 30 µl of T7-long-F in (10 pmol/µl). 30 µl of Flag(Histag) in R (10 pmol/µl), and 20 µl of KOD plus polymerase (TOYOBO) to give a total volume of 1000 µl, and the resulting mixture was subjected to a PCR reaction. The PCR reaction was performed at 94°C for 5 minutes, followed by 8 cycles of 94°C for 30 seconds, 58°C for 30 seconds and 68°C for 2 minutes, and then a reaction at 68°C for 5 minutes. The resulting cDNA library was purified with the Wizard SV Gel PCR Clean-Up System (Promega) and collected as 50 µl of a DNA solution to obtain T7-ASGR3.

RNase-free water was added to the pcDNA 3.3 vector comprising a kozak-Igk-T7tag sequence, a His sequence and a stop codon in a volume of 8 µl, 80 µl of 10 × KOD plus buffer (TOYOBO), 80 µl of 2 mM dNTPs (TOYOBO), 32 µl of 25 mM MgSO₄, 24 µl of His-stop-topo F (10 pmol/µl), 24 µl of Igk-T7-R (10 pmol/µl), and 16 µl of KOD plus polymerase (TOYOBO) to give a total volume of 800 µl, and the resulting mixture was subjected to a PCR reaction. The PCR reaction was performed at 94°C for 2 minutes, followed by 25 cycles of 98°C for 10 seconds and 68°C for 5 minutes and 11 seconds. The resulting cDNA library was purified with the Wizard SV Gel PCR Clean-Up System (Promega) and collected as 40 µl of a DNA solution to obtain the KIgk-stop 3.3 vector.

RNase-free water was added to a mixture of 0.08 µl of T7-ASGR3. 0.14 µl of the KIgk-stop 3.3 vector, and 1.0 µl of 5x In-Fusion HD Enzyme Premix (Takara) to give a total volume of 5 µl, and the reaction was allowed to proceed at 50°C for 15 minutes. One Shot TOP 10 competent cells were transformed with 2.5 µl of the solution after the reaction and cultured overnight at 37°C to obtain KIgk-ASGR3 clones. The resulting clones were sequenced using the ValueRead Premix provided by the Eurofins DNA Sequencing Service.

**[Table 14]**

| Table 14: Primer sequences (5) | | |
|---|---|---|
| Name | Sequence (5'→3') | SEQ ID NO |
| Flag His tag A02 | TTTTTTTTTATGGTGATGGTGGTGATGGTGCTTGTCGT CGTCGTCC | 121 |
| SP6 Omega | ATTTAGGTGACACTATAGAACAACAACAACAACAAA CAACAACAAAATGGCTAGCATGACTGGTGGACAGCA AATGGGT | 122 |
| T7-long-F | ATGGCTAGCATGACTGGTGGACAGCAAATGGGT | 123 |
| Flag(Histag) in R | ATGGTGATGGTGGTGATGGTGCTTGTCGTCGTCGTCC | 124 |
| His-stop-topo F | CACCACCATCACCATTAGAAGGGTTCGATC | 125 |
| Igk-T7-R | AGTCATGCTAGCCATGTCACCAGTGGAACC | 126 |

### (15-6-ii) Library sequencing

Sequencing of the library revealed that ASGR-binding single-chain antibody clones ASGR3-10M, ASGR3-39D, ASGR4-70D, and ASGR5-24M were obtained from the 3rd, the 4th, and the 5th rounds of selection experiments, as shown in Table 15.

**[Table 151**

| Table 15. Amino acid sequences of CDRs of ASGR-binding single-chain antibodies obtained by IVV screening | | | |
|---|---|---|---|
| Clone name | CDR | Amino acid sequence | SEQ ID NO |
| ASGR3-10M | HCDR1 | SSYWIE | 83 |
| ASGR3-10M | HCDR2 | EILPGSGSTNYNEKFKG | 84 |
| ASGR3-10M | HCDR3 | YGNYDAMDY | 85 |
| ASGR3-10M | LCDR1 | RASQSVSSSYLA | 86 |
| ASGR3-10M | LCDR2 | YASSRAT | 87 |
| ASGR3-10M | LCDR3 | QQSSSTPFT | 88 |
| ASGR3-39D | HCDR1 | SNYGMA | 89 |
| ASGR3-39D | HCDR2 | TISYDGSFTYYRDSVKG | 90 |
| ASGR3-39D | HCDR3 | KGRQADTEEDF | 91 |
| ASGR3-39D | LCDR1 | RASQSVSISSHDLMQ | 92 |
| ASGR3-39D | LCDR2 | DAFNLAS | 93 |
| ASGR3-39D | LCDR3 | QQSKDYPYT | 94 |
| ASGR4-70D | HCDR1 | SNYGMA | 95 |
| ASGR4-70D | HCDR2 | TISYDGSITYYRDSVKG | 96 |
| ASGR4-70D | HCDR3 | AHHRNTICSDF | 97 |
| ASGR4-70D | LCDR1 | RASQSVSISSHDLMQ | 98 |
| ASGR4-70D | LCDR2 | DAFNLAS | 99 |
| ASGR4-70D | LCDR3 | QQSKDDPYT | 100 |
| ASGR5-24M | HCDR1 | SSFGMH | 101 |
| ASGR5-24M | HCDR2 | YISSGSSTIYYANTVKG | 102 |
| ASGR5-24M | HCDR3 | LGPTVVATPFAY | 103 |
| ASGR5-24M | LCDR1 | KASQDVSTAVA | 104 |
| ASGR5-24M | LCDR2 | SASYRYS | 105 |
| ASGR5-24M | LCDR3 | QQYNNYPLT | 106 |

### (15-7) Evaluation of the activities of clones

### (15-7-i) Preparation of proteins

Clones were inoculated from master plates into LB medium containing 20 µg/ml carbenicillin and incubated at 37°C for 16 hours. Plasmids were purified from the bacterial pellets using the PureLink HiPure Plasmid Maxiprep Kit (Invitrogen).

### (14-7-ii) Preparation of proteins

Human embryonic kidney 293T cells were cultured in DMEM (1.0 g/l glucose) with L-Gln and Sodium Pyruvate, liquid (Nacalai) supplemented with 10% FCS (NICHIREI). Plating was performed in a 1:2 ratio on 6 cm dishes 24 hours before transfection. Lipofectamine 2000 or PEI-MAX in a volume of 20 µl was added to 500 µl of Opti-MEM I and allowed to stand for 5 minutes at room temperature, and the resulting mixture was gently added to a 6 cm dish containing 500 µl of Opti-MEM I and 8 µg of a plasmid DNA. The resulting mixture was allowed to stand for 20 minutes at room temperature, then added to a 6 cm dish containing 293T cells, and the cells were cultured at 37°C in 5% CO₂ for 4 to 6 day.

### (15-7-iii) Pull-down experiment

Resin (Streptavidin MagneSphere Paramagnetic Particles, Promega) was washed using the MagneSphere Technology Magnetic Separation Stand (tweleve-position). The resin in a volume of 40 µl was added to each 1.5 ml tube, and after removal of the solution, washed three times with 1 ml of PBS. The resin was transferred to new tubes and mixed with the biotinylated extracellular domain ASGR1ex or biotinylated extracellular domain ASGR2ex or with an equal volume of PBS as a no bait control, and the resulting mixtures were agitated at 4°C for 1 hour by rotation on a mini-disk rotor (BIO CRAFT) to allow binding. After removal of the solution, the resin was washed three times with 1 ml of TBST (containing 0.05% Tween 20). The resin was transferred to new tubes, blocked with 1 ml of ELISA BSA buffer (TBS, 0.05%, Tween 20, 1% BSA, Nacalai Tesque, Inc.) at 4°C for 1 hour, and then washed three times with 1 ml of ELISA BSA buffer. The resin was transferred to new tubes, and 1000 µl of the culture supernatant containing expressed ASGR3-10M, ASGR3-39D, ASGR4-70D, or ASGR5-24M was mixed with 40 µl of the resin treated with the biotinylated extracellular domain ASGR 1ex or biotinylated extracellular domain ASGR2ex or without the bait, and the resulting mixtures were agitated at 4°C for 1 hour and 30 minutes by rotation on a mini-disk rotor (BIO CRAFT) to allow binding. After removal of the solution, the resin was washed three times with 500 µl of TBST (Tween 20, 0.1%), and the recovered resin was used for Western blotting.

Each resin was mixed with water and LDS sample buffer (4x), 0.2 mM DTT. heated at 70°C for 10 minutes, and then subjected to SDS-PAGE. SDS-PAGE was performed using 4-12% Bis-Tris gel and NuPAGE MES-SDS electrophoresis buffer (Invitrogen) at 200 V and 400 mA for 35 minutes. After electrophoresis, transfer was performed using Mini Format, 0.2-µm PVDF, Single-application (BIO-RAD) Trans-Blot Turbo. The membrane was blocked with a Blocking One Buffer : TBST (1 :9) solution and reacted with an anti-Flag-HRP (Sigma: A8592) diluted 2:3000 in a Blocking One Buffer : TBST (1:9) solution. Detection was performed using ECL (Enhanced ChemiLuminescence) on a ChemiDoc (BIO-RAD).

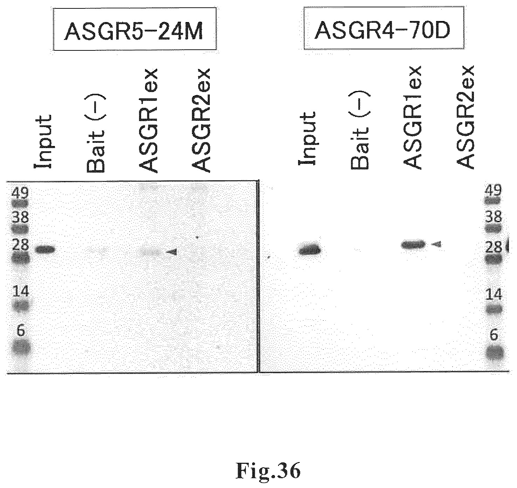

Pull-down assays with ASGR-binding single-chain antibodies were performed (Figs. 35 and 36). ASGR3-10M and ASGR3-39D, obtained by 3 rounds of selection experiments, recognized the antigens strongly compared to the control. ASGR3-10M recognized both antigens, while ASGR3-39D recognized the ASGR1 antigen particularly strongly. ASGR4-70D and ASGR5-24M, obtained by 4 and 5 rounds of selection experiments, recognized the antigens well compared to the control. ASGR4-70D and ASGR5-24M recognized the ASGR1 antigen particularly strongly.

### [Example 16] : Preparation of LIPG-binding peptide-anti-ASGR single-chain antibody fusions

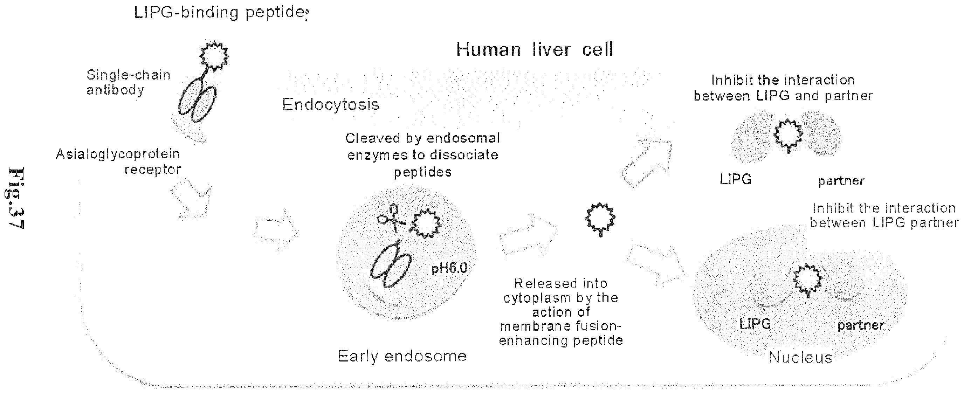

Fig. 37 shows a schematic diagram of hepatocyte-selective delivery of LIPG-binding peptides using an anti-ASGR single-chain antibody. A LIPG-binding peptide linked to an anti-ASGR antibody is bound to the receptor and then incorporated into early endosomes by endocytosis. Thanks to a cleavage sequence that is cleavable by an endosomal enzyme and is placed upstream of a LIPG-binding peptide, the peptide is dissociated when the cleavage sequence is cleaved. A membrane fusion-enhancing peptide is placed downstream of a LIPG-binding peptide, by which the peptide is released into the cytoplasm. When a LIPG-binding peptide should be delivered to the nucleus, a nuclear localization signal is added to the peptide.

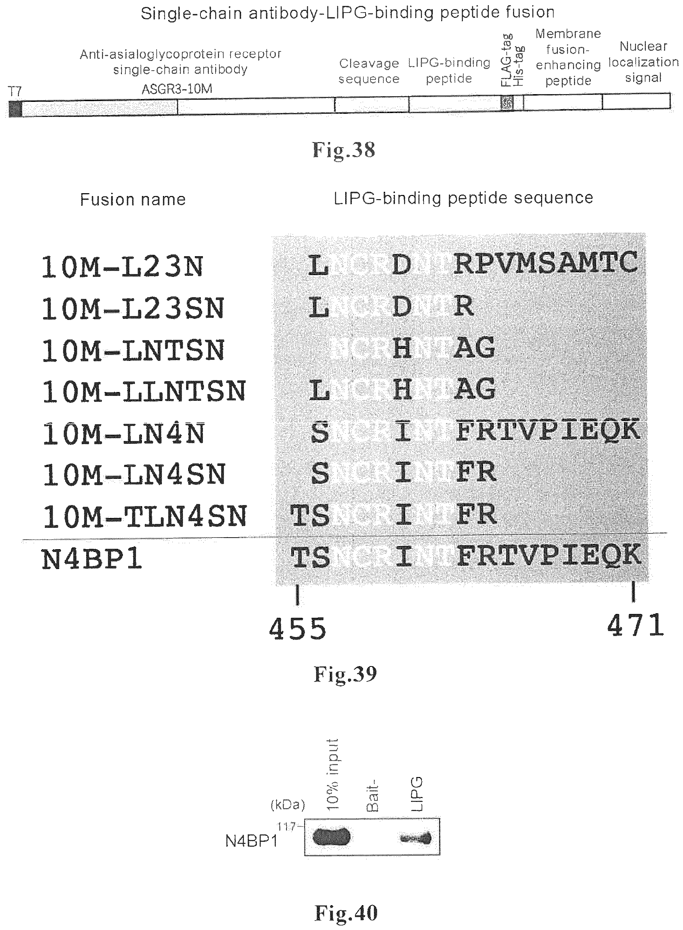

In this example, single-chain antibody-LIPG-binding peptide fusions having the structure shown in Fig. 38 were produced.

Multiple LIPG-binding peptides and multiple ASGR-binding single-chain antibodies were obtained as results of selection experiments based on the IVV method. As shown in Fig. 39, fusions were generated using ASGR3-10M as an anti-ASGR single-chain antibody and LIPH4-23 (SEQ ID NO: 1), LIPH4-23s (SEQ ID NO: 23), LIPH4-NTNBs partial polypeptide of Netrin-1 (SEQ ID NO: 25), LIPH4-LNTNBs partial polypeptide of Netrin-1 (SEQ ID NO: 77), LIPH4-N4BP1a partial polypeptide of N4BP1 (SEQ ID NO: 78). LIPH4-N4BP1b partial polypeptide of N4BP1 (SEQ ID NO: 79), or LIPH4-N4BP1c partial polypeptide of N4BP1 (SEQ ID NO: 80) as a LIPG-binding peptide. The required functional peptides were cloned into the expression vectors carrying the genes by in-fusion cloning to generate constructs. In this example, the sequence recognized by the endogenous protease Furin (RVRR, SEQ ID NO: 111) was used as a cleavage sequence, and the membrane fusion-enhancing peptide S28 (PFVIGAGVLGALGTGIGGITTSTQFYYK. SEQ ID NO: 107) or S39 (PFVIGAGVLGALGTGIGGITTSTQFYYKLSQELNGDMER, SEQ ID NO: 108) was used as a membrane penetration-enhancing peptide, and the sequence PAAKRVKLD (SEQ ID NO: 110) was used as a nuclear localization signal. The prepared constructs were named 10M-L23N, 10M-L23SN, 10M-L23SN-S39, 10M-LNTSN, 10M-LLNTSN, 10M-LLNTSN-S39, 10M-LN4N, 10M-LN4SN, and 10M-TLN4SN (Table 16). In these constructs, the amino acid sequences of the portions of the cleavage sequence + a LIPG-binding peptide + the S28/S39 + the nuclear localization signal are set forth in SEQ ID NOs: 127 to 135.

**[Table 16]**

| Name of fusion | Name of LIPG-binding peptide | Peptide sequence | Sequence of cleavage sequence + LIPG-binding peptide + S28/S39 + nuclear localization signal |
|---|---|---|---|
| 10M-L23N | LIPH4-23 | LNCRDNTRPVMSAMTC (SEQ ID NO: 1) | rvrrLNCRDNTRPVMSAMTCDYKDDDD KHHHHHHHPFVIGAGVLGALGTGIGGI TTSTQFYYKPAAKRVKLD (SEQ ID NO: 127) |
| 10M-L23SN | LIPH4-23s | LNCRDNTR (SEQ ID NO: 23) | rvrr LNCRDNTR DYKDDDDKHHHHHH PFVIGAGVLGALGTGIGGITTSTQFYY KPAAKRVKLD (SEQ ID NO: 128) |
| 10M-L23SN-S39 | LIPH4-23s | LNCRDNTR (SEQ ID NO: 23) | rvrr LNCRDNTR DYKDDDDKHHHHHHH PFVIGAGVLGALGTGIGGITTSTQFYY KLSQELNGDMERPAAKRVKLD (SEQ ID NO: 129) |
| 10M-LNTSN | LIPH4-NTNBs | NCRHNTAG (SEQ ID NO: 25) | rvrrNCRHNTAGDYKDDDDKHHHHHHH PFVIGAGVLGALGTGIGGITTSTQFYY KPAAKRVKLD (SEQ ID NO: 130) |
| 10M-LLNTSN | LIPH4-LNTNBs | LNCRHNTAG (SEQ ID NO: 77) | rvrr LNCRHNTAG DYKDDDDKHHHHHH HPFVIGAGVLGALGTGIGGITTSTQFY YKPAAKRVKLD (SEQ ID NO: 131) |
| 10M-LLNTSN-S39 | LIPH4-LNTNBs | LNCRHNTAG (SEQ ID NO: 77) | rvrr LNCRHNTAG DYKDDDDKHHHHHH HPFVIGAGVLGALGTGIGGITTSTQFY YKLSQELNGDMERPAAKRVKLD (SEQ ID NO: 132) |
| 10M-LN4N | LIPH4-N4BP1a | SNCRINTFRTVPIEQK (SEQ ID NO: 78) | rvrrSNCRINTFRTVPIEQKDYKDDDD KHHHHHHPFVIGAGVLGALGTGIGGI TTSTQFYYKPAAKRVKLD (SEQ ID NO: 133) |
| 10M-LN4SN | LIPU4-N4BP1b | SNCRINTFR (SEQ ID NO: 79) | rvrr SNCRINTFR DYKDDDDK HHHHHH HPFVIGAGVLGALGTGIGGITTSTQFY YKPAAKRVKLD (SEQ ID NO: 134) |
| 10M-TLN4SN | LIPH4-N4BP1c | TSNCRINTFR (SEQ ID NO: 80) | rvrr TSNCRINTFR DYKDDDDKHHHHH HHPFVIGAGVLGALGTGIGGITTSTQF YYKPAAKRVKLD (SEQ ID NO: 135) |

| | | | |
|---|---|---|---|
| Lowercase letters: cleavage sequence, enclosed letters: LIPG-binding peptide. single-underlined letters: Flag-tag. wavy-underlined letters: His-tag. dotted-underlined letters: S28 or S39. double-underlined letters: nuclear localization signal | | | |

### [Example 17] : Pull-down assay of N4BP1 against full-length LIPG

Since the LIPG-binding peptides were found to have a homology to N4BP1, a pull-down assay was performed to determine whether or not N4BP1 binds to LIPG.

Resin (Streptavidin MagneSphere Paramagnetic Particles, Promega) was washed using the MagneSphere Technology Magnetic Separation Stand ( tweleve-position). The resin in a volume of 40 µl was added to each 1.5 ml tube, and after removal of the solution, washed three times with 1 ml of PBS. The resin was transferred to new tubes and mixed with the biotinylated full-length LIPG or with an equal volume of PBS as a no bait control, and the resulting mixtures were agitated at 4°C for 1 hour by rotation on a mini-disk rotor (BIO CRAFT) to allow binding. After removal of the solution, the resin was washed three times with 1 ml of TBST (0.05% Tween 20). The resin was transferred to new tubes, blocked with 1 ml of ELISA BSA buffer (TBS. 0.05%. Tween 20, 1% BSA, Nacalai Tesque, Inc.) at 4°C for 1 hour, and then washed three times with 1 ml of ELISA BSA buffer. The resin was transferred to new tubes, and 1000 µl of the culture supernatant containing expressed N4BP1 was mixed with 40 µl of the resin treated with the biotinylated full-length LIPG or without the bait, and the resulting mixtures were agitated at 4°C for 1 hour and 30 minutes by rotation on a mini-disk rotor (BIO CRAFT) to allow binding. After removal of the solution, the resin was washed three times with 500 µl of TBST (Tween 20, 0.1%), and the recovered resin was used for Western blotting.

Each resin was mixed with water and LDS sample buffer (4x), 0.2 mM DTT, heated at 70°C for 10 minutes, and then subjected to SDS-PAGE. SDS-PAGE was performed using 4-12% Bis-Tris gel and NuPAGE MES-SDS electrophoresis buffer (Invitrogen) at 200 V and 400 mA for 35 minutes. After electrophoresis, transfer was performed using Mini Format. 0.2-µm PVDF, Single-application (BIO-RAD) Trans-Blot Turbo. The membrane was blocked with a Blocking One Buffer : TBST (1:9) solution and reacted with a T7-tag HRP conjugate (Novagen 69048) diluted 2:3000 in a Blocking One Buffer : TBST (1 :9) solution. Detection was performed using ECL (Enhanced ChemiLuminescence) on a ChemiDoc (BIO-RAD).

The pull-down assay of N4BP1, which was found to be homologous to the LIPG-binding peptides, against LIPG (Fig. 40) has revealed that N4BP1 binds to LIPG.

### [Example 18] : Anti-HBV activities of LIPG-binding peptides delivered into PXB cells -1

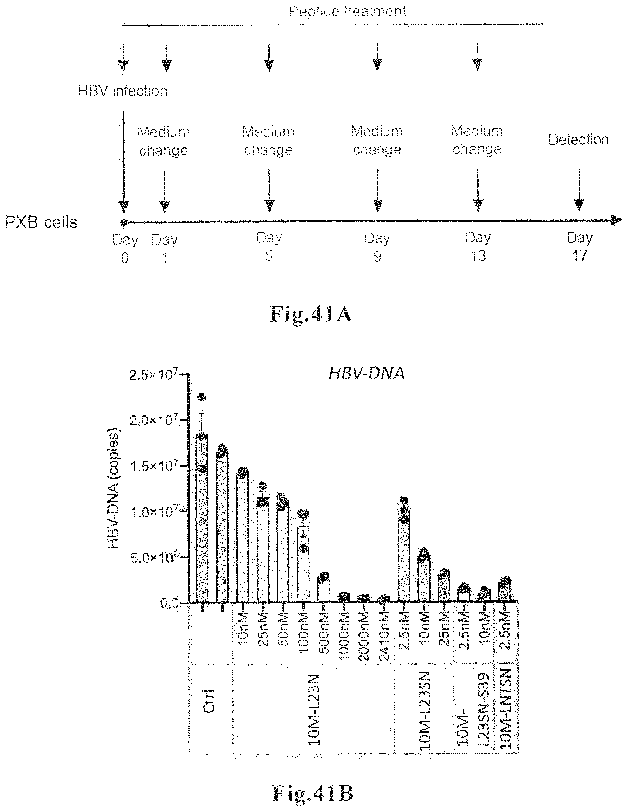

The anti-HBV activities of LIPG-binding peptides delivered into cells were evaluated using PXB cells and anti-ASGR single-chain antibody-LIPG-binding peptide fusions. Of the fusions prepared in Example 16, 10M-L23N, 10M-L23SN, 10M-L23SN-S39. and 10M-LNTSN were used. According to the timeline shown in Fig. 41A, the anti-HBV activities of the fusions were evaluated using the methods described in "(10-5) Infection experiment using PXB cells" and "(10-6) Quantification of HBV-DNA and cccDNA".

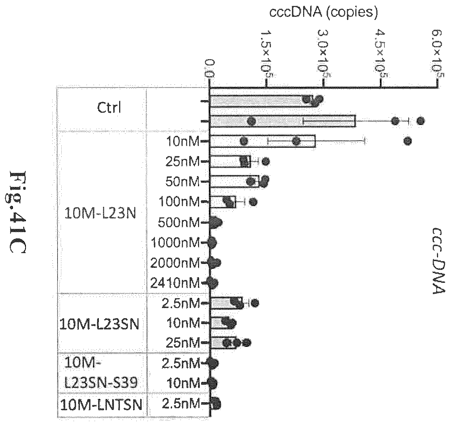

The anti-HBV activities of LIPG-binding peptides delivered into PXB cells are shown in Fig. 41B and C. The LIPG-binding peptides 10M-L23N, 10M-L23SN, 10M-L23SN-S39, and 10M-LNTSN all remarkably reduced HBV-DNA and cccDNA copy numbers at peptide concentrations (nM) indicated in the figures, indicating that the LIPG-binding peptides exhibit very high anti-HBV activity.

10M-L23SN and 10M-L23SN-S39 were prepared using the LIPG-binding peptide LlPH4-23s. while 10M-LNTSN was prepared using the LIPG-binding peptide LIPFI4-NTNBs. The anti-HBV activities obtained when LlPH4-23s or LIPH4-NTNBs was added to a culture of PXB cells without attachment to a carrier for delivery into liver cells are shown in Fig. 19B to E. The concentrations of the added peptides ranged from 1 to 100 µM. In contrast, the fusions 10M-L23SN-S39 and 10M-LNTSN remarkably reduced HBV-DNA and cccDNA copy numbers at added concentrations ranging from as low as 2.5 to 25 nM (Fig. 41B and C). Comparison of these data revealed that the anti-HBV activity of a LIPG-binding peptide is increased approximately 100 to 1000-fold when the peptide is delivered into liver cells.

### [Example 19] : Anti-HBV activities of LIPG-bindingpeptides delivered into PXB cells -2

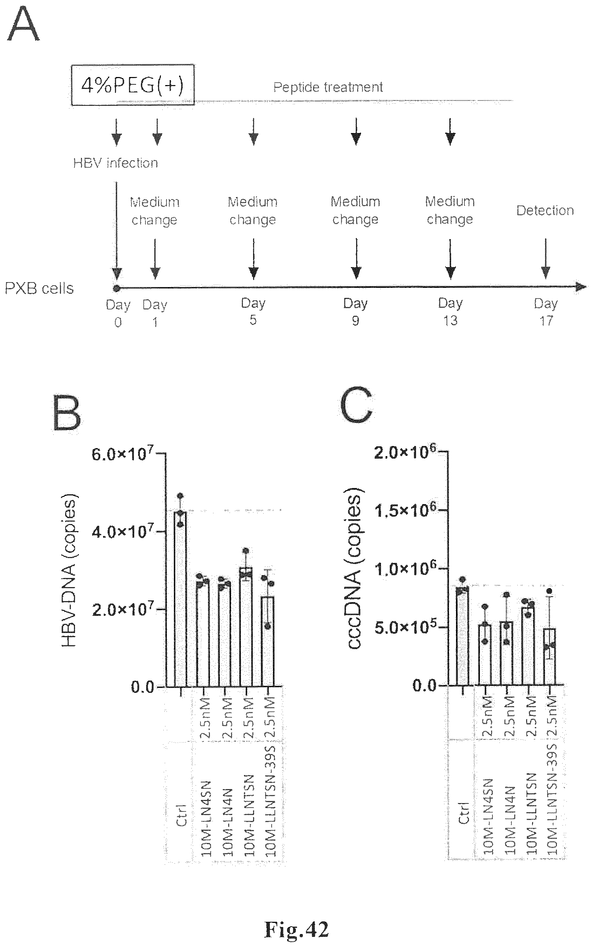

The anti-HBV activities of LIPG-binding peptides delivered into cells were evaluated using PXB cells and anti-ASGR single-chain antibody-LIPG-binding peptide fusions. The anti-HBV activity was evaluated in the presence of 4% PEG. The evaluation of the anti-HBV effect in the presence of PEG is more stringent than under the conditions in Example 18 without the presence of PEG because the HBV-DNA and cccDNA copy numbers are increased in the presence of PEG. Of the fusions prepared in Example 16, 10M-LN4SN, 10M-LN4N, 10M-LLNTSN. and 10M-LLNTSN-S39 were used. According to the timeline shown in Fig. 42A. the anti-HBV activities of the fusions were evaluated using the methods described in "(10-5) Infection experiment using PXB cells" and "(10-6) Quantification of HBV-DNA and cccDNA".

The anti-HBV activities of LIPG-binding peptides delivered into PXB cells are shown in Fig. 42B and C. The LIPG-binding peptides 10M-LN4SN. 10M-LN4N. 10M-LLNTSN, and 10M-LLNTSN-S39 all remarkably reduced HBV-DNA and cccDNA copy numbers even under the stringent condition due to the presence of PEG. indicating that the LIPG-binding peptides have very high anti-HBV activity.

### [Example 20] : Mode of action of LIPG-binding peptides in cells

As described above, the LIPG-binding peptide LIPH4-23, which was obtained as a LIPG-binding peptide by the IVV screening, was found to be homologous not only to the sequence of netrins but also to the sequence of N4BP1 (NEDD4 Binding Protein 1). Since N4BP1 has RNase activity (the region of residues 617 to 769 in SEQ ID NO: 82 is an RNase domain, and D623, D704, and D723 are catalytic sites), N4BP1 may act to maintain HBV by binding to LIPG to suppress degradation of viral mRNA in HBV-infected cells.

Therefore, we tested *in vitro* whether or not LIPG (500 aa. MW: 56.8 kDa) binds to N4BP1 to inhibit the degradation of HBV pregenomic RNA and promote HBV proliferation, and whether or not a LIPG-binding peptide binds to LIPG to rescue the inhibition of the N4BP1 RNase activity by LIPG and promote the degradation of HBV pregenomic RNA, thereby suppressively acting on HBV proliferation. The RNA used was a 1203-base RNA encoding the HBV L protein. The LIPG-binding peptide used was LIPH4-NTNBs (SEQ ID NO: 25), which is also highly homologous to N4BP1. First, N4BP1, LIPG, and LIPH4-NTNBs were preincubated at 37°C for 30 minutes, and the RNA was added thereto, followed by incubation of the resulting mixture at 37°C for 15 minutes to allow the reaction to proceed. The concentrations of the RNA, N4BP1, LIPG, and LIPH4-NTNBS were 20 nM, 50 pM, 50 pM, and 50 pM, respectively, and the composition of the buffer was 25 mM HEPES, 50 mM potassium acetate. 5 mM DTT. 5 mM magnesium acetate, and 0.2 U/ml RNase inhibitor (TOYOBO). and the total volume of the reaction mixture was 10 µl.

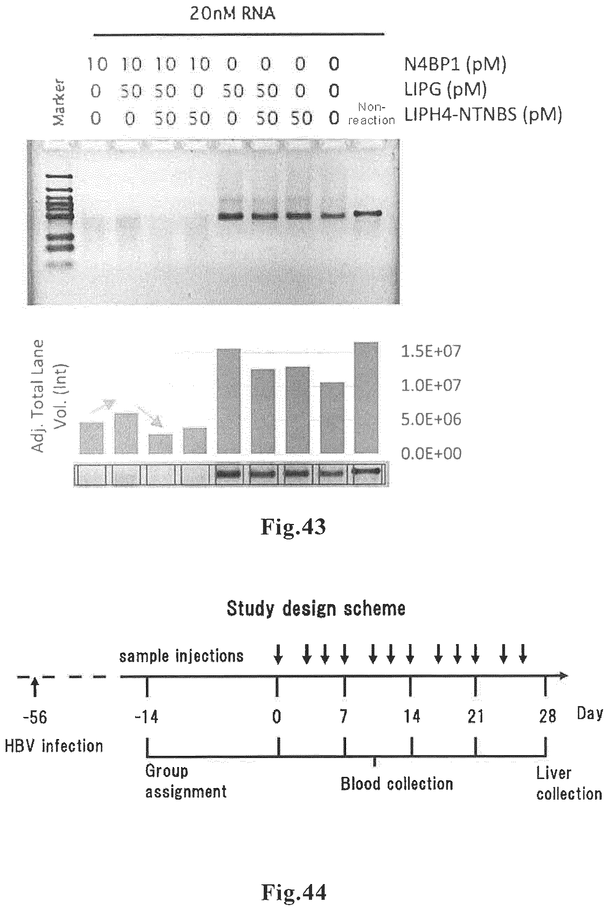

Fig. 43 shows the reaction products electrophoresed on a 2% TAE agarose gel (buffer: TAE) and stained with ethidium bromide. As shown in lane 1. the RNA was confirmed to be degraded by N4BP1 . As shown in lane 2, the degradation of the RNA was suppressed in the presence of LIPG by binding of LIPG to N4BP1. When the LIPG-binding peptide LIPH4-NTNBs was added, as shown in lane 3, LIPH4-NTNBs inhibited the binding of LIPG to N4BP1 and thus rescued the degradation of the RNA by N4BP1. As shown in lane 4. the degradation of the RNA by N4BP1 occurred when LIPG was absent. Lanes 5 to 8 show that the materials other than N4BP1 have no effect on the degradation of the RNA.

These results suggest that LIPG represses the RNase activity of N4BP1 and facilitates HBV maintenance, and that when binding between LIPG and N4BP1 is inhibited by a LIPG-binding peptide, the RNase activity of N4BP1 is rescued to suppress HBV proliferation.

### [Example 21] : Drug efficacy testing of anti-ASGR antihody-LIPG-binding peptide fusion using HBV-infected PXB mice

To confirm the drug efficacy of a LIPG-binding peptide fused to a carrier for delivery into hepatocytes, an anti-ASGR single-chain antibody-LIPG-binding peptide fusion was administered to HBV-infected PXB mice to evaluate the anti-HBV effect of the fusion. The fusion used was 10M-LNTSN. a fusion of LIPH4-NTNBs (SEQ ID NO: 25) and an anti-ASGR single-chain antibody.

### (21-1) Test substance, solvent, and preparation method

### Agent A: 10M-LNTSN

Characteristics: solution in PBS
Quantity: 936 µg/21.6mL (78 µg/1800 µL × 12 vials)
Storage: refrigeration
Preparation method: the stock solution with a concentration of 78 µg/1800 µL was diluted 1-fold and administered at a dose of 300 µL/body (13 µg/body).

### Agent B: 10M-LNTSN

Characteristics: solution in PBS
Quantity: 936 µg/21.6mL (78 µg/1800 µL × 12 vials)
Storage: refrigeration
Preparation method: the agent A was diluted 5-fold in PBS and administered at a dose of 300 µL/body (2.6 µg/body).

### Solvent

PBS was used, similar to Example 13.

### (21-2) Virus used

HBV strain PBB004 (Genotype C) (viral titer: 1.1E+09 copies/mL) purchased from PhoenixBio Co., Ltd. was adjusted to 1.0E+06 copies/mL with saline, similar to Example 13.

### (21-3) Animal used

PXB mice purchased from PhoenixBio Co., Ltd. were used, similar to Example 13. In this example, the number of animals used was 30.

### (21-4) Animal care conditions

The conditions were the same as those in Example 13.

### (21-5) Test group organization and dosing schedule

The test group organization and the closing schedule were as shown in Table 17 below.

**[Table 17]**

| Group | Administered substance | Dosage (/body) | Dosing route | Dosing period*¹ | Sampling date*² | Animal ID number (n) |
|---|---|---|---|---|---|---|
| 1 | agent A | 300 µL | ip | Day 0 to 27 | Day 28 | 01-05 (5) |
| 2 | agent B | 300 µL | ip | Day 0 to 27 | Day 28 | 11-15 (5) |
| 3 | solvent | 300 µL | ip | Day 0 to 27 | Day 28 | 21-25 (5) |

* 1, Day 0: the day of the first dose of a test substance (at 8 weeks after virus inoculation)
*2, samples were collected every two to three days (on Monday, Wednesday, and Friday) during the dosing period.

### Virus inoculation, test substance administration

The same procedures were performed as in Example 13.

### Blood collection

Approximately 70 µL of blood was collected from the subclavian vein at week 6 of the virus inoculation period (2 weeks before the first dose) and weekly from the time just prior to the first dose (day 0: week 8 of the virus inoculation period) through day 21. At the time of autopsy (day 28), as much blood as possible (400 µL or more) was collected from the abdominal vena cava or heart of the mice under isoflurane inhalation anesthesia. After blood collection was completed, for measurement of the blood h-Alb concentration 2 µL of the blood was mixed with 200 µL ofsaline and centrifuged at 390 ×g for 10 minutes at room temperature, and the supernatant was transferred to a 1.5 ml tube (INA-OPTIKA, RC-0150) and cryopreserved (at -60°C or lower) until sent to an analysis contractor. The remainder of the blood was centrifuged at 4°C for 15 minutes at 6000 rpm to separate the serum (during consecutive blood collection (for measurement of HBV-related factors): 30 µL (5 µL × 2 tubes, 20 µL × 1 tube), at autopsy: 160 µL or more (5 µL × 2 tubes, 20 µL × 1 tube, the rest: 1 tube)) and cryopreserved (at -60°C or lower) until sent to an analysis contractor.

### Autopsy

The same procedure was performed as in Example 13.

### Measurement of HBsAg, HBeAg, and HBcrAg

The HBsAg concentration in the sera was measured by SRL, Inc. (Tokyo). The Lumipulse (registered trademark) PrestoII (FUJIREBIO Inc., Tokyo) using the chemiluminescence enzyme immunoassay (CLEIA) was used for the measurement. The measurement range was from 0.005 to 150 lU/mL. The dilution ratio used for a measured sample in this study was 30, and the measurement range under this dilution ratio was from 0.15 to 4500 IU/mL.

The HBsAg concentration in the sera was measured by SRL, Inc. (Tokyo). The Lumipulse (registered trademark) PrestoII using the CLEIA was used for the measurement. The measurement range was from 0.1 to 1590 C.O.I. The dilution ratio used for a measured sample in this study was 30, and the measurement range under this dilution ratio was from 3 to 47700 C.O.I.

The HBc-rAg concentration in the sera was measured by SRL, Inc. (Tokyo). The LUMIPULSE HBcrAg and LUMIPULSE F (FUJIREBIO Inc., Tokyo) using the CLEIA were used for the measurement. The lower detection limit was 3.0 log U/mL. The dilution ratio used for a measured sample in this study was 300, and the lower detection limit under this dilution ratio was 5.5 log U/mL.

### Measurement of h-Alb

The following procedure was performed at PhoenixBio Co., Ltd. Two µL of blood was diluted with 200 µL of saline and centrifuged at 390 ×g for 10 minutes at room temperature. Blood h-Alb concentration was measured on a BioMajestyTM automated analyzer (JCA-BM6050, JEOL, Tokyo) using the latex agglutination turbidimetric immunoassay (LZ Test 'Eiken' U-ALB, EIKEN CHEMICAL Co., Ltd., Tokyo).

### Measurement of ALT

ALT was measured by PhoenixBio Co., Ltd. using the sera collected at the autopsy. Plasma ALT activity was measured using 10 µL each of the collected plasma samples. The substance to be measured was a diarylimidazole leuco dye (the diarylimidazole leuco dye was colored blue by a peroxidase and hydrogen peroxide generated by a pyruvate oxidase), and the DRI-CHEM 7000/NX500sV was used for the measurement.

### Measurement of blood HBV-DNA levels

The measurement of the HBV-DNA level in the blood was performed KUBIX Inc. via PhoenixBio Co., Ltd.

The sera were mixed with the HBV Pre-treatment Solution for HBV-DNA in Serum and heated at 98°C for 5 minutes to inactivate HBV and to expose the HBV-DNA. The KUBIX HBV qPCR Kit (KUBIX Inc.) and the CFX96 Touch^{™} Real-Time PCR Detection System (Bio-Rad Laboratories, Inc., Hercules, CA, USA) were used for PCR reaction and analysis. Ten µL of HBV 2 × PCR solution was mixed with 10 µL of the heat-treated standard or measurement sample. The PCR reaction was performed at 95°C for 2 minutes → (95°C for 5 seconds -> 54°C for 30 seconds) × 45 cycles. The concentration of HBV DNA in the blood was calculated as the average of those in 2 wells. The lower detection limit of this quantification method is 4.0E+04 copies/mL. Sera obtained from HBV-infected PXB mice were used as the HBV DNA standard. The concentration of HBV DNA in the sera was quantified by digital PCR. The primers and probe used were the same as those used in Example 13 to measure HBV-DNA levels in the liver. Sera obtained from HBV-infected PXB mice were used as the HBV DNA standard.

### Measurement of HBV-DNA in the liver

The same procedure was performed as in Example 13.

### Measurement of cccDNA in the liver

The same procedure was performed as in Example 13.

### Pathohistological examination (HBsAg immunostaining, HBcAg immunostaining)

The same procedure was performed as in Example 13.

### (21-6) Observation and measurement items

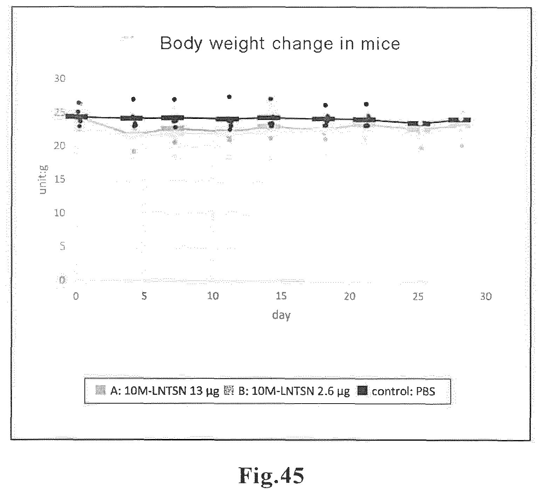

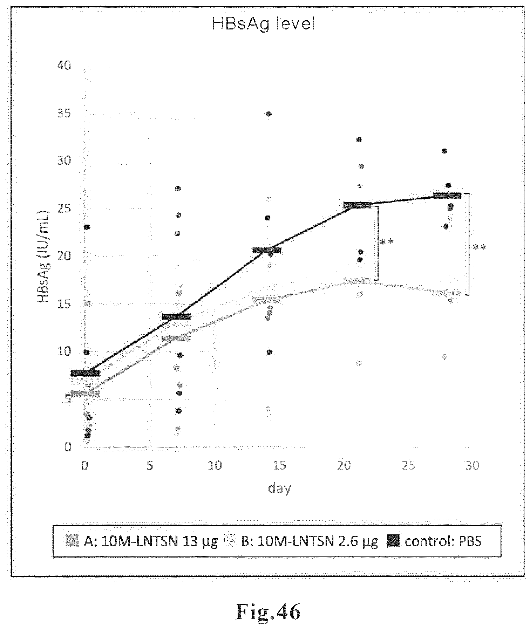

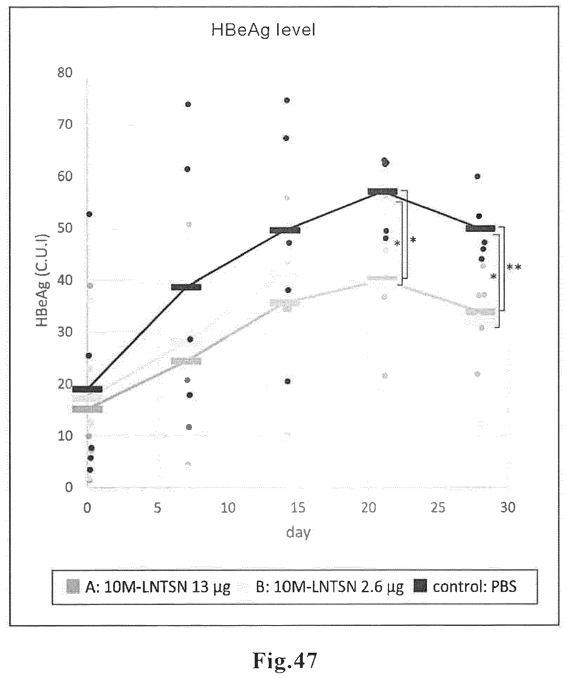

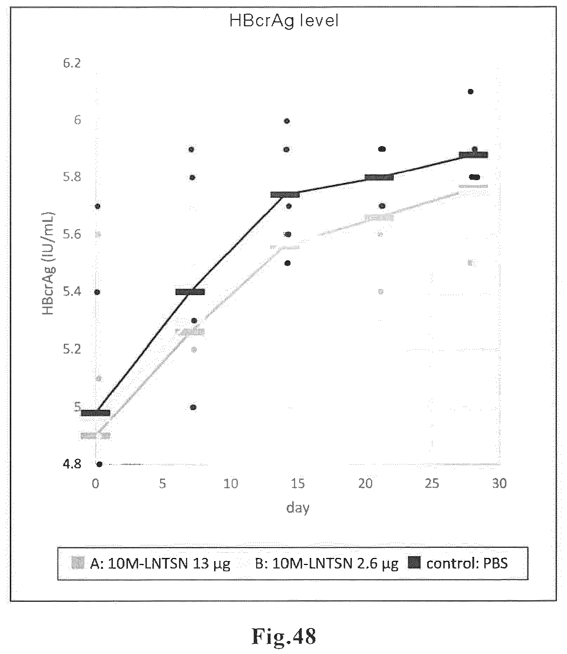

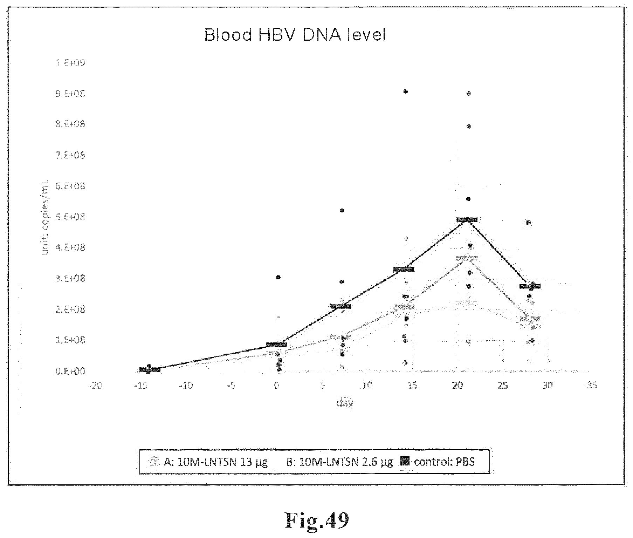

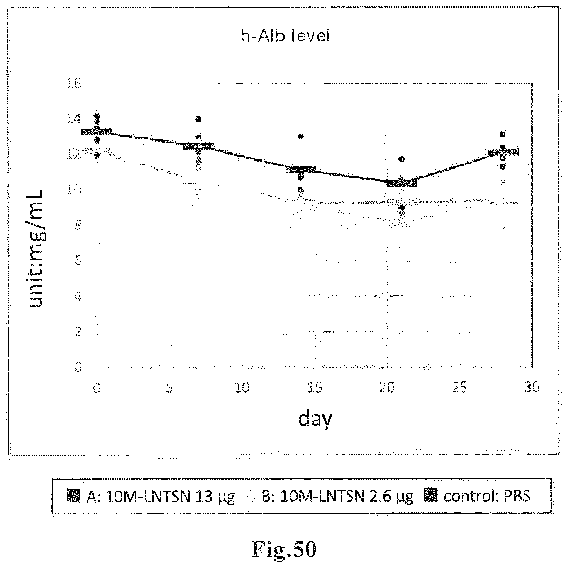

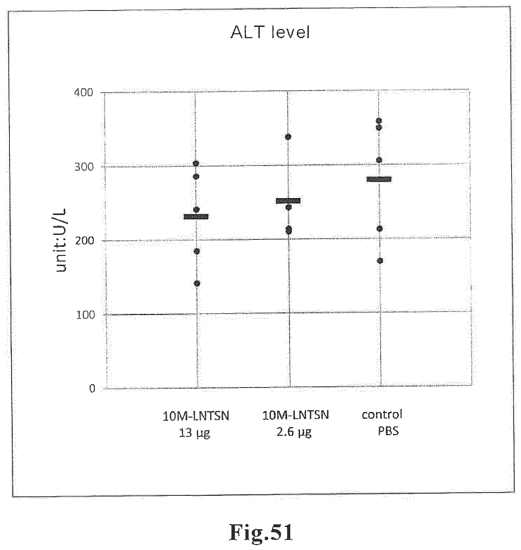

Observation and measurements were made according to the sample dosing schedule shown in Fig. 44. According to the observation of change in body weight (Fig. 45) and general condition of the mice, the 10M-LNTSN administration group showed no abnormalities compared to the control (PBS). When 10M-LNTSN is compared to the control (PBS) for HBsAg (Fig. 46), HBeAg (Fig. 47) and HBcrAg (Fig. 48), the result indicates that 10M-LNTSN was inhibitory to all factors. 10M-LNTSN was also inhibitory to HBV DNA in the blood (Fig. 49). The result of the h-Alb measurement (Fig. 50) indicates that an abundance of human albumin was produced in the chimeric mice. This assay was excellent as an assay to carry out evaluation in human liver cells. The result of the ALT measurement (Fig. 51) indicates that 10M-LNTSN did not exacerbate hepatitis compared to the control (PBS).

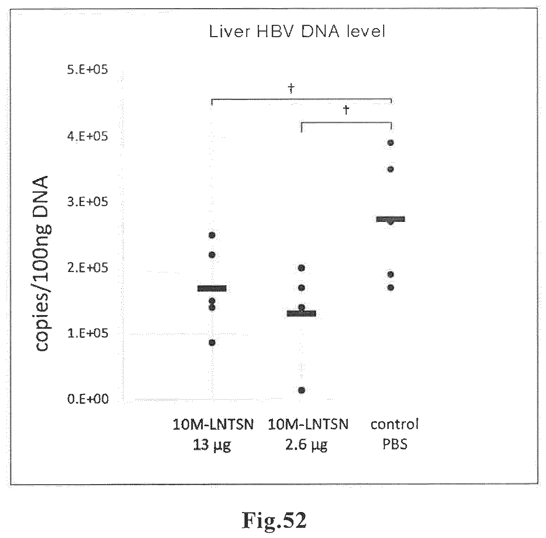

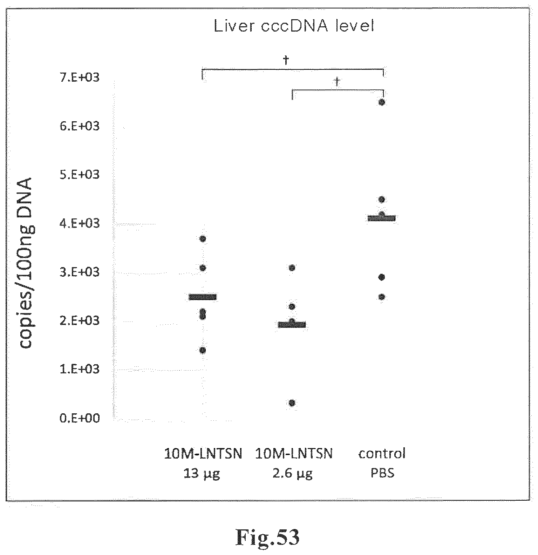

The HBV-DNA level was measured in the livers obtained during the autopsy (Fig. 52), and it was found that 10M-LNTSN reduced the copy number of HBV-DNA compared to the control administration group. Similarly, the copy number of cccDNA in the liver was measured (Fig. 53), and it was found that 10M-LNTSN reduced the copy number of FIBV-DNA compared to the control administration group.

These results have demonstrated that 10M-LNTSN significantly reduces HBV-DNA and cccDNA in the liver and HBsAg and HBeAg in the blood in the animal experiment using human hepatocyte chimeric mice infected with HBV and has an apparent anti-HBV effect compared to the control group without the addition of the peptide. As a result, it can be seen that the POC (proof of concept) for 10M-LNTSN has been achieved in animal studies using human hepatocyte chimeric mice.

10M-LNTSN used in Example 21 is a fusion prepared using the LIPG-binding peptide LIPH4-NTNBs. In the experiment of Example 13, LIPH4-NTNBs without attachment to a carrier for delivery into hepatocytes is administered to PXB mice, and the results of the experiment are shown in Figs. 23 to 26. When the results of the measurement of the liver HBV DNA and cccDNA in Example 13 (Figs. 23 and 24) are compared with the results of the measurement of the liver HBV DNA and cccDNA in Example 21 (Figs. 52 and 53), it is apparent that the fusion exerts an anti-HBV effect equivalent to that of the unfused form of the original LIPG-binding peptide at approximately 1000 times lower dosage (in grams). Comparison of these data indicated that the *in vivo* anti-HBV effect of a LIPG-binding peptide is increased approximately 1000-fold or more when the peptide is delivered into liver cells.

## Claims

1. An anti-hepatitis B virus agent containing as an active ingredient a substance that binds to LIPG and inhibits the function(s) of LIPG.

2. The anti-hepatitis B virus agent according to claim 1, wherein the inhibition of LIPG function(s) is at least one selected from inhibition of binding of LIPG to N4BP1 and inhibition of suppression by LIPG of the RNase activity of N4BP1.

3. The anti-hepatitis B virus agent according to claim 1 or 2, wherein the substance has an activity to inhibit hepatitis B virus entry into cells mediated by LIPG.

4. The anti-hepatitis B virus agent according to claim 3. wherein the substance binds to the heparin-binding region of LIPG to inhibit the binding of LIPG and heparan sulfate proteoglycan to hepatitis B virus or the binding of the LIPG-heparan sulfate proteoglycan complex to hepatitis B virus.

5. The anti-hepatitis B virus agent according to claim 1. wherein the substance is at least one polypeptide selected from the following polypeptides (1) to (30):
(1) a polypeptide of the sequence NCRHNTAG (SEQ ID NO: 25);
(2) a polypeptide of the sequence NARHNTAG (SEQ ID NO: 26);
(3) a polypeptide of the sequence LNCRDNTR (SEQ ID NO: 23);
(4) a polypeptide of the sequence LNARDNTR (SEQ ID NO: 24);
(5) a polypeptide of the sequence LNCRDNTRPVMSAMTC (SEQ ID NO: 1);
(6) a polypeptide of the sequence IRNVNHSDH (SEQ ID NO: 9);
(7) a polypeptide of the sequence LNVGYVFYP (SEQ ID NO: 18);
(8) a polypeptide of the sequence SL YTG FRAI-I (SEQ ID NO: 15);
(9) a polypeptide of the sequence RCRQSWNTM (SEQ ID NO: 14);
(10) a polypeptide of the sequence GADLRRGCC (SEQ ID NO: 11);
(11) a polypeptide of the sequence RKSGGVCENCRHNTAG (SEQ ID NO: 21);
(12) a polypeptide of the sequence LNCRHNTAGRHCHYCK (SEQ ID NO: 22);
(13) a polypeptide of an amino acid sequence shown in SEQ ID NO: 28;
(14) a polypeptide composed of a partial sequence of the polypeptide (13) and containing a region of amino acid positions 370 to 377 in the sequence of SEQ ID NO: 28;
(15) a polypeptide having 95% or more identity to the amino acid sequence shown in SEQ ID NO: 28, which is represented by an amino acid sequence identical to the amino acid sequence shown in SEQ ID NO: 28 except that one or more amino acids are substituted, deleted, inserted, and/or added in any region outside a region of amino acid positions 370 to 377;
(16) a polypeptide composed of a partial sequence of the polypeptide (15) and containing the region of amino acid positions 370 to 377;
(17) a polypeptide of the sequence LCECRDVLSCYYITDT (SEQ ID NO: 4);
(18) a polypeptide of the sequence CPCVNGATRHRPTSLC (SEQ ID NO: 8);
(19) a polypeptide of the sequence LVPWLRY A Y (SEQ ID NO: 17);
(20) a polypeptide of the sequence NXRHNTAG (SEQ ID NO: 75) (X represents an amino acid except for cysteine and alanine):
(21) a polypeptide of the sequence LNXRDNTR (SEQ ID NO: 76) (X represents an amino acid except for cysteine and alanine);
(22) a polypeptide of the sequence LNCRHNTAG (SEQ ID NO: 77);
(23) a polypeptide of the sequence SNCRINTFRTVPIEQK (SEQ ID NO: 78);
(24) a polypeptide of the sequence SNCRINTFR (SEQ ID NO: 79);
(25) a polypeptide of the sequence TSNCRINTFR (SEQ ID NO: 80);
(26) a polypeptide of an amino acid sequence shown in SEQ ID NO: 82;
(27) a polypeptide composed of a partial sequence of the polypeptide (26) and containing a region of amino acid positions 456 to 464 in the sequence of SEQ ID NO: 82;
(28) a polypeptide having 95% or more identity to the amino acid sequence shown in SEQ ID NO: 82, which is represented by an amino acid sequence identical to the amino acid sequence shown in SEQ ID NO: 82 except that one or more amino acids are substituted, deleted, inserted, and/or added in any region outside a region of amino acid positions 456 to 464;
(29) a polypeptide composed of a partial sequence of the polypeptide (28) and containing the region of amino acid positions 456 to 464;
(30) a polypeptide having an identity of 80% or more and less than 100% to any of (1) to (14) and (17) to (27).

6. The anti-hepatitis B virus agent according to claim 5, wherein the polypeptide (15) has 98% or more identity to SEQ ID NO: 28 and the polypeptide (28) has 98% or more identity to SEQ ID NO: 82.

7. The anti-hepatitis B virus agent according to claim 5, containing at least one polypeptide selected from said (1) to (13) and (22) to (26) as an active ingredient.

8. The anti-hepatitis B virus agent according to claim 5, containing at least one polypeptide selected from said (1) to (12) and (17) to (25) as an active ingredient.

9. The anti-hepatitis B virus agent according to claim 5, wherein the polypeptide is in a form in which the polypeptide is attached to a carrier molecule for delivery into hepatocytes.

10. The anti-hepatitis B virus agent according to claim 9, wherein the carrier molecule is an antibody, antibody fragment, or single-chain antibody that binds to the asialoglycoprotein receptor.

11. The anti-hepatitis B virus agent according to claim 10, wherein the antibody, antibody fragment, or single-chain antibody has:
a heavy chain CDR1 containing an amino acid sequence shown in SEQ ID NO: 83. 89, 95, or 101, or an amino acid sequence which is identical thereto except that the amino acid residues are partially substituted and has 80% or more identity thereto,
a heavy chain CDR2 containing an amino acid sequence shown in SEQ ID NO: 84, 90, 96, or 102, or an amino acid sequence which is identical thereto except that the amino acid residues are partially substituted and has 80% or more identity thereto,
a heavy chain CDR3 containing an amino acid sequence shown in SEQ ID NO: 85, 91, 97, or 103, or an amino acid sequence which is identical thereto except that the amino acid residues are partially substituted and has 80% or more identity thereto,
a light chain CDR1 containing an amino acid sequence shown in SEQ ID NO: 86, 92, 98, or 104, or an amino acid sequence which is identical thereto except that the amino acid residues are partially substituted and has 80% or more identity thereto,
a light chain CDR2 containing an amino acid sequence shown in SEQ ID NO: 87, 93, 99, or 105, or an amino acid sequence which is identical thereto except that the amino acid residues are partially substituted and has 80% or more identity thereto, and
a light chain CDR3 containing an amino acid sequence shown in SEQ ID NO: 88, 94. 100. or 106. or an amino acid sequence which is identical thereto except that the amino acid residues are partially substituted and has 80% or more identity thereto.

12. The anti-hepatitis B virus agent according to claim 10 or 11, wherein the antibody, antibody fragment, or single-chain antibody is attached to the polypeptide via a cleavage sequence that is cleaved by an endogenous enzyme.

13. The anti-hepatitis B virus agent according to claim 5, wherein the polypeptide is in a form in which the polypeptide is attached to a cell membrane penetration-enhancing molecule.

14. The anti-hepatitis B virus agent according to claim 13, wherein the cell membrane penetration-enhancing molecule is a polypeptide of an amino acid sequence shown in SEQ ID NO: 107 or 108.

15. The anti-hepatitis B virus agent according to claim 5, wherein the polypeptide is in a form in which a nuclear localization signal is attached to the polypeptide.

16. Use of at least one selected from the following polypeptides (1) to (30) as a LIPG-binding peptide:
(1) a polypeptide of the sequence NCRHNTAG (SEQ ID NO: 25);
(2) a polypeptide of the sequence NARHNTAG (SEQ ID NO: 26);
(3) a polypeptide of the sequence LNCRDNTR (SEQ ID NO: 23);
(4) a polypeptide of the sequence LNARDNTR (SEQ ID NO: 24);
(5) a polypeptide of the sequence LNCRDNTRPVMSAMTC (SEQ ID NO: 1):
(6) a polypeptide of the sequence IRNVNHSDH (SEQ ID NO: 9):
(7) a polypeptide of the sequence LNVGYVFYP (SEQ ID NO: 18);
(8) a polypeptide of the sequence SLYTGFRAH (SEQ ID NO: 15):
(9) a polypeptide of the sequence RCRQSWNTM (SEQ ID NO: 14);
(10) a polypeptide of the sequence GADLRRGCC (SEQ ID NO: 11):
(11) a polypeptide of the sequence RKSGGVCENCRIINTAG (SEQ ID NO: 21):
(12) a polypeptide of the sequence LNCRHNTAGRHCHYCK (SEQ ID NO: 22):
(13) a polypeptide of an amino acid sequence shown in SEQ ID NO: 28;
(14) a polypeptide composed of a partial sequence of the polypeptide (13) and containing a region of amino acid positions 370 to 377 in the sequence of SEQ ID NO: 28;
(15) a polypeptide having 95% or more identity to the amino acid sequence shown in SEQ ID NO: 28, which is represented by an amino acid sequence identical to the amino acid sequence shown in SEQ ID NO: 28 except that one or more amino acids are substituted, deleted, inserted, and/or added in any region outside a region of amino acid positions 370 to 377;
(16) a polypeptide composed of a partial sequence of the polypeptide (15) and containing the region of amino acid positions 370 to 377;
(17) a polypeptide of the sequence LCECRDVLSCYYITDT (SEQ ID NO: 4):
(18) a polypeptide of the sequence CPCVNGATRHRPTSLC (SEQ ID NO: 8);
(19) a polypeptide of the sequence LVPWLRYAY (SEQ ID NO: 17);
(20) a polypeptide of the sequence NXRHNTAG (SEQ ID NO: 75) (X represents an amino acid except for cysteine and alanine);
(21) a polypeptide of the sequence LNXRDNTR (SEQ ID NO: 76) (X represents an amino acid except for cysteine and alanine);
(22) a polypeptide of the sequence LNCRHNTAG (SEQ ID NO: 77);
(23) a polypeptide of the sequence SNCRINTFRTVPIEQK (SEQ ID NO: 78);
(24) a polypeptide of the sequence SNCRINTFR (SEQ ID NO: 79):
(25) a polypeptide of the sequence TSNCRINTFR (SEQ ID NO: 80):
(26) a polypeptide of an amino acid sequence shown in SEQ ID NO: 82:
(27) a polypeptide composed of a partial sequence of the polypeptide (26) and containing a region of amino acid positions 456 to 464 in the sequence of SEQ ID NO: 82;
(28) a polypeptide having 95% or more identity to the amino acid sequence shown in SEQ ID NO: 82, which is represented by an amino acid sequence identical to the amino acid sequence shown in SEQ ID NO: 82 except that one or more amino acids are substituted, deleted, inserted, and/or added in any region outside a region of amino acid positions 456 to 464;
(29) a polypeptide composed of a partial sequence of the polypeptide (28) and containing the region of amino acid positions 456 to 464;
(30) a polypeptide having an identity of 80% or more and less than 100% to any of (1) to (14) and (17) to (27).

17. A LIPG-binding peptide consisting of at least one polypeptide selected from the following polypeptides (1) to (30):
(1) a polypeptide of the sequence NCRHNTAG (SEQ ID NO: 25);
(2) a polypeptide of the sequence NARHNTAG (SEQ ID NO: 26);
(3) a polypeptide of the sequence LNCRDNTR (SEQ ID NO: 23);
(4) a polypeptide of the sequence LNARDNTR (SEQ ID NO: 24);
(5) a polypeptide of the sequence LNCRDNTRPVMSAMTC (SEQ ID NO: 1);
(6) a polypeptide of the sequence IRNVNHSDH (SEQ ID NO: 9);
(7) a polypeptide of the sequence LNVGYVFYP (SEQ ID NO: 18);
(8) a polypeptide of the sequence SLYTGFRAH (SEQ ID NO: 15);
(9) a polypeptide of the sequence RCRQSWNTM (SEQ ID NO: 14);
(10) a polypeptide of the sequence GADLRRGCC (SEQ ID NO: 11);
(11) a polypeptide of the sequence RKSCGVCENCRHNTAG (SEQ ID NO: 21);
(12) a polypeptide of the sequence ENCRHNTAGRHCHYCK (SEQ ID NO: 22);
(13) a polypeptide of an amino acid sequence shown in SEQ ID NO: 28;
(14) a polypeptide composed of a partial sequence of the polypeptide (13) and containing a region of amino acid positions 370 to 377 in the sequence of SEQ ID NO: 28;
(15) a polypeptide having 95% or more identity to the amino acid sequence shown in SEQ ID NO: 28, which is represented by an amino acid sequence identical to the amino acid sequence shown in SEQ ID NO: 28 except that one or more amino acids are substituted, deleted, inserted, and/or added in any region outside a region of amino acid positions 370 to 377;
(16) a polypeptide composed of a partial sequence of the polypeptide (15) and containing the region of amino acid positions 370 to 377;
(17) a polypeptide of the sequence LCECRDVLSCYYITDT (SEQ ID NO: 4);
(18) a polypeptide of the sequence CPCVNGATRHRPTSLC (SEQ ID NO: 8);
(19) a polypeptide of the sequence LVPWLRYAY (SEQ ID NO: 17);
(20) a polypeptide of the sequence NXRHNTAG (SEQ ID NO: 75) (X represents an amino acid except for cysteine and alanine);
(21) a polypeptide of the sequence LNXRDNTR (SEQ ID NO: 76) (X represents an amino acid except for cysteine and alanine);
(22) a polypeptide of the sequence LNCRHNTAG (SEQ ID NO: 77);
(23) a polypeptide of the sequence SNCRINTFRTVPIEQK (SEQ ID NO: 78);
(24) a polypeptide of the sequence SNCRINTFR (SEQ ID NO: 79);
(25) a polypeptide of the sequence TSNCRINTFR (SEQ ID NO: 80);
(26) a polypeptide of an amino acid sequence shown in SEQ ID NO: 82;
(27) a polypeptide composed of a partial sequence of the polypeptide (26) and containing a region of amino acid positions 456 to 464 in the sequence of SEQ ID NO: 82;
(28) a polypeptide having 95% or more identity to the amino acid sequence shown in SEQ ID NO: 82, which is represented by an amino acid sequence identical to the amino acid sequence shown in SEQ ID NO: 82 except that one or more amino acids are substituted, deleted, inserted, and/or added in any region outside a region of amino acid positions 456 to 464;
(29) a polypeptide composed of a partial sequence of the polypeptide (28) and containing the region of amino acid positions 456 to 464;
(30) a polypeptide having an identity of 80% or more and less than 100% to any of (1) to (14) and (17) to (27).
